# EUROPEAN PATENT SPECIFICATION

(11) **EP 3 000 900 B1**
(45) Date of publication and mention of the grant of the patent: **28.02.2018**
(21) Application number: 15192470.1
(22) Date of filing: 19.04.2011
(51) Int. Cl.: C12Q 1/70

(54) **DETECTION OF HPV DNA REARRANGEMENTS**
NACHWEIS VON HPV-DNA-UMLAGERUNGEN
DÉTECTION DU HPV ADN RÉARRANGEMENTS

(30) Priority: 23.04.2010 US 327397 P
(43) Date of publication of application: 30.03.2016
(62) Divisional of application: 11738473.5
(73) Proprietor: Genomic Vision, 92220 Bagneux (FR)
(72) Inventor: MAHIET, Charlotte, London SE1 5RS (GB); SALVAIRE, Fabrice, 95870 Bezons (FR); CONSEILLER, Emmanuel, 75015 Paris (FR); BARRADEAU, Sébastien, 75020 Paris (FR)
(74) Representative: Desaix, Anne

(56) References cited:
- EP-A1- 1 331 277
- US-A1- 2009 123 926
- JÜRGEN REISINGER ET AL: "Visualization of episomal and integrated Epstein-Barr virus DNA by fiber fluorescence in situ hybridization", INTERNATIONAL JOURNAL OF CANCER (WILEY-LISS INC), vol. 118, no. 7, 1 April 2006 (2006-04-01) , pages 1603-1608, XP055008000, Hoboken, N.J, USA ISSN: 0020-7136, DOI: 10.1002/ijc.21498

### SEQUENCE LISTING

<110> Genomic Vision
<120> DETECTION OF HPV DNA REARRANGEMENTS
<130> B09419AEA_AD/PW
<140> US 61/327,397
   <141> 2010-04-23
<160> 24
<170> PatentIn version 3.5
<210> 1
   <211> 20
   <212> DNA
   <213> Artificial
<220>
   <223> Forward Primer HSV-P4
<400> 1
   tggttgtgtt actgggcaaa 20
<210> 2
   <211> 20
   <212> DNA
   <213> Artificial
<220>
   <223> Reverse Primer HSV-P4
<400> 2
   tcgatcgacg acaccataaa 20
<210> 3
   <211> 20
   <212> DNA
   <213> Artificial
<220>
   <223> Forward Primer HSV-P5
<400> 3
   cagatacgac tcccgcagat 20
<210> 4
   <211> 20
   <212> DNA
   <213> Artificial
<220>
   <223> Reverse Primer HSV-P5
<400> 4
   cgacgacctc gacgttattt 20
<210> 5
   <211> 20
   <212> DNA
   <213> Artificial
<220>
   <223> Forward Primer HSV-P6
<400> 5
   cgtgaggtcc aaaatcacct 20
<210> 6
   <211> 20
   <212> DNA
   <213> Artificial
<220>
   <223> Reverse Primer HSV-P6
<400> 6
   gacaggcaag ctcaaagtcc 20
<210> 7
   <211> 19
   <212> DNA
   <213> Artificial
<220>
   <223> Forward Primer HSV-P8
<400> 7
   agatgtccac gagcaccag 19
<210> 8
   <211> 20
   <212> DNA
   <213> Artificial
<220>
   <223> Reverse Primer HSV-P8
<400> 8
   cctgactttg tggggctaaa 20
<210> 9
   <211> 19
   <212> DNA
   <213> Artificial
<220>
   <223> Forward Primer HIV-S1
<400> 9
   tggaagggct aatttggtc 19
<210> 10
   <211> 20
   <212> DNA
   <213> Artificial
<220>
   <223> Reverse Primer HIV-S1
<400> 10
   tattgctggt gatcctttcc 20
<210> 11
   <211> 21
   <212> DNA
   <213> Artificial
<220>
   <223> Forward Primer HIV-S2
<400> 11
   ccagcaatat tccagtgtag c 21
<210> 12
   <211> 20
   <212> DNA
   <213> Artificial
<220>
   <223> Reverse Primer HIV-S2
<400> 12
   tgaaacaaac ttggcaatga 20
<210> 13
   <211> 20
   <212> DNA
   <213> Artificial
<220>
   <223> Forward Primer HIV-S3
<400> 13
   catctcctat ggcaggaaga 20
<210> 14
   <211> 22
   <212> DNA
   <213> Artificial
<220>
   <223> Reverse Primer HIV-S3
<400> 14
   tgctagagat tttccacact ga 22
<210> 15
   <211> 152261
   <212> DNA
   <213> Herpes simplex HSV-1 (NC_001806.1)
<400> 15
<210> 16
   <211> 154746
   <212> DNA
   <213> Herpes simplex HSV-2 (NC_001798.1)
<400> 16
<210> 17
   <211> 9181
   <212> DNA
   <213> Human Immunodeficiency Virus HIV1 (NC_001802.1)
<400> 17
   ggtctctctg gttagaccag atctgagcct gggagctctc tggctaacta gggaacccac      60
   tgcttaagcc tcaataaagc ttgccttgag tgcttcaagt agtgtgtgcc cgtctgttgt     120
   gtgactctgg taactagaga tccctcagac ccttttagtc agtgtggaaa atctctagca     180
   gtggcgcccg aacagggacc tgaaagcgaa agggaaacca gaggagctct ctcgacgcag     240
   gactcggctt gctgaagcgc gcacggcaag aggcgagggg cggcgactgg tgagtacgcc     300
   aaaaattttg actagcggag gctagaagga gagagatggg tgcgagagcg tcagtattaa     360
   gcgggggaga attagatcga tgggaaaaaa ttcggttaag gccaggggga aagaaaaaat     420
   ataaattaaa acatatagta tgggcaagca gggagctaga acgattcgca gttaatcctg     480
   gcctgttaga aacatcagaa ggctgtagac aaatactggg acagctacaa ccatcccttc     540
   agacaggatc agaagaactt agatcattat ataatacagt agcaaccctc tattgtgtgc 600
   atcaaaggat agagataaaa gacaccaagg aagctttaga caagatagag gaagagcaaa 660
   acaaaagtaa gaaaaaagca cagcaagcag cagctgacac aggacacagc aatcaggtca 720
   gccaaaatta ccctatagtg cagaacatcc aggggcaaat ggtacatcag gccatatcac 780
   ctagaacttt aaatgcatgg gtaaaagtag tagaagagaa ggctttcagc ccagaagtga 840
   tacccatgtt ttcagcatta tcagaaggag ccaccccaca agatttaaac accatgctaa 900
   acacagtggg gggacatcaa gcagccatgc aaatgttaaa agagaccatc aatgaggaag 960
   ctgcagaatg ggatagagtg catccagtgc atgcagggcc tattgcacca ggccagatga 1020
   gagaaccaag gggaagtgac atagcaggaa ctactagtac ccttcaggaa caaataggat 1080
   ggatgacaaa taatccacct atcccagtag gagaaattta taaaagatgg ataatcctgg 1140
   gattaaataa aatagtaaga atgtatagcc ctaccagcat tctggacata agacaaggac 1200
   caaaggaacc ctttagagac tatgtagacc ggttctataa aactctaaga gccgagcaag 1260
   cttcacagga ggtaaaaaat tggatgacag aaaccttgtt ggtccaaaat gcgaacccag 1320
   attgtaagac tattttaaaa gcattgggac cagcggctac actagaagaa atgatgacag 1380
   catgtcaggg agtaggagga cccggccata aggcaagagt tttggctgaa gcaatgagcc 1440
   aagtaacaaa ttcagctacc ataatgatgc agagaggcaa ttttaggaac caaagaaaga 1500
   ttgttaagtg tttcaattgt ggcaaagaag ggcacacagc cagaaattgc agggccccta 1560
   ggaaaaaggg ctgttggaaa tgtggaaagg aaggacacca aatgaaagat tgtactgaga 1620
   gacaggctaa ttttttaggg aagatctggc cttcctacaa gggaaggcca gggaattttc 1680
   ttcagagcag accagagcca acagccccac cagaagagag cttcaggtct ggggtagaga 1740
   caacaactcc ccctcagaag caggagccga tagacaagga actgtatcct ttaacttccc 1800
   tcaggtcact ctttggcaac gacccctcgt cacaataaag ataggggggc aactaaagga 1860
   agctctatta gatacaggag cagatgatac agtattagaa gaaatgagtt tgccaggaag 1920
   atggaaacca aaaatgatag ggggaattgg aggttttatc aaagtaagac agtatgatca 1980
   gatactcata gaaatctgtg gacataaagc tataggtaca gtattagtag gacctacacc 2040
   tgtcaacata attggaagaa atctgttgac tcagattggt tgcactttaa attttcccat 2100
   tagccctatt gagactgtac cagtaaaatt aaagccagga atggatggcc caaaagttaa 2160
   acaatggcca ttgacagaag aaaaaataaa agcattagta gaaatttgta cagagatgga 2220
   aaaggaaggg aaaatttcaa aaattgggcc tgaaaatcca tacaatactc cagtatttgc 2280
   cataaagaaa aaagacagta ctaaatggag aaaattagta gatttcagag aacttaataa 2340
   gagaactcaa gacttctggg aagttcaatt aggaatacca catcccgcag ggttaaaaaa 2400
   gaaaaaatca gtaacagtac tggatgtggg tgatgcatat ttttcagttc ccttagatga 2460
   agacttcagg aagtatactg catttaccat acctagtata aacaatgaga caccagggat 2520
   tagatatcag tacaatgtgc ttccacaggg atggaaagga tcaccagcaa tattccaaag 2580
   tagcatgaca aaaatcttag agccttttag aaaacaaaat ccagacatag ttatctatca 2640
   atacatggat gatttgtatg taggatctga cttagaaata gggcagcata gaacaaaaat 2700
   agaggagctg agacaacatc tgttgaggtg gggacttacc acaccagaca aaaaacatca 2760
   gaaagaacct ccattccttt ggatgggtta tgaactccat cctgataaat ggacagtaca 2820
   gcctatagtg ctgccagaaa aagacagctg gactgtcaat gacatacaga agttagtggg 2880
   gaaattgaat tgggcaagtc agatttaccc agggattaaa gtaaggcaat tatgtaaact 2940
   ccttagagga accaaagcac taacagaagt aataccacta acagaagaag cagagctaga 3000
   actggcagaa aacagagaga ttctaaaaga accagtacat ggagtgtatt atgacccatc 3060
   aaaagactta atagcagaaa tacagaagca ggggcaaggc caatggacat atcaaattta 3120
   tcaagagcca tttaaaaatc tgaaaacagg aaaatatgca agaatgaggg gtgcccacac 3180
   taatgatgta aaacaattaa cagaggcagt gcaaaaaata accacagaaa gcatagtaat 3240
   atggggaaag actcctaaat ttaaactgcc catacaaaag gaaacatggg aaacatggtg 3300
   gacagagtat tggcaagcca cctggattcc tgagtgggag tttgttaata cccctcccct 3360
   agtgaaatta tggtaccagt tagagaaaga acccatagta ggagcagaaa ccttctatgt 3420
   agatggggca gctaacaggg agactaaatt aggaaaagca ggatatgtta ctaatagagg 3480
   aagacaaaaa gttgtcaccc taactgacac aacaaatcag aagactgagt tacaagcaat 3540
   ttatctagct ttgcaggatt cgggattaga agtaaacata gtaacagact cacaatatgc 3600
   attaggaatc attcaagcac aaccagatca aagtgaatca gagttagtca atcaaataat 3660
   agagcagtta ataaaaaagg aaaaggtcta tctggcatgg gtaccagcac acaaaggaat 3720
   tggaggaaat gaacaagtag ataaattagt cagtgctgga atcaggaaag tactattttt 3780
   agatggaata gataaggccc aagatgaaca tgagaaatat cacagtaatt ggagagcaat 3840
   ggctagtgat tttaacctgc cacctgtagt agcaaaagaa atagtagcca gctgtgataa 3900
   atgtcagcta aaaggagaag ccatgcatgg acaagtagac tgtagtccag gaatatggca 3960
   actagattgt acacatttag aaggaaaagt tatcctggta gcagttcatg tagccagtgg 4020
   atatatagaa gcagaagtta ttccagcaga aacagggcag gaaacagcat attttctttt 4080
   aaaattagca ggaagatggc cagtaaaaac aatacatact gacaatggca gcaatttcac 4140
   cggtgctacg gttagggccg cctgttggtg ggcgggaatc aagcaggaat ttggaattcc 4200
   ctacaatccc caaagtcaag gagtagtaga atctatgaat aaagaattaa agaaaattat 4260
   aggacaggta agagatcagg ctgaacatct taagacagca gtacaaatgg cagtattcat 4320
   ccacaatttt aaaagaaaag gggggattgg ggggtacagt gcaggggaaa gaatagtaga 4380
   cataatagca acagacatac aaactaaaga attacaaaaa caaattacaa aaattcaaaa 4440
   ttttcgggtt tattacaggg acagcagaaa tccactttgg aaaggaccag caaagctcct 4500
   ctggaaaggt gaaggggcag tagtaataca agataatagt gacataaaag tagtgccaag 4560
   aagaaaagca aagatcatta gggattatgg aaaacagatg gcaggtgatg attgtgtggc 4620
   aagtagacag gatgaggatt agaacatgga aaagtttagt aaaacaccat atgtatgttt 4680
   cagggaaagc taggggatgg ttttatagac atcactatga aagccctcat ccaagaataa 4740
   gttcagaagt acacatccca ctaggggatg ctagattggt aataacaaca tattggggtc 4800
   tgcatacagg agaaagagac tggcatttgg gtcagggagt ctccatagaa tggaggaaaa 4860
   agagatatag cacacaagta gaccctgaac tagcagacca actaattcat ctgtattact 4920
   ttgactgttt ttcagactct gctataagaa aggccttatt aggacacata gttagcccta 4980
   ggtgtgaata tcaagcagga cataacaagg taggatctct acaatacttg gcactagcag 5040
   cattaataac accaaaaaag ataaagccac ctttgcctag tgttacgaaa ctgacagagg 5100
   atagatggaa caagccccag aagaccaagg gccacagagg gagccacaca atgaatggac 5160
   actagagctt ttagaggagc ttaagaatga agctgttaga cattttccta ggatttggct 5220
   ccatggctta gggcaacata tctatgaaac ttatggggat acttgggcag gagtggaagc 5280
   cataataaga attctgcaac aactgctgtt tatccatttt cagaattggg tgtcgacata 5340
   gcagaatagg cgttactcga cagaggagag caagaaatgg agccagtaga tcctagacta 5400
   gagccctgga agcatccagg aagtcagcct aaaactgctt gtaccaattg ctattgtaaa 5460
   aagtgttgct ttcattgcca agtttgtttc ataacaaaag ccttaggcat ctcctatggc 5520
   aggaagaagc ggagacagcg acgaagagct catcagaaca gtcagactca tcaagcttct 5580
   ctatcaaagc agtaagtagt acatgtaatg caacctatac caatagtagc aatagtagca 5640
   ttagtagtag caataataat agcaatagtt gtgtggtcca tagtaatcat agaatatagg 5700
   aaaatattaa gacaaagaaa aatagacagg ttaattgata gactaataga aagagcagaa 5760
   gacagtggca atgagagtga aggagaaata tcagcacttg tggagatggg ggtggagatg 5820
   gggcaccatg ctccttggga tgttgatgat ctgtagtgct acagaaaaat tgtgggtcac 5880
   agtctattat ggggtacctg tgtggaagga agcaaccacc actctatttt gtgcatcaga 5940
   tgctaaagca tatgatacag aggtacataa tgtttgggcc acacatgcct gtgtacccac 6000
   agaccccaac ccacaagaag tagtattggt aaatgtgaca gaaaatttta acatgtggaa 6060
   aaatgacatg gtagaacaga tgcatgagga tataatcagt ttatgggatc aaagcctaaa 6120
   gccatgtgta aaattaaccc cactctgtgt tagtttaaag tgcactgatt tgaagaatga 6180
   tactaatacc aatagtagta gcgggagaat gataatggag aaaggagaga taaaaaactg 6240
   ctctttcaat atcagcacaa gcataagagg taaggtgcag aaagaatatg cattttttta 6300
   taaacttgat ataataccaa tagataatga tactaccagc tataagttga caagttgtaa 6360
   cacctcagtc attacacagg cctgtccaaa ggtatccttt gagccaattc ccatacatta 6420
   ttgtgccccg gctggttttg cgattctaaa atgtaataat aagacgttca atggaacagg 6480
   accatgtaca aatgtcagca cagtacaatg tacacatgga attaggccag tagtatcaac 6540
   tcaactgctg ttaaatggca gtctagcaga agaagaggta gtaattagat ctgtcaattt 6600
   cacggacaat gctaaaacca taatagtaca gctgaacaca tctgtagaaa ttaattgtac 6660
   aagacccaac aacaatacaa gaaaaagaat ccgtatccag agaggaccag ggagagcatt 6720
   tgttacaata ggaaaaatag gaaatatgag acaagcacat tgtaacatta gtagagcaaa 6780
   atggaataac actttaaaac agatagctag caaattaaga gaacaatttg gaaataataa 6840
   aacaataatc tttaagcaat cctcaggagg ggacccagaa attgtaacgc acagttttaa 6900
   ttgtggaggg gaatttttct actgtaattc aacacaactg tttaatagta cttggtttaa 6960
   tagtacttgg agtactgaag ggtcaaataa cactgaagga agtgacacaa tcaccctccc 7020
   atgcagaata aaacaaatta taaacatgtg gcagaaagta ggaaaagcaa tgtatgcccc 7080
   tcccatcagt ggacaaatta gatgttcatc aaatattaca gggctgctat taacaagaga 7140
   tggtggtaat agcaacaatg agtccgagat cttcagacct ggaggaggag atatgaggga 7200
   caattggaga agtgaattat ataaatataa agtagtaaaa attgaaccat taggagtagc 7260
   acccaccaag gcaaagagaa gagtggtgca gagagaaaaa agagcagtgg gaataggagc 7320
   tttgttcctt gggttcttgg gagcagcagg aagcactatg ggcgcagcct caatgacgct 7380
   gacggtacag gccagacaat tattgtctgg tatagtgcag cagcagaaca atttgctgag 7440
   ggctattgag gcgcaacagc atctgttgca actcacagtc tggggcatca agcagctcca 7500
   ggcaagaatc ctggctgtgg aaagatacct aaaggatcaa cagctcctgg ggatttgggg 7560
   ttgctctgga aaactcattt gcaccactgc tgtgccttgg aatgctagtt ggagtaataa 7620
   atctctggaa cagatttgga atcacacgac ctggatggag tgggacagag aaattaacaa 7680
   ttacacaagc ttaatacact ccttaattga agaatcgcaa aaccagcaag aaaagaatga 7740
   acaagaatta ttggaattag ataaatgggc aagtttgtgg aattggttta acataacaaa 7800
   ttggctgtgg tatataaaat tattcataat gatagtagga ggcttggtag gtttaagaat 7860
   agtttttgct gtactttcta tagtgaatag agttaggcag ggatattcac cattatcgtt 7920
   tcagacccac ctcccaaccc cgaggggacc cgacaggccc gaaggaatag aagaagaagg 7980
   tggagagaga gacagagaca gatccattcg attagtgaac ggatccttgg cacttatctg 8040
   ggacgatctg cggagcctgt gcctcttcag ctaccaccgc ttgagagact tactcttgat 8100
   tgtaacgagg attgtggaac ttctgggacg cagggggtgg gaagccctca aatattggtg 8160
   gaatctccta cagtattgga gtcaggaact aaagaatagt gctgttagct tgctcaatgc 8220
   cacagccata gcagtagctg aggggacaga tagggttata gaagtagtac aaggagcttg 8280
   tagagctatt cgccacatac ctagaagaat aagacagggc ttggaaagga ttttgctata 8340
   agatgggtgg caagtggtca aaaagtagtg tgattggatg gcctactgta agggaaagaa 8400
   tgagacgagc tgagccagca gcagataggg tgggagcagc atctcgagac ctggaaaaac 8460
   atggagcaat cacaagtagc aatacagcag ctaccaatgc tgcttgtgcc tggctagaag 8520
   cacaagagga ggaggaggtg ggttttccag tcacacctca ggtaccttta agaccaatga 8580
   cttacaaggc agctgtagat cttagccact ttttaaaaga aaagggggga ctggaagggc 8640
   taattcactc ccaaagaaga caagatatcc ttgatctgtg gatctaccac acacaaggct 8700
   acttccctga ttagcagaac tacacaccag ggccaggggt cagatatcca ctgacctttg 8760
   gatggtgcta caagctagta ccagttgagc cagataagat agaagaggcc aataaaggag 8820
   agaacaccag cttgttacac cctgtgagcc tgcatgggat ggatgacccg gagagagaag 8880
   tgttagagtg gaggtttgac agccgcctag catttcatca cgtggcccga gagctgcatc 8940
   cggagtactt caagaactgc tgacatcgag cttgctacaa gggactttcc gctggggact 9000
   ttccagggag gcgtggcctg ggcgggactg gggagtggcg agccctcaga tcctgcatat 9060
   aagcagctgc tttttgcctg tactgggtct ctctggttag accagatctg agcctgggag 9120
   ctctctggct aactagggaa cccactgctt aagcctcaat aaagcttgcc ttgagtgctt 9180
   c 9181
<210> 18
   <211> 10359
   <212> DNA
   <213> Human Immunodeficiency Virus HIV2 (NC_001722.1)
<400> 18
   tgcaagggat gttttacagt aggaggagac atagaatcct agacatatac ctagaaaaag      60
   aggaagggat aataccagat tggcagaatt atactcatgg gccaggagta aggtacccaa     120
   tgtacttcgg gtggctgtgg aagctagtat cagtagaact ctcacaagag gcagaggaag     180
   atgaggccaa ctgcttagta cacccagcac aaacaagcag acatgatgat gagcatgggg     240
   agacattagt gtggcagttt gactccatgc tggcctataa ctacaaggcc ttcactctgt     300
   acccagaaga gtttgggcac aagtcaggat tgccagagaa agaatggaag gcaaaactga     360
   aagcaagagg gataccatat agtgaataac aggaacaacc atacttggtc aaggcaggaa     420
   gtagctacta agaaacagct gaggctgcag ggactttcca gaaggggctg taaccaaggg     480
   agggacatgg gaggagctgg tggggaacgc cctcatactt actgtataaa tgtacccgct     540
   tcttgcattg tattcagtcg ctctgcggag aggctggcag atcgagccct gagaggttct      600
   ctccagcact agcaggtaga gcctgggtgt tccctgctgg actctcacca gtacttggcc      660
   ggtactgggc agacggctcc acgcttgctt gcttaaagac ctcttcaata aagctgccag      720
   ttagaagcaa gttaagtgtg tgttcccatc tctcctagtc gccgcctggt cattcggtgt      780
   tcatctgagt aacaagaccc tggtctgtta ggacccttct cgctttggga atccaaggca      840
   ggaaaatccc tagcaggttg gcgcccgaac agggacttga agaggactga gaagccctgg      900
   aactcggctg agtgaaggca gtaagggcgg caggaacaaa ccacgacgga gtgctcctag      960
   aaaggcgcgg gccgaggtac caaaggcggc gtgtggagcg ggagtgaaag aggcctccgg     1020
   gtgaaggtaa gtacctacac caaaaactgt agccagaaaa ggcttgttat cctaccttta     1080
   gacaggtaga agattgtggg agatgggcgc gagaaactcc gtcttgagag ggaaaaaagc     1140
   agacgaatta gaaaaagtta ggttacggcc cggcggaaag aaaaagtaca ggttaaaaca     1200
   tattgtgtgg gcagcgaatg aattggataa attcggattg gcagagagcc tgttggagtc     1260
   aaaagaaggt tgccaaaaga ttctcagagt tttagatcca ttagtaccaa cagggtcaga     1320
   aaatttaaaa agccttttta ataccgtctg cgtcatttgg tgcttgcacg cagaagagaa     1380
   agtgaaagat actgaggaag caaagaaact agcacagaga catctagtgg cagaaactgg     1440
   aactgcagag aaaatgccaa atacaagtag accaacagca ccacctagtg ggaaaagagg     1500
   aaactacccc gtgcaacaag cgggtggcaa ctatgtccat gtgccactga gcccccgaac     1560
   tctaaatgca tgggtaaaat tagtggagga aaagaagttc ggggcagaag tagtgccagg     1620
   atttcaggca ctctcagaag gctgcacgcc ctatgatatt aatcaaatgc ttaattgtgt     1680
   gggcgatcac caagcagcta tgcaaataat cagagagatt attaatgaag aagcagcaga     1740
   ctgggattcg cagcacccaa taccaggccc cttaccagca ggacagctca gagacccaag     1800
   agggtctgac atagcaggaa caacaagcac agtagatgaa cagatccagt ggatgtatag     1860
   gccacaaaat cccgtaccgg tagggaacat ctacagaaga tggatccaaa tagggctgca     1920
   aaagtgtgtc agaaagtaca acccaactaa catcttagac ataaaacagg gaccaaaaga     1980
   accgttccaa agctatgtag acaggttcta caaaagcttg agggcagaac aaacagaccc     2040
   agcagtaaaa aattggatga cccaaacgct gctaatacag aatgccaacc cagactgcaa     2100
   gttagtacta aaaggactgg ggatgaatcc caccctagaa gagatgctaa ccgcctgcca     2160
   gggggtaggc ggaccaggcc agaaagccag gctaatggct gaagccctaa aagaggctat     2220
   gggaccaagc cctatcccat ttgcagcagc ccaacaaaga aaggcaatta ggtattggaa     2280
   ctgtggaaag gagggacact cggcaagaca gtgccgagca cctagaagac agggctgctg     2340
   gaagtgtggc aagccaggac acatcatggc aaactgcccg gaaagacagg caggtttttt     2400
   agggttgggc ccacggggaa agaagcctcg caacttcccc gtgacccaag cccctcaggg     2460
   gctgatacca acagcacctc cggcagatcc agcagcggaa ctgttggaga gatatatgca 2520
   gcaagggaga aagcagaggg agcagaggga gagaccatac aaagaggtga cggaggactt 2580
   gctgcacctc gagcagagag agacacctca cagagaggag acagaggact tgctgcacct 2640
   caattctctc tttggaaaag accagtagtc acagcgtaca tcgaggatca gccggtagaa 2700
   gtcttactag acacaggggc tgatgactca atagtagcag gaatagaatt aggggacaat 2760
   tacactccaa aaatagtagg gggaataggg ggatttataa acaccaaaga atacaaaaat 2820
   gtagaaataa aagtactaaa taaaagagta agagccacca taatgacagg agatacccca 2880
   atcaacatct ttggcagaaa tattctgaca gccttaggca tgtcattaaa tttaccagtt 2940
   gccaagatag agccaataaa agtaacattg aagccaggga aagatggacc aaggctgaaa 3000
   caatggcccc taacaaaaga gaaaatagaa gcactaaaag agatctgtga aaaaatggaa 3060
   aaagagggcc agctagaaga ggcacctcca actaatcctt ataatacccc cacatttgca 3120
   attaagaaaa aggacaagaa caaatggagg atgctgatag attttagaga actaaataag 3180
   gtgactcaag atttcacaga aattcagcta ggaattccac acccggcagg actagccaaa 3240
   aagaaaagga tctctatatt agatgtaggg gatgcctatt tttccatacc actacatgaa 3300
   gattttaggc agtatactgc atttacccta ccagcagtaa acaatatgga accaggaaaa 3360
   agatatatat ataaagtctt gccacaagga tggaagggat caccagcaat ttttcaatac 3420
   acaatgaggc aagtcttaga acctttcaga aaagcaaacc cagatgtcat tctcatccag 3480
   tacatggatg atatcttaat agctagtgac aggacaggtt tagagcatga caaagtggtc 3540
   ctgcagctaa aagaacttct aaatggccta gggttttcta ctccagatga gaagttccaa 3600
   aaagaccctc catttcaatg gatgggctgt gaactatggc caactaaatg gaagctgcag 3660
   aaactacaac tgccccagaa agacatatgg acagtcaatg acatccaaaa gctagtggga 3720
   gtcttaaatt gggcggcaca aatctattca ggaataaaaa ccaaacactt atgtagacta 3780
   attagaggaa aaatgacact cacagaagaa gtgcagtgga cagaactagc agaagcagag 3840
   ctagaagaaa acaaaattat cttgagccag gaacaagaag gatattatta ccaagaagaa 3900
   aaagaattag aggcaacaat ccaaaaaagc caaggacatc aatggacata caaaatacac 3960
   caggaagaga aaatcctaaa agtaggaaag tatgcaaaga taaaaaatac ccataccaat 4020
   ggggtcagat tactagcaca ggtagttcag aaaataggaa aagaggcact agtcatttgg 4080
   ggacggatac caaaatttca cctgccagtg gagagagaga cctgggagca gtggtgggat 4140
   aactactggc aagtgacatg gatcccagag tgggactttg tatctacccc accactggtc 4200
   aggttaacat ttaacctagt aggagatcct ataccaggcg cagagacctt ctacacagat 4260
   ggatcatgca atagacagtc aaaagaggga aaagcaggat atgtaacaga tagaggaaaa 4320
   gacaaagtaa aagtattaga acaaactacc aatcagcagg cagaattaga agtctttcgg 4380
   atggcactgg cagactcagg cccaaaggtt aatatcatag tagattcaca gtatgtaatg 4440
   gggatagtag caggccagcc aacagagtca gaaaatagaa tagtgaacca gatcatagaa 4500
   gaaatgataa agaaggaagc agtctatgtt gcatgggtcc cagcccataa aggcatagga 4560
   ggaaaccagg aagtagacca tttagtaagt caaggcatca gacaagtatt attcctggaa 4620
   aagatagagc ccgctcaaga ggaacatgaa aaatatcata gcattataaa agaactaacc 4680
   cataaatttg gaatacccct tctagtagca agacagatag taaactcatg tgcccaatgc 4740
   caacagaaag gagaagccat acatgggcaa gtaaatgcag aaataggcgt ttggcaaatg 4800
   gactacacac acttagaagg aaaaatcatt atagtagcag tacatgttgc aagtggattc 4860
   atagaagcag aagtcatccc acaggaatca ggaaggcaga cagcactctt cctattaaaa 4920
   ctggccagta ggtggccaat aacgcacttg cacacagaca atggccccaa cttcacttca 4980
   caggaagtga agatggtggc atggtgggta ggtatagaac aatcctttgg agtaccttac 5040
   aacccacaaa gccagggagt agtagaagca atgaatcacc acctaaagaa tcagataagt 5100
   agaattagag aacaggcaaa tacaatagaa acaatagtac tgatggcagt tcattgcatg 5160
   aattttaaaa gaaggggagg aataggggat atgaccccag cagaaagact aatcaacatg 5220
   attaccacag aacaagaaat acaattcctc caaagaaaaa attcaaattt taaaaatttc 5280
   caggtctatt acagagaagg cagagatcag ctgtggaaag gacctggtga actactgtgg 5340
   aagggagaag gagcagtcat agtcaaggta gggacagaca taaaagtagt accaagaagg 5400
   aaggccaaga ttatcaggga ctatggagga agacaggaac tggatagtag tccccacctg 5460
   gagggtgcca gggaggatgg agaaatggca tgcccttgtc aagtacctga aatacagaac 5520
   aaaagaccta gaggaggtgc gctatgttcc ccaccacaag gtgggatggg catggtggac 5580
   ttgcagcagg gtaatattcc cactacaagg aaaaagtcat ctagaaatac aggcatattg 5640
   gaacctaaca ccagaaaaag gatggctctc ctctcatgca gtaagattaa cctggtatac 5700
   agaaaagttc tggacagatg ttaccccaga ctgtgcagac atcctaatac atagcactta 5760
   tttctcttgc tttacggcag gtgaagtaag aagagccatc agaggggaaa agttattgtc 5820
   ctgctgcaac tatccccaag ctcataaagc acaggtacca tcacttcaat acctagccct 5880
   agtagtagta caacaaaatg acagacccca gagaaagggt accgccagga aacagtggag 5940
   aagagaccat tggagaggcc ttcgagtggc tagagaggac catagaagcc ttaaacaggg 6000
   aggcagtgaa ccatctgccc cgagagctca ttttccaggt gtggcaaagg tcctggagat 6060
   attggcatga tgaacaaggg atgtcagcaa gctacacaaa gtatagatat ttgtgcctaa 6120
   tgcaaaaagc tatatttaca catttcaaga gagggtgcac ttgctggggg gaggacatgg 6180
   gccgggaagg attggaagac caaggacctc cccctcctcc ccctccaggt ctagtctaat 6240
   gactgaagca ccaacagagt ttcccccaga agatgggacc ccacggaggg acttagggag 6300
   tgactgggta atagaaactc tgagggaaat aaaggaagaa gccttaagac attttgatcc 6360
   ccgcttgcta attgctcttg gctactatat ccataataga catggagaca cccttgaagg 6420
   cgccagagag ctcattaaaa ccctacaacg agccctcttc gtgcacttca gagcgggatg 6480
   taaccgctca agaattggct aaacaaggag aagaactcct tgcccagctg caccgacccc 6540
   tagagccatg cactaacaaa tgctattgta agcgatgcag tttccattgc cagctgtgtt 6600
   tctcgaaaaa ggggctcgga atatcatatg agcgaaaggg cagacgaaga aggactccaa 6660
   ggaaaactaa gactccttcg ccttctgcac cagacaagtg agtatggagc ctggtaggaa 6720
   tcagctgttt gttgtcattt tactaacaag tgcttgctta gtatattgta gccagtatgt 6780
   gactgttttc tatggcatac ccgcgtggaa aaatgcatct attcccttat tttgtgcaac 6840
   taaaaataga gacacttggg ggaccataca gtgcttgcca gacaatgatg attatcagga 6900
   aataatttta aatgtgacag aggcttttga tgcatggaat aatacagtga cagaacaagc 6960
   agtagaagat gtctggcatc tatttgagac atcaataaaa ccatgtgtca agctaacacc 7020
   tctatgtgtg gcaatgaatt gtagcagggt tcaagggaat accacgaccc cgaatcccag 7080
   gacctcgagt tccacaacct cgagaccacc cacatccgca gcctccataa taaatgaaac 7140
   ttctaactgc atagaaaaca acacatgcgc aggattaggg tatgaggaga tgatgcaatg 7200
   tgagttcaat atgaaggggt tagaacaaga taagaaaagg aggtataagg acacatggta 7260
   tttagaagat gtggtttgtg acaacacaac agctggcaca tgttacatga gacattgcaa 7320
   cacatcaatc atcaaagagt catgtgataa gcactattgg gatgctatga ggtttagata 7380
   ctgtgcacca ccgggctttg ccctattaag atgtaatgat accaactatt caggctttga 7440
   acctaagtgc actaaagtag tagctgcttc atgcacaagg atgatggaaa cgcaaacttc 7500
   tacttggttt ggctttaatg gcactagagc agaaaataga acatatatct attggcatgg 7560
   cagagataat aggactatca ttagcttaaa caagtattat aatctcacaa tgcgttgtaa 7620
   gagaccagga aataagacag ttttaccaat aacacttatg tcaggattag tgtttcactc 7680
   tcagccaatc aacacaaggc ctaggcaggc atggtgccgg tttggaggca gatggaggga 7740
   agccatgcag gaggtgaagc aaacccttgt acaacatccc agatacaaag gaatcaatga 7800
   tacagggaaa attaacttta cgaaaccggg agcaggctca gacccggaag tggcatttat 7860
   gtggactaac tgcagaggag aatttctcta ctgtaacatg acttggttcc tcaattgggt 7920
   agaagacaag aaccaaacac ggcgcaacta ttgccatata aagcagataa ttaatacctg 7980
   gcataaagta gggaaaaatg tatatttgcc tcctagggaa ggggagttgg cctgtgaatc 8040
   aacagtaacc agcataattg ctaacattga catagataaa aatcggactc ataccaacat 8100
   tacctttagt gcagaagtgg cagaactgta ccgattagaa ctgggagact acaaattaat      8160
   agaaataaca ccaattggct tcgcacctac agatcagaga aggtactcct caactccagt      8220
   gaggaacaaa agaggtgtgt tcgtgctagg gttcttgggt tttctcgcga cagcaggttc      8280
   tgcaatgggc gcgcggtccc tgacgctgtc agcccagtcc cggactttac tggccgggat      8340
   agtgcagcaa cagcaacagc tgttggacgt agtcaagaga caacaagaaa tgttgcgact      8400
   gaccgtctgg ggaacgaaaa acctccaggc aagagtcact gctatcgaga agtacctaaa      8460
   gcatcaggca cagctaaatt catggggatg tgcgtttaga caggtctgcc acactactgt      8520
   accgtgggta aatgactctt tatcgcctga ctggaaaaat atgacatggc aggagtggga      8580
   gaaacaagtc cgctacctag aggcaaatat cagtcaaagt ttagaagaag cccaaattca      8640
   acaagaaaag aatatgtatg aattacaaaa attaaatagc tgggatattc ttggcaactg      8700
   gtttgactta acctcctggg tcaagtatat tcaatatgga gtgcatatag tagtgggaat      8760
   aatagcttta agaatagcaa tctatgtagt gcaattgtta agtagattta gaaagggcta      8820
   taggcctgtt ttctcttccc cccccggtta tctccaacag atccatatcc acaaggaccg      8880
   gggacagcca gccaacgaag gaacagaaga agacgtcgga ggcgacagtg gttacgactt      8940
   gtggccttgg ccaataaact atgtgcagtt cctgatccac ctactgactc gcctcttgat      9000
   cgggctatac aacatctgca gagacttact atccaagaac tccccgaccc gccgactgat      9060
   ctcccagagt ctaacagcaa tcagggactg gctgagactt aaggcggccc aactgcaata      9120
   tgggtgcgag tggatccaag aagctttcca agcattcgcg aggactacga gagagactct      9180
   tgcgggcgcg tggggatggt tatgggaagc agcgcgacgc atcgggaggg gaatactcgc      9240
   agttccaaga agaatcaggc agggagcaga actcgccctc ctgtgaggga cagcagtatc      9300
   agcagggaga gtacatgaac agcccatgga gaaacccagc aacagaaaga cagaaagatt      9360
   tgtataggca gcaaaatatg gatgatgtag attctgatga tgatgaccta ataggagttc      9420
   ctgttacacc aagagtacca cggagagaaa tgacctataa attggcaata gatatgtcac      9480
   attttataaa agaaaaaggg ggactgcaag ggatgtttta cagtaggagg agacatagaa      9540
   tcctagacat atacctagaa aaagaggaag ggataatacc agattggcag aattatactc      9600
   atgggccagg agtaaggtac ccaatgtact tcgggtggct gtggaagcta gtatcagtag      9660
   aactctcaca agaggcagag gaagatgagg ccaactgctt agtacaccca gcacaaacaa      9720
   gcagacatga tgatgagcat ggggagacat tagtgtggca gtttgactcc atgctggcct      9780
   ataactacaa ggccttcact ctgtacccag aagagtttgg gcacaagtca ggattgccag      9840
   agaaagaatg gaaggcaaaa ctgaaagcaa gagggatacc atatagtgaa taacaggaac      9900
   aaccatactt ggtcaaggca ggaagtagct actaagaaac agctgaggct gcagggactt      9960
   tccagaaggg gctgtaacca agggagggac atgggaggag ctggtgggga acgccctcat     10020
   acttactgta taaatgtacc cgcttcttgc attgtattca gtcgctctgc ggagaggctg    10080
   gcagatcgag ccctgagagg ttctctccag cactagcagg tagagcctgg gtgttccctg    10140
   ctggactctc accagtactt ggccggtact gggcagacgg ctccacgctt gcttgcttaa    10200
   agacctcttc aataaagctg ccagttagaa gcaagttaag tgtgtgttcc catctctcct    10260
   agtcgccgcc tggtcattcg gtgttcatct gagtaacaag accctggtct gttaggaccc    10320
   ttctcgcttt gggaatccaa ggcaggaaaa tccctagca                           10359
<210> 19
   <211> 3215
   <212> DNA
   <213> Hepatitis B Virus HBV (NC_003977.1)
<400> 19
   ctccacaaca ttccaccaag ctctgctaga tcccagagtg aggggcctat attttcctgc 60
   tggtggctcc agttccggaa cagtaaaccc tgttccgact actgcctcac ccatatcgtc 120
   aatcttctcg aggactgggg accctgcacc gaacatggag agcacaacat caggattcct 180
   aggacccctg ctcgtgttac aggcggggtt tttcttgttg acaagaatcc tcacaatacc 240
   acagagtcta gactcgtggt ggacttctct caattttcta gggggagcac ccacgtgtcc 300
   tggccaaaat tcgcagtccc caacctccaa tcactcacca acctcttgtc ctccaacttg 360
   tcctggctat cgctggatgt gtctgcggcg ttttatcata ttcctcttca tcctgctgct 420
   atgcctcatc ttcttgttgg ttcttctgga ctaccaaggt atgttgcccg tttgtcctct 480
   acttccagga acatcaacta ccagcacggg accatgcaga acctgcacga ttcctgctca 540
   aggaacctct atgtttccct cttgttgctg tacaaaacct tcggacggaa actgcacttg 600
   tattcccatc ccatcatcct gggctttcgc aagattccta tgggagtggg cctcagtccg 660
   tttctcctgg ctcagtttac tagtgccatt tgttcagtgg ttcgtagggc tttcccccac 720
   tgtttggctt tcagctatat ggatgatgtg gtattggggg ccaagtctgt acaacatctt 780
   gagtcccttt ttacctctat taccaatttt cttttgtctt tgggtataca tttgaaccct 840
   aataaaacca aacgttgggg ctactccctt aacttcatgg gatatgtaat tggaagttgg 900
   ggtactttac cgcaggaaca tattgtacaa aaactcaagc aatgttttcg aaaattgcct 960
   gtaaatagac ctattgattg gaaagtatgt caaagaattg tgggtctttt gggctttgct 1020
   gcccctttta cacaatgtgg ctatcctgcc ttgatgcctt tatatgcatg tatacaatct 1080
   aagcaggctt tcactttctc gccaacttac aaggcctttc tgtgtaaaca atatctaaac 1140
   ctttaccccg ttgcccggca acggtcaggt ctctgccaag tgtttgctga cgcaaccccc 1200
   acgggttggg gcttggccat aggccatcgg cgcatgcgtg gaacctttgt ggctcctctg 1260
   ccgatccata ctgcggaact cctagcagct tgttttgctc gcagccggtc tggagcgaaa 1320
   cttatcggaa ccgacaactc agttgtcctc tctcggaaat acacctcctt tccatggctg 1380
   ctaggctgtg ctgccaactg gatcctgcgc gggacgtcct ttgtctacgt cccgtcggcg 1440
   ctgaatcccg cggacgaccc gtctcggggc cgtttgggcc tctaccgtcc ccttcttcat 1500
   ctgccgttcc ggccgaccac ggggcgcacc tctctttacg cggtctcccc gtctgtgcct 1560
   tctcatctgc cggaccgtgt gcacttcgct tcacctctgc acgtagcatg gagaccaccg 1620
   tgaacgccca ccaggtcttg cccaaggtct tacacaagag gactcttgga ctctcagcaa 1680
   tgtcaacgac cgaccttgag gcatacttca aagactgttt gtttaaagac tgggaggagt 1740
   tgggggagga gattaggtta aaggtctttg tactaggagg ctgtaggcat aaattggtct 1800
   gttcaccagc accatgcaac tttttcccct ctgcctaatc atctcatgtt catgtcctac 1860
   tgttcaagcc tccaagctgt gccttgggtg gctttggggc atggacattg acccgtataa 1920
   agaatttgga gcttctgtgg agttactctc ttttttgcct tctgacttct ttccttctat 1980
   tcgagatctc ctcgacaccg cctctgctct gtatcgggag gccttagagt ctccggaaca 2040
   ttgttcacct caccatacag cactcaggca agctattctg tgttggggtg agttgatgaa 2100
   tctggccacc tgggtgggaa gtaatttgga agacccagca tccagggaat tagtagtcag 2160
   ctatgtcaat gttaatatgg gcctaaaaat tagacaacta ttgtggtttc acatttcctg 2220
   ccttactttt ggaagagaaa ctgtccttga gtatttggtg tcttttggag tgtggattcg 2280
   cactcctccc gcttacagac caccaaatgc ccctatctta tcaacacttc cggaaactac 2340
   tgttgttaga cgacgaggca ggtcccctag aagaagaact ccctcgcctc gcagacgaag 2400
   gtctcaatcg ccgcgtcgca gaagatctca atctcgggaa tctcaatgtt agtatccctt 2460
   ggactcataa ggtgggaaac tttactgggc tttattcttc tactgtacct gtctttaatc 2520
   ctgattggaa aactccctcc tttcctcaca ttcatttaca ggaggacatt attaatagat 2580
   gtcaacaata tgtgggccct ctgacagtta atgaaaaaag gagattaaaa ttaattatgc 2640
   ctgctaggtt ctatcctaac cttaccaaat atttgccctt ggacaaaggc attaaaccgt 2700
   attatcctga atatgcagtt aatcattact tcaaaactag gcattattta catactctgt 2760
   ggaaggctgg cattctatat aagagagaaa ctacacgcag cgcctcattt tgtgggtcac 2820
   catattcttg ggaacaagag ctacagcatg ggaggttggt cttccaaacc tcgacaaggc 2880
   atggggacga atctttctgt tcccaatcct ctgggattct ttcccgatca ccagttggac 2940
   cctgcgttcg gagccaactc aaacaatcca gattgggact tcaaccccaa caaggatcac 3000
   tggccagagg caaatcaggt aggagcggga gcatttggtc cagggttcac cccaccacac 3060
   ggaggccttt tggggtggag ccctcaggct cagggcatat tgacaacact gccagcagca 3120
   cctcctcctg cctccaccaa tcggcagtca ggaagacagc ctactcccat ctctccacct 3180
   ctaagagaca gtcatcctca ggccatgcag tggaa 3215
<210> 20
   <211> 7905
   <212> DNA
   <213> Human Papilloma Virus HPV16 (NC_001526.2)
<400> 20
   actacaataa ttcatgtata aaactaaggg cgtaaccgaa atcggttgaa ccgaaaccgg     60
   ttagtataaa agcagacatt ttatgcacca aaagagaact gcaatgtttc aggacccaca    120
   ggagcgaccc agaaagttac cacagttatg cacagagctg caaacaacta tacatgatat    180
   aatattagaa tgtgtgtact gcaagcaaca gttactgcga cgtgaggtat atgactttgc    240
   ttttcgggat ttatgcatag tatatagaga tgggaatcca tatgctgtat gtgataaatg    300
   tttaaagttt tattctaaaa ttagtgagta tagacattat tgttatagtt tgtatggaac    360
   aacattagaa cagcaataca acaaaccgtt gtgtgatttg ttaattaggt gtattaactg    420
   tcaaaagcca ctgtgtcctg aagaaaagca aagacatctg gacaaaaagc aaagattcca    480
   taatataagg ggtcggtgga ccggtcgatg tatgtcttgt tgcagatcat caagaacacg    540
   tagagaaacc cagctgtaat catgcatgga gatacaccta cattgcatga atatatgtta    600
   gatttgcaac cagagacaac tgatctctac tgttatgagc aattaaatga cagctcagag    660
   gaggaggatg aaatagatgg tccagctgga caagcagaac cggacagagc ccattacaat    720
   attgtaacct tttgttgcaa gtgtgactct acgcttcggt tgtgcgtaca aagcacacac    780
   gtagacattc gtactttgga agacctgtta atgggcacac taggaattgt gtgccccatc    840
   tgttctcaga aaccataatc taccatggct gatcctgcag gtaccaatgg ggaagagggt    900
   acgggatgta atggatggtt ttatgtagag gctgtagtgg aaaaaaaaac aggggatgct    960
   atatcagatg acgagaacga aaatgacagt gatacaggtg aagatttggt agattttata   1020
   gtaaatgata atgattattt aacacaggca gaaacagaga cagcacatgc gttgtttact   1080
   gcacaggaag caaaacaaca tagagatgca gtacaggttc taaaacgaaa gtatttggta   1140
   gtccacttag tgatattagt ggatgtgtag acaataatat tagtcctaga ttaaaagcta  1200
   tatgtataga aaaacaaagt agagctgcaa aaaggagatt atttgaaagc gaagacagcg   1260
   ggtatggcaa tactgaagtg gaaactcagc agatgttaca ggtagaaggg cgccatgaga   1320
   ctgaaacacc atgtagtcag tatagtggtg gaagtggggg tggttgcagt cagtacagta   1380
   gtggaagtgg gggagagggt gttagtgaaa gacacactat atgccaaaca ccacttacaa   1440
   atattttaaa tgtactaaaa actagtaatg caaaggcagc aatgttagca aaatttaaag   1500
   agttatacgg ggtgagtttt tcagaattag taagaccatt taaaagtaat aaatcaacgt   1560
   gttgcgattg gtgtattgct gcatttggac ttacacccag tatagctgac agtataaaaa   1620
   cactattaca acaatattgt ttatatttac acattcaaag tttagcatgt tcatggggaa 1680
   tggttgtgtt actattagta agatataaat gtggaaaaaa tagagaaaca attgaaaaat 1740
   tgctgtctaa actattatgt gtgtctccaa tgtgtatgat gatagagcct ccaaaattgc 1800
   gtagtacagc agcagcatta tattggtata aaacaggtat atcaaatatt agtgaagtgt 1860
   atggagacac gccagaatgg atacaaagac aaacagtatt acaacatagt tttaatgatt 1920
   gtacatttga attatcacag atggtacaat gggcctacga taatgacata gtagacgata 1980
   gtgaaattgc atataaatat gcacaattgg cagacactaa tagtaatgca agtgcctttc 2040
   taaaaagtaa ttcacaggca aaaattgtaa aggattgtgc aacaatgtgt agacattata 2100
   aacgagcaga aaaaaaacaa atgagtatga gtcaatggat aaaatataga tgtgataggg 2160
   tagatgatgg aggtgattgg aagcaaattg ttatgttttt aaggtatcaa ggtgtagagt 2220
   ttatgtcatt tttaactgca ttaaaaagat ttttgcaagg catacctaaa aaaaattgca 2280
   tattactata tggtgcagct aacacaggta aatcattatt tggtatgagt ttaatgaaat 2340
   ttctgcaagg gtctgtaata tgttttgtaa attctaaaag ccatttttgg ttacaaccat 2400
   tagcagatgc caaaataggt atgttagatg atgctacagt gccctgttgg aactacatag 2460
   atgacaattt aagaaatgca ttggatggaa atttagtttc tatggatgta aagcatagac 2520
   cattggtaca actaaaatgc cctccattat taattacatc taacattaat gctggtacag 2580
   attctaggtg gccttattta cataatagat tggtggtgtt tacatttcct aatgagtttc 2640
   catttgacga aaacggaaat ccagtgtatg agcttaatga taagaactgg aaatcctttt 2700
   tctcaaggac gtggtccaga ttaagtttgc acgaggacga ggacaaggaa aacgatggag 2760
   actctttgcc aacgtttaaa tgtgtgtcag gacaaaatac taacacatta tgaaaatgat 2820
   agtacagacc tacgtgacca tatagactat tggaaacaca tgcgcctaga atgtgctatt 2880
   tattacaagg ccagagaaat gggatttaaa catattaacc accaagtggt gccaacactg 2940
   gctgtatcaa agaataaagc attacaagca attgaactgc aactaacgtt agaaacaata 3000
   tataactcac aatatagtaa tgaaaagtgg acattacaag acgttagcct tgaagtgtat 3060
   ttaactgcac caacaggatg tataaaaaaa catggatata cagtggaagt gcagtttgat 3120
   ggagacatat gcaatacaat gcattataca aactggacac atatatatat ttgtgaagaa 3180
   gcatcagtaa ctgtggtaga gggtcaagtt gactattatg gtttatatta tgttcatgaa 3240
   ggaatacgaa catattttgt gcagtttaaa gatgatgcag aaaaatatag taaaaataaa 3300
   gtatgggaag ttcatgcggg tggtcaggta atattatgtc ctacatctgt gtttagcagc 3360
   aacgaagtat cctctcctga aattattagg cagcacttgg ccaaccaccc cgccgcgacc 3420
   cataccaaag ccgtcgcctt gggcaccgaa gaaacacaga cgactatcca gcgaccaaga 3480
   tcagagccag acaccggaaa cccctgccac accactaagt tgttgcacag agactcagtg 3540
   gacagtgctc caatcctcac tgcatttaac agctcacaca aaggacggat taactgtaat 3600
   agtaacacta cacccatagt acatttaaaa ggtgatgcta atactttaaa atgtttaaga 3660
   tatagattta aaaagcattg tacattgtat actgcagtgt cgtctacatg gcattggaca 3720
   ggacataatg taaaacataa aagtgcaatt gttacactta catatgatag tgaatggcaa 3780
   cgtgaccaat ttttgtctca agttaaaata ccaaaaacta ttacagtgtc tactggattt 3840
   atgtctatat gacaaatctt gatactgcat ccacaacatt actggcgtgc tttttgcttt 3900
   gcttttgtgt gcttttgtgt gtctgcctat taatacgtcc gctgcttttg tctgtgtcta 3960
   catacacatc attaataata ttggtattac tattgtggat aacagcagcc tctgcgttta 4020
   ggtgttttat tgtatatatt atatttgttt atataccatt atttttaata catacacatg 4080
   cacgcttttt aattacataa tgtatatgta cataatgtaa ttgttacata taattgttgt 4140
   ataccataac ttactatttt ttctttttta ttttcatata taattttttt ttttgtttgt 4200
   ttgtttgttt tttaataaac tgttattact taacaatgcg acacaaacgt tctgcaaaac 4260
   gcacaaaacg tgcatcggct acccaacttt ataaaacatg caaacaggca ggtacatgtc 4320
   cacctgacat tatacctaag gttgaaggca aaactattgc tgaacaaata ttacaatatg 4380
   gaagtatggg tgtatttttt ggtgggttag gaattggaac agggtcgggt acaggcggac 4440
   gcactgggta tattccattg ggaacaaggc ctcccacagc tacagataca cttgctcctg 4500
   taagaccccc tttaacagta gatcctgtgg gcccttctga tccttctata gtttctttag 4560
   tggaagaaac tagttttatt gatgctggtg caccaacatc tgtaccttcc attcccccag 4620
   atgtatcagg atttagtatt actacttcaa ctgataccac acctgctata ttagatatta 4680
   ataatactgt tactactgtt actacacata ataatcccac tttcactgac ccatctgtat 4740
   tgcagcctcc aacacctgca gaaactggag ggcattttac actttcatca tccactatta 4800
   gtacacataa ttatgaagaa attcctatgg atacatttat tgttagcaca aaccctaaca 4860
   cagtaactag tagcacaccc ataccagggt ctcgcccagt ggcacgccta ggattatata 4920
   gtcgcacaac acaacaggtt aaagttgtag accctgcttt tgtaaccact cccactaaac 4980
   ttattacata tgataatcct gcatatgaag gtatagatgt ggataataca ttatattttt 5040
   ctagtaatga taatagtatt aatatagctc cagatcctga ctttttggat atagttgctt 5100
   tacataggcc agcattaacc tctaggcgta ctggcattag gtacagtaga attggtaata 5160
   aacaaacact acgtactcgt agtggaaaat ctataggtgc taaggtacat tattattatg 5220
   atttaagtac tattgatcct gcagaagaaa tagaattaca aactataaca ccttctacat 5280
   atactaccac ttcacatgca gcctcaccta cttctattaa taatggatta tatgatattt 5340
   atgcagatga ctttattaca gatacttcta caaccccggt accatctgta ccctctacat 5400
   ctttatcagg ttatattcct gcaaatacaa caattccttt tggtggtgca tacaatattc 5460
   ctttagtatc aggtcctgat atacccatta atataactga ccaagctcct tcattaattc 5520
   ctatagttcc agggtctcca caatatacaa ttattgctga tgcaggtgac ttttatttac 5580
   atcctagtta ttacatgtta cgaaaacgac gtaaacgttt accatatttt ttttcagatg 5640
   tctctttggc tgcctagtga ggccactgtc tacttgcctc ctgtcccagt atctaaggtt 5700
   gtaagcacgg atgaatatgt tgcacgcaca aacatatatt atcatgcagg aacatccaga 5760
   ctacttgcag ttggacatcc ctattttcct attaaaaaac ctaacaataa caaaatatta 5820
   gttcctaaag tatcaggatt acaatacagg gtatttagaa tacatttacc tgaccccaat 5880
   aagtttggtt ttcctgacac ctcattttat aatccagata cacagcggct ggtttgggcc 5940
   tgtgtaggtg ttgaggtagg tcgtggtcag ccattaggtg tgggcattag tggccatcct 6000
   ttattaaata aattggatga cacagaaaat gctagtgctt atgcagcaaa tgcaggtgtg 6060
   gataatagag aatgtatatc tatggattac aaacaaacac aattgtgttt aattggttgc 6120
   aaaccaccta taggggaaca ctggggcaaa ggatccccat gtaccaatgt tgcagtaaat 6180
   ccaggtgatt gtccaccatt agagttaata aacacagtta ttcaggatgg tgatatggtt 6240
   catactggct ttggtgctat ggactttact acattacagg ctaacaaaag tgaagttcca 6300
   ctggatattt gtacatctat ttgcaaatat ccagattata ttaaaatggt gtcagaacca 6360
   tatggcgaca gcttattttt ttatttacga agggaacaaa tgtttgttag acatttattt 6420
   aatagggctg gtactgttgg tgaaaatgta ccagacgatt tatacattaa aggctctggg 6480
   tctactgcaa atttagccag ttcaaattat tttcctacac ctagtggttc tatggttacc 6540
   tctgatgccc aaatattcaa taaaccttat tggttacaac gagcacaggg ccacaataat 6600
   ggcatttgtt ggggtaacca actatttgtt actgttgttg atactacacg cagtacaaat 6660
   atgtcattat gtgctgccat atctacttca gaaactacat ataaaaatac taactttaag 6720
   gagtacctac gacatgggga ggaatatgat ttacagttta tttttcaact gtgcaaaata 6780
   accttaactg cagacgttat gacatacata cattctatga attccactat tttggaggac 6840
   tggaattttg gtctacaacc tcccccagga ggcacactag aagatactta taggtttgta 6900
   acccaggcaa ttgcttgtca aaaacataca cctccagcac ctaaagaaga tgatcccctt 6960
   aaaaaataca ctttttggga agtaaattta aaggaaaagt tttctgcaga cctagatcag 7020
   tttcctttag gacgcaaatt tttactacaa gcaggattga aggccaaacc aaaatttaca 7080
   ttaggaaaac gaaaagctac acccaccacc tcatctacct ctacaactgc taaacgcaaa 7140
   aaacgtaagc tgtaagtatt gtatgtatgt tgaattagtg ttgtttgttg tgtatatgtt 7200
   tgtatgtgct tgtatgtgct tgtaaatatt aagttgtatg tgtgtttgta tgtatggtat 7260
   aataaacacg tgtgtatgtg tttttaaatg cttgtgtaac tattgtgtca tgcaacataa 7320
   ataaacttat tgtttcaaca cctactaatt gtgttgtggt tattcattgt atataaacta 7380
   tatttgctac atcctgtttt tgttttatat atactatatt ttgtagcgcc aggcccattt 7440
   tgtagcttca accgaattcg gttgcatgct ttttggcaca aaatgtgttt ttttaaatag 7500
   ttctatgtca gcaactatgg tttaaacttg tacgtttcct gcttgccatg cgtgccaaat 7560
   ccctgttttc ctgacctgca ctgcttgcca accattccat tgttttttac actgcactat 7620
   gtgcaactac tgaatcacta tgtacattgt gtcatataaa ataaatcact atgcgccaac 7680
   gccttacata ccgctgttag gcacatattt ttggcttgtt ttaactaacc taattgcata 7740
   tttggcataa ggtttaaact tctaaggcca actaaatgtc accctagttc atacatgaac 7800
   tgtgtaaagg ttagtcatac attgttcatt tgtaaaactg cacatgggtg tgtgcaaacc 7860
   gattttgggt tacacattta caagcaactt atataataat actaa 7905
<210> 21
   <211> 7857
   <212> DNA
   <213> Human Papilloma Virus HPV18 (X05015.1)
<400> 21
   attaatactt ttaacaattg tagtatataa aaaagggagt aaccgaaaac ggtcgggacc      60
   gaaaacggtg tatataaaag atgtgagaaa cacaccacaa tactatggcg cgctttgagg     120
   atccaacacg gcgaccctac aagctacctg atctgtgcac ggaactgaac acttcactgc     180
   aagacataga aataacctgt gtatattgca agacagtatt ggaacttaca gaggtatttg     240
   aatttgcatt taaagattta tttgtggtgt atagagacag tataccccat gctgcatgcc     300
   ataaatgtat agatttttat tctagaatta gagaattaag acattattca gactctgtgt     360
   atggagacac attggaaaaa ctaactaaca ctgggttata caatttatta ataaggtgcc     420
   tgcggtgcca gaaaccgttg aatccagcag aaaaacttag acaccttaat gaaaaacgac     480
   gatttcacaa catagctggg cactatagag gccagtgcca ttcgtgctgc aaccgagcac     540
   gacaggaacg actccaacga cgcagagaaa cacaagtata atattaagta tgcatggacc     600
   taaggcaaca ttgcaagaca ttgtattgca tttagagccc caaaatgaaa ttccggttga     660
   ccttctatgt cacgagcaat taagcgactc agaggaagaa aacgatgaaa tagatggagt     720
   taatcatcaa catttaccag cccgacgagc cgaaccacaa cgtcacacaa tgttgtgtat     780
   gtgttgtaag tgtgaagcca gaattgagct agtagtagaa agctcagcag acgaccttcg     840
   agcattccag cagctgtttc tgaacaccct gtcctttgtg tgtccgtggt gtgcatccca     900
   gcagtaagca acaatggctg atccagaagg tacagacggg gagggcacgg gttgtaacgg     960
   ctggttttat gtacaagcta ttgtagacaa aaaaacagga gatgtaatat cagatgacga    1020
   ggacgaaaat gcaacagaca cagggtcgga tatggtagat tttattgata cacaaggaac    1080
   attttgtgaa caggcagagc tagagacagc acaggcattg ttccatgcgc aggaggtcca   1140
   caatgatgca caagtgttgc atgttttaaa acgaaagttt gcaggaggca gcacagaaaa   1200
   cagtccatta ggggagcggc tggaggtgga tacagagtta agtccacggt tacaagaaat   1260
   atctttaaat agtgggcaga aaaaggcaaa aaggcggctg tttacaatat cagatagtgg   1320
   ctatggctgt tctgaagtgg aagcaacaca gattcaggta actacaaatg gcgaacatgg   1380
   cggcaatgta tgtagtggcg gcagtacgga ggctatagac aacgggggca cagagggcaa   1440
   caacagcagt gtagacggta caagtgacaa tagcaatata gaaaatgtaa atccacaatg   1500
   taccatagca caattaaaag acttgttaaa agtaaacaat aaacaaggag ctatgttagc   1560
   agtatttaaa gacacatatg ggctatcatt tacagattta gttagaaatt ttaaaagtga   1620
   taaaaccacg tgtacagatt gggttacagc tatatttgga gtaaacccaa caatagcaga   1680
   aggatttaaa acactaatac agccatttat attatatgcc catattcaat gtctagactg   1740
   taaatgggga gtattaatat tagccctgtt gcgttacaaa tgtggtaaga gtagactaac   1800
   agttgctaaa ggtttaagta cgttgttaca cgtacctgaa acttgtatgt taattcaacc   1860
   accaaaattg cgaagtagtg ttgcagcact atattggtat agaacaggaa tatcaaatat   1920
   tagtgaagta atgggagaca cacctgagtg gatacaaaga cttactatta tacaacatgg   1980
   aatagatgat agcaattttg atttgtcaga aatggtacaa tgggcatttg ataatgagct   2040
   gacagatgaa agcgatatgg catttgaata tgccttatta gcagacagca acagcaatgc   2100
   agctgccttt ttaaaaagca attgccaagc taaatattta aaagattgtg ccacaatgtg   2160
   caaacattat aggcgagccc aaaaacgaca aatgaatatg tcacagtgga tacgatttag   2220
   atgttcaaaa atagatgaag ggggagattg gagaccaata gtgcaattcc tgcgatacca   2280
   acaaatagag tttataacat ttttaggagc cttaaaatca tttttaaaag gaacccccaa   2340
   aaaaaattgt ttagtatttt gtggaccagc aaatacagga aaatcatatt ttggaatgag   2400
   ttttatacac tttatacaag gagcagtaat atcatttgtg aattccacta gtcatttttg   2460
   gttggaaccg ttaacagata ctaaggtggc catgttagat gatgcaacga ccacgtgttg   2520
   gacatacttt gatacctata tgagaaatgc gttagatggc aatccaataa gtattgatag   2580
   aaagcacaaa ccattaatac aactaaaatg tcctccaata ctactaacca caaatataca   2640
   tccagcaaag gataatagat ggccatattt agaaagtaga ataacagtat ttgaatttcc   2700
   aaatgcattt ccatttgata aaaatggcaa tccagtatat gaaataaatg acaaaaattg   2760
   gaaatgtttt tttgaaagga catggtccag attagatttg cacgaggaag aggaagatgc   2820
   agacaccgaa ggaaaccctt tcggaacgtt taagttgcgt gcaggacaaa atcatagacc   2880
   actatgaaaa tgacagtaaa gacatagaca gccaaataca gtattggcaa ctaatacgtt   2940
   gggaaaatgc aatattcttt gcagcaaggg aacatggcat acagacatta aaccaccagg 3000
   tggtgccagc ctataacatt tcaaaaagta aagcacataa agctattgaa ctgcaaatgg 3060
   ccctacaagg ccttgcacaa agtcgataca aaaccgagga ttggacactg caagacacat 3120
   gcgaggaact atggaataca gaacctactc actgctttaa aaaaggtggc caaacagtac 3180
   aagtatattt tgatggcaac aaagacaatt gtatgaccta tgtagcatgg gacagtgtgt 3240
   attatatgac tgatgcagga acatgggaca aaaccgctac ctgtgtaagt cacaggggat 3300
   tgtattatgt aaaggaaggg tacaacacgt tttatataga atttaaaagt gaatgtgaaa 3360
   aatatgggaa cacaggtacg tgggaagtac attttgggaa taatgtaatt gattgtaatg 3420
   actctatgtg cagtaccagt gacgacacgg tatccgctac tcagcttgtt aaacagctac 3480
   agcacacccc ctcaccgtat tccagcaccg tgtccgtggg caccgcaaag acctacggcc 3540
   agacgtcggc tgctacacga cctggacact gtggactcgc ggagaagcag cattgtggac 3600
   ctgtcaaccc acttctcggt gcagctacac ctacaggcaa caacaaaaga cggaaactct 3660
   gtagtggtaa cactacgcct ataatacatt taaaaggtga cagaaacagt ttaaaatgtt 3720
   tacggtacag attgcgaaaa catagcgacc actatagaga tatatcatcc acctggcatt 3780
   ggacaggtgc aggcaatgaa aaaacaggaa tactgactgt aacataccat agtgaaacac 3840
   aaagaacaaa atttttaaat actgttgcaa ttccagatag tgtacaaata ttggtgggat 3900
   acatgacaat gtaatacata tgctgtagta ccaatatgtt atcacttatt tttttatttt 3960
   gcttttgtgt atgcatgtat gtgtgctgcc atgtcccgct tttgccatct gtctgtatgt 4020
   gtgcgtatgc atgggtattg gtatttgtgt atattgtggt aataacgtcc cctgccacag 4080
   cattcacagt atatgtattt tgttttttat tgcccatgtt actattgcat atacatgcta 4140
   tattgtcttt acagtaattg tataggttgt tttatacagt gtattgtaca ttgtatattt 4200
   tgttttatac cttttatgct ttttgtattt ttgtaataaa agtatggtat cccaccgtgc 4260
   cgcacgacgc aaacgggctt cggtaactga cttatataaa acatgtaaac aatctggtac 4320
   atgtccacct gatgttgttc ctaaggtgga gggcaccacg ttagcagata aaatattgca 4380
   atggtcaagc cttggtatat ttttgggtgg acttggcata ggtactggca gtggtacagg 4440
   gggtcgtaca gggtacattc cattgggtgg gcgttccaat acagtggtgg atgttggtcc 4500
   tacacgtccc ccagtggtta ttgaacctgt gggccccaca gacccatcta ttgttacatt 4560
   aatagaggac tccagtgtgg ttacatcagg tgcacctagg cctacgttta ctggcacgtc 4620
   tgggtttgat ataacatctg cgggtacaac tacacctgcg gttttggata tcacaccttc 4680
   gtctacctct gtgtctattt ccacaaccaa ttttaccaat cctgcatttt ctgatccgtc 4740
   cattattgaa gttccacaaa ctggggaggt ggcaggtaat gtatttgttg gtacccctac 4800
   atctggaaca catgggtatg aggaaatacc tttacaaaca tttgcttctt ctggtacggg 4860
   ggaggaaccc attagtagta ccccattgcc tactgtgcgg cgtgtagcag gtccccgcct 4920
   ttacagtagg gcctaccaac aagtgtcagt ggctaaccct gagtttctta cacgtccatc 4980
   ctctttaatt acatatgaca acccggcctt tgagcctgtg gacactacat taacatttga 5040
   tcctcgtagt gatgttcctg attcagattt tatggatatt atccgtctac ataggcctgc 5100
   tttaacatcc aggcgtggga ctgttcgctt tagtagatta ggtcaacggg caactatgtt 5160
   tacccgcagc ggtacacaaa taggtgctag ggttcacttt tatcatgata taagtcctat 5220
   tgcaccttcc ccagaatata ttgaactgca gcctttagta tctgccacgg aggacaatga 5280
   cttgtttgat atatatgcag atgacatgga ccctgcagtg cctgtaccat cgcgttctac 5340
   tacctccttt gcatttttta aatattcgcc cactatatct tctgcctctt cctatagtaa 5400
   tgtaacggtc cctttaacct cctcttggga tgtgcctgta tacacgggtc ctgatattac 5460
   attaccatct actacctctg tatggcccat tgtatcaccc acggcccctg cctctacaca 5520
   gtatattggt atacatggta cacattatta tttgtggcca ttatattatt ttattcctaa 5580
   gaaacgtaaa cgtgttccct atttttttgc agatggcttt gtggcggcct agtgacaata 5640
   ccgtatatct tccacctcct tctgtggcaa gagttgtaaa taccgatgat tatgtgactc 5700
   ccacaagcat attttatcat gctggcagct ctagattatt aactgttggt aatccatatt 5760
   ttagggttcc tgcaggtggt ggcaataagc aggatattcc taaggtttct gcataccaat 5820
   atagagtatt tagggtgcag ttacctgacc caaataaatt tggtttacct gatactagta 5880
   tttataatcc tgaaacacaa cgtttagtgt gggcctgtgc tggagtggaa attggccgtg 5940
   gtcagccttt aggtgttggc cttagtgggc atccatttta taataaatta gatgacactg 6000
   aaagttccca tgccgccacg tctaatgttt ctgaggacgt tagggacaat gtgtctgtag 6060
   attataagca gacacagtta tgtattttgg gctgtgcccc tgctattggg gaacactggg 6120
   ctaaaggcac tgcttgtaaa tcgcgtcctt tatcacaggg cgattgcccc cctttagaac 6180
   ttaaaaacac agttttggaa gatggtgata tggtagatac tggatatggt gccatggact 6240
   ttagtacatt gcaagatact aaatgtgagg taccattgga tatttgtcag tctatttgta 6300
   aatatcctga ttatttacaa atgtctgcag atccttatgg ggattccatg tttttttgct 6360
   tacggcgtga gcagcttttt gctaggcatt tttggaatag agcaggtact atgggtgaca 6420
   ctgtgcctca atccttatat attaaaggca caggtatgcc tgcttcacct ggcagctgtg 6480
   tgtattctcc ctctccaagt ggctctattg ttacctctga ctcccagttg tttaataaac 6540
   catattggtt acataaggca cagggtcata acaatggtgt ttgctggcat aatcaattat 6600
   ttgttactgt ggtagatacc actcccagta ccaatttaac aatatgtgct tctacacagt 6660
   ctcctgtacc tgggcaatat gatgctacca aatttaagca gtatagcaga catgttgagg 6720
   aatatgattt gcagtttatt tttcagttgt gtactattac tttaactgca gatgttatgt 6780
   cctatattca tagtatgaat agcagtattt tagaggattg gaactttggt gttccccccc 6840
   ccccaactac tagtttggtg gatacatatc gttttgtaca atctgttgct attacctgtc 6900
   aaaaggatgc tgcaccggct gaaaataagg atccctatga taagttaaag ttttggaatg 6960
   tggatttaaa ggaaaagttt tctttagact tagatcaata tccccttgga cgtaaatttt 7020
   tggttcaggc tggattgcgt cgcaagccca ccataggccc tcgcaaacgt tctgctccat 7080
   ctgccactac gtcttctaaa cctgccaagc gtgtgcgtgt acgtgccagg aagtaatatg 7140
   tgtgtgtgta tatatatata catctattgt tgtgtttgta tgtcctgtgt ttgtgtttgt 7200
   tgtatgattg cattgtatgg tatgtatggt tgttgttgta tgttgtatgt tactatattt 7260
   gttggtatgt ggcattaaat aaaatatgtt ttgtggttct gtgtgttatg tggttgcgcc 7320
   ctagtgagta acaactgtat ttgtgtttgt ggtatgggtg ttgcttgttg ggctatatat 7380
   tgtcctgtat ttcaagttat aaaactgcac accttacagc atccatttta tcctacaatc 7440
   ctccattttg ctgtgcaacc gatttcggtt gcctttggct tatgtctgtg gttttctgca 7500
   caatacagta cgctggcact attgcaaact ttaatctttt gggcactgct cctacatatt 7560
   ttgaacaatt ggcgcgcctc tttggcgcat ataaggcgca cctggtatta gtcattttcc 7620
   tgtccaggtg cgctacaaca attgcttgca taactatatc cactccctaa gtaataaaac 7680
   tgcttttagg cacatatttt agtttgtttt tacttaagct aattgcatac ttggcttgta 7740
   caactacttt catgtccaac attctgtcta cccttaacat gaactataat atgactaagc 7800
   tgtgcataca tagtttatgc aaccgaaata ggttgggcag cacatactat acttttc 7857
<210> 22
   <211> 7912
   <212> DNA
   <213> Human Papilloma Virus HPV31 (J04353.1)
<400> 22
   taataataat aatcttagta taaaaaagta gggagtgacc gaaagtggtg aaccgaaaac 60
   ggttggtata taaagcacat agtattttgt gcaaacctac agacgccatg ttcaaaaatc 120
   ctgcagaaag acctcggaaa ttgcatgaac taagctcggc attggaaata ccctacgatg 180
   aactaagatt gaattgtgtc tactgcaaag gtcagttaac agaaacagag gtattagatt 240
   ttgcatttac agatttaaca atagtatata gggacgacac accacacgga gtgtgtacaa 300
   aatgtttaag attttattca aaagtaagtg aatttagatg gtatagatat agtgtgtatg 360
   gaacaacatt agaaaaattg acaaacaaag gtatatgtga tttgttaatt aggtgtataa 420
   cgtgtcaaag accgttgtgt ccagaagaaa aacaaagaca tttggataaa aagaaacgat 480
   tccacaacat aggaggaagg tggacaggac gttgcatagc atgttggaga agacctcgta 540
   ctgaaaccca agtgtaaaca tgcgtggaga aacacctacg ttgcaagact atgtgttaga 600
   tttgcaacct gaggcaactg acctccactg ttatgagcaa ttacccgaca gctcagatga 660
   ggaggatgtc atagacagtc cagctggaca agcagaaccg gacacatcca attacaatat 720
   cgttaccttt tgttgtcagt gtaagtctac acttcgtttg tgtgtacaga gcacacaagt 780
   agatattcgc atattgcaag agctgttaat gggctcattt ggaatcgtgt gccccaactg 840
   ttctactaga ctgtaactac aatggctgat ccagcaggta cagatgggga ggggacggga 900
   tgcaatggtt ggttttatgt agaagcagta attgacagac agacagggga caacatttca 960
   gaggacgaaa atgaagacag tagtgatact ggggaggata tggttgactt tattgacaat 1020
   tgtaatgtat acaacaatca ggcagaagca gagacagcac aggcattgtt tcatgcacag 1080
   gaagcggagg aacatgcaga ggctgtgcag gttctaaaac gaaagtatgt aggtagtcct 1140
   ttaagtgata ttagtagttg tgtggattat aatattagtc cacggttaaa agctatatgc 1200
   atagaaaata acagtaaaac agcaaaacga agactctttg aacttccaga cagcgggtat 1260
   ggcaatactg aagtggaaac gcagcagatg gtacaggtag aggagcaaca aacaacatta 1320
   agttgtaatg gtagtgacgg gacacatagt gaacgagaga atgaaactcc aacacgtaat 1380
   atattgcaag tgttaaaaac tagcaatggt aaagctgcta tgttaggtaa atttaaagaa 1440
   ttatatggtg taagttttat ggaactaatt aggccatttc aaagcaataa aagcacatgt 1500
   actgattggt gtgtagctgc gtttggagtt acaggtacag ttgcagaagg atttaaaacc 1560
   ctattgcaac catattgttt gtattgccat ttacaaagtt tagcatgttc ctggggcatg 1620
   gttatgttaa tgcttgtgag atttaaatgt gcaaaaaata gaataacaat tgaaaaatta 1680
   ttagaaaaat tattgtgtat atctacaaat tgtatgttaa ttcagccacc caaattacgt 1740
   agcacagctg cagcattata ttggtacaga acaggaatgt caaacattag cgatgtatat 1800
   ggtgaaacac cagaatggat agaaagacaa acagtattac agcatagttt taatgacaca 1860
   acatttgatt tgtcccaaat ggtacaatgg gcatatgaca atgatgttat ggatgatagt 1920
   gaaattgcct ataaatatgc acaattagct gacagtgata gtaatgcatg tgcattttta 1980
   aaaagtaatt cgcaggcaaa aatagttaaa gattgtggaa caatgtgtag acattataaa 2040
   cgagcagaaa aacgacaaat gtccatggga cagtggatta aaagtagatg tgacaaagtt 2100
   agtgacgaag gtgactggag ggacatagta aagtttttaa gatatcaaca aatagaattt 2160
   gtgtcatttt tatctgcatt aaagctgttt ttaaaaggag tgccaaagaa aaactgtatt 2220
   ttaatacatg gtgcacctaa tacaggtaaa tcatattttg gaatgagcct tattagcttt 2280
   ttacaaggat gtataatatc atatgcaaat tcaaaaagtc atttttggtt acaaccactg 2340
   gctgatgcta aaataggcat gttagatgat gctacaacgc catgttggca ttatatagac 2400
   aattacctac gaaatgcact agatggcaac cctgtatcta tagatgtaaa gcataaagct 2460
   ttaatgcagt taaaatgtcc tcctttattg attacatcta atataaatgc aggtaaggat 2520
   gacagatggc catacctaca tagcagactg gtggttttta catttccaaa tccatttcca 2580
   tttgacaaaa acggaaatcc agtatatgaa ttaagtgata aaaactggaa atcctttttc 2640
   tcaaggacgt ggtgcagatt aaatttgcac gaggaagagg acaaagaaaa cgatggagac 2700
   tctttctcaa cgtttaaatg tgtgtcagga caaaatatta gaacattatg aaaatgatag 2760
   taaacgactt tgtgatcata tagactattg gaaacatatt cgacttgaat gtgtattaat 2820
   gtataaagca agagaaatgg gaatacacag tattaaccac caggtggtgc cagcgttgtc 2880
   agtatcaaag gccaaagcct tacaagctat tgaactacaa atgatgttgg aaacattaaa 2940
   taacactgaa tacaaaaatg aggactggac aatgcagcaa acaagtcttg aactgtattt 3000
   aactgcacct acagggtgtt taaaaaaaca tggatatact gtagaggtgc aatttgatgg 3060
   tgatgtacac aacaccatgc attatactaa ctggaaattt atatacctat gtatagatgg 3120
   ccaatgtact gttgtggaag ggcaagttaa ttgtaagggc atttattatg tacatgaagg 3180
   acatataaca tattttgtaa attttacaga agaggcaaaa aaatatggga ctggtaaaaa 3240
   atgggaagtg catgcgggtg gtcaggtaat tgtttttcct gaatctgtat ttagcagtga 3300
   cgaaatatcc tttgctggga ttgttacaaa gctaccaaca gccaacaaca ccaccacatc 3360
   gaattccaaa acctgcgcct tgggcaccag tgaaggtgtg cggcgggcga cgacgtctac 3420
   taagcgacca agaacagagc cagagcacag aaacacccac caccccaaca agttgttgcg 3480
   aggcgactcc gtggacagtg tcaactgtgg ggttatcagt gcagctgcat gcacaaacca 3540
   aacaagggct gtcagttgtc ctgcaactac acctataata cacttaaaag gtgatgcaaa 3600
   tatattaaaa tgtttaagat ataggctgtc aaaatataaa caattgtatg aacaagtgtc 3660
   atctacatgg cattggacat gtacagatgg aaaacataaa aatgctattg taaccttaac 3720
   atatataagt acatcacaaa gagacgattt tttaaatact gtaaaaatac ctaacacagt 3780
   atcagtgtca acaggatata tgactattta gcctaatgat tgaactaaat atttctacag 3840
   taagcattgt gctatgcttt ttgctttgct tttgtgtgct actatttgtg tgtcttgtca 3900
   tacgtccact tgtgctgtct gtgtcggtat atgcaacact actattatta attgtgattt 3960
   tatgggttat tgcaacctct ccattacgtt gtttttgtat atatgttgtg tttatatata 4020
   ttccattatt tgtaattcat acacatgcat cttttttaag tcaacagtaa cttttttact 4080
   tgtgtatact gttgtttgta ttggtattgg tattggtatt ggtattggta ttggtataat 4140
   aaactttttt actttttttt tattattacc atgcggtcca aacgctctac aaaacgcact 4200
   aaacgtgcgt ctgctacaca attatatcaa acatgtaaag cagcaggtac ttgtccatca 4260
   gacgttatac ctaaaataga acatactacc attgcagacc aaatattaag gtatggtagt 4320
   atgggtgttt tttttggtgg gttgggtatt gggtccggct ctggtactgg gggtcgcact     4380
   ggatatgtcc ctcttagtac acgtccttct acagtatctg aggcaagtat acctattaga     4440
   ccaccagtta gcattgaccc tgtaggtccc ttggacccct ctatagtaag tcttgttgaa     4500
   gaatctggaa ttgttgatgt tggtgcccct gctcctatac cacaccctcc tacaacatct     4560
   gggtttgaca ttgctacaac tgcagacaca acacctgcaa ttttagatgt aacaagtgtt     4620
   agcacacatg aaaatcctac ttttactgat ccatctgtat tgcagcctcc tacacctgca     4680
   gaaacatcag gtcatttact actttcatca tcatctatta gcacacataa ttatgaggaa     4740
   atacctatgg atacatttat tgtttctact aataatgaaa acataacaag tagcacaccc     4800
   attccagggg tgcgccgtcc tgcacgttta gggttatata gtaaggctac acaacaagta     4860
   aaagttattg atccaacgtt tcttagtgct ccaaaacagc taattacata tgaaaaccct     4920
   gcctatgaaa ctgtaaatgc tgaagaatct ttatactttt ccaatacatc gcataatata     4980
   gcccctgatc ccgactttct agatattata gcattacata ggcctgccct tacctcacgt     5040
   aggaacactg ttagatatag tagactaggt aataaacaaa ctttgcgcac tcgtagtggt     5100
   gctactattg gtgcaagggt gcattattat tatgatatta gtagtattaa tcctgcaggt     5160
   gaaagtattg aaatgcaacc tttaggggcg tctgcaacta ctacttctac tttaaatgat     5220
   ggcttatatg acatttatgc agacactgat tttactgtgg atacacctgc cacacataat     5280
   gtttcccctt ctactgctgt acagtccaca tctgctgtgt ctgcctatgt acctacaaat     5340
   accactgtgc cactaagtac aggttttgac attcccatat tttctgggcc tgatgtacct     5400
   atagagcatg cacctacaca ggttttccca tttcctttgg cccctacaac gccacaagtg     5460
   tctatttttg ttgatggggg tgatttttat ttgcacccta gttattatat gttaaaacgt     5520
   cgacgtaaac gtgtatcata tttttttaca gatgtctctg tggcggccta gcgaggctac     5580
   tgtctactta ccacctgtcc cagtgtctaa agttgtaagc acggatgaat atgtaacacg     5640
   aaccaacata tattatcacg caggcagtgc taggctgctt acagtaggcc atccatatta     5700
   ttccatacct aaatctgaca atcctaaaaa aatagttgta ccaaaggtgt caggattaca     5760
   atatagggta tttagggttc gtttaccaga tccaaacaaa tttggatttc ctgatacatc     5820
   tttttataat cctgaaactc aacgcttagt ttgggcctgt gttggtttag aggtaggtcg     5880
   cgggcagcca ttaggtgtag gtattagtgg tcatccatta ttaaataaat ttgatgacac     5940
   tgaaaactct aatagatatg ccggtggtcc tggcactgat aatagggaat gtatatcaat     6000
   ggattataaa caaacacaac tgtgtttact tggttgcaaa ccacctattg gagagcattg     6060
   gggtaaaggt agtccttgta gtaacaatgc tattacccct ggtgattgtc ctccattaga     6120
   attaaaaaat tcagttatac aagatgggga tatggttgat acaggctttg gagctatgga     6180
   ttttactgct ttacaagaca ctaaaagtaa tgttcctttg gacatttgta attctatttg     6240
   taaatatcca gattatctta aaatggttgc tgagccatat ggcgatacat tattttttta     6300
   tttacgtagg gaacaaatgt ttgtaaggca tttttttaat agatcaggca cggttggtga     6360
   atcggtccct actgacttat atattaaagg ctccggttca acagctactt tagctaacag     6420
   tacatacttt cctacaccta gcggctccat ggttacttca gatgcacaaa tttttaataa     6480
   accatattgg atgcaacgtg ctcagggaca caataatggt atttgttggg gcaatcagtt     6540
   atttgttact gtggtagata ccacacgtag taccaatatg tctgtttgtg ctgcaattgc     6600
   aaacagtgat actacattta aaagtagtaa ttttaaagag tatttaagac atggtgagga     6660
   atttgattta caatttatat ttcagttatg caaaataaca ttatctgcag acataatgac     6720
   atatattcac agtatgaatc ctgctatttt ggaagattgg aattttggat tgaccacacc     6780
   tccctcaggt tctttggagg atacctatag gtttgtcacc tcacaggcca ttacatgtca     6840
   aaaaactgcc ccccaaaagc ccaaggaaga tccatttaaa gattatgtat tttgggaggt     6900
   taatttaaaa gaaaagtttt ctgcagattt agatcagttt ccactgggtc gcaaattttt     6960
   attacaggca ggatataggg cacgtcctaa atttaaagca ggtaaacgta gtgcaccctc     7020
   agcatctacc actacaccag caaaacgtaa aaaaactaaa aagtaatgga tgtgtatgta     7080
   atacatgtgt ctgtatgtgt atgtgcttgt gctgtattgt atatgtgtgt gtttgtgtgt     7140
   tatatatggt atatgtatgt ttatgtatgc gtgtgtactt gtatatatgt atagtatgtt     7200
   atgtgtgtat gtatgctatg tatgttaata aatatgtgta tacctgtgtg tgttgtgtat     7260
   gttgtcctta tatacaccct attagtaaca tactattact attttataaa ctattgttcc     7320
   tacttgttcc tacttgttcc tgctcctccc aatagtcatg tacttatttc tgcctataat     7380
   ttaggtgtca cgccatagta aaagttgtac acccggtccg ttttttgcaa ctaaagctac     7440
   tccattttga ttttatgcag ccattttaaa tccctaaccg ttttcggttg cattgtttaa     7500
   acatgctagt acaactatgc tgatgcagta gttctgcggt tttggtttcc ctgaatacta     7560
   gtttttgcca acattctggc ttgtagtttc ctgcctaaca caccttgcca acatataatc     7620
   cagtccaact ttgcaattat actatgaatc atgtttgttt aaatacaact gtagttcaac     7680
   tatgtgtcat gcacatatat tatattatcc tacacacctt aaactgcttt taggcacata     7740
   ttttgtagat tatctatatc cttgattgca gtgctggctt ttgcacatgt ttaaactgcc     7800
   aaggttgtgt catgcattat aaataagttg tatgttactc atataattaa ttgcatatag     7860
   gtattacacc gttttcggtt acagttttac aagcaattgt tctttttata ct             7912
<210> 23
   <211> 7909
   <212> DNA
   <213> Human Papillomavirus HPV33 (M12732.1)
<400> 23
   gtaaactata atgccaagtt ttaaaaaagt agggtgtaac cgaaagcggt tcaaccgaaa 60
   acggtgcata tataaagcaa acattttgca gtaaggtact gcacgactat gtttcaagac 120
   actgaggaaa aaccacgaac attgcatgat ttgtgccaag cattggagac aactatacac 180
   aacattgaac tacagtgcgt ggaatgcaaa aaacctttgc aacgatctga ggtatatgat 240
   tttgcatttg cagatttaac agttgtatat agagagggaa atccatttgg aatatgtaaa 300
   ctgtgtttgc ggttcttatc taaaattagt gaatatagac attataatta ttctgtatat 360
   ggaaatacat tagaacaaac agttaaaaaa cctttaaatg aaatattaat taggtgtatt 420
   atatgtcaaa gacctttgtg tcctcaagaa aaaaaacgac atgtggattt aaacaaacga 480
   tttcataata tttcgggtcg ttgggcaggg cgctgtgcgg cgtgttggag gtcccgacgt 540
   agagaaactg cactgtgacg tgtaaaaacg ccatgagagg acacaagcca acgttaaagg 600
   aatatgtttt agatttatat cctgaaccaa ctgacctata ctgctatgag caattaagtg 660
   acagctcaga tgaggatgaa ggcttggacc ggccagatgg acaagcacaa ccagccacag 720
   ctgattacta cattgtaacc tgttgtcaca cttgtaacac cacagttcgt ttatgtgtca 780
   acagtacagc aagtgaccta cgaaccatac agcaactact tatgggcaca gtgaatattg 840
   tgtgccctac ctgtgcacaa caataaacat catctacaat ggccgatcct gaaggtacaa 900
   atggggctgg gatggggtgt actggttggt ttgaggtaga agcagtcata gagagaagaa 960
   caggagataa tatttcagaa gatgaggatg aaacagcaga tgacagtggc acggatttac 1020
   tagagtttat agatgattct atggaaaata gtatacaggc agacacagag gcagcccggg 1080
   cattgtttaa tatacaggaa ggggaggatg atttaaatgc tgtgtgtgca ctaaaacgaa 1140
   agtttgccgc atgttcacaa agtgctgcgg aggacgttgt tgatcgtgct gcaaacccgt 1200
   gtagaacgtc tattaataaa aataaagaat gcacatacag aaaacgaaaa atagatgagc 1260
   tagaagacag cggatatggc aatactgaag tggaaactca gcagatggta caacaggtag 1320
   aaagtcaaaa tggcgacaca aacttaaatg acttagaatc tagtggggtg ggggatgatt 1380
   cagaagtaag ctgtgagaca aatgtagata gctgtgaaaa tgttacgttg caggaaatta 1440
   gtaatgttct acatagtagt aatacaaaag caaatatatt atataaattt aaagaggcct 1500
   atggaataag ttttatggaa ttagtaagac catttaaaag tgataaaaca agctgtacag 1560
   attggtgtat aacaggatat ggaattagtc catcagtagc agaaagttta aaagtattaa 1620
   ttaaacagca tagtttgtat actcatttac aatgtttaac ttgcgataga ggaataataa 1680
   tattattgtt aattagattt aggtgtagca aaaacaggtt aacagtagca aaactaatga 1740
   gtaatttatt atcaatacct gaaacatgta tggttataga gccaccaaaa ttacggagcc 1800
   aaacatgtgc attgtattgg tttagaacag caatgtcaaa cattagtgat gtacaaggta 1860
   caacacctga atggatagat agactaactg ttttacaaca tagctttaat gataatatat 1920
   ttgatttaag tgaaatggta cagtgggcat atgataacga gttaacggac gatagtgaca 1980
   ttgcatatta ttatgcacaa cttgcagatt caaatagtaa tgctgctgca tttttaaaaa 2040
   gtaactcaca agcaaaaata gtaaaggact gtggaataat gtgtagacat tataaaaaag 2100
   cagaaaaacg taaaatgtca ataggacaat ggatacaaag tagatgtgaa aaaacaaatg 2160
   atggaggaaa ttggagacca atagtacagt tgttaagata tcaaaacatt gaatttacag 2220
   catttttagg tgcatttaaa aagtttttaa aaggtatacc aaaaaaaagc tgtatgctaa 2280
   tttgtggacc agcaaataca ggaaagtcat attttggaat gagtttaata cagtttttaa 2340
   aagggtgtgt tatatcatgt gtaaattcta aaagtcactt ttggttgcag ccattatcag 2400
   atgcaaaaat aggaatgata gatgatgtaa cgccaataag ttggacatat atagatgatt 2460
   acatgagaaa tgcgttagat ggaaatgaaa tttcaataga tgtgaaacat agggcattag 2520
   tgcaattaaa atgtccacca ctgcttctta cctcaaatac aaatgcaggc acagactcta 2580
   gatggccata tttacatagt agattaacag tatttgaatt taaaaatcca ttcccatttg 2640
   atgaaaatgg taacccagtg tatgcaataa atgatgaaaa ttggaaatcc tttttctcaa 2700
   ggacgtggtg caaattagat ttaatagagg aagaggacaa ggaaaaccat ggaggaaata 2760
   tcagcacgtt taaatgcagt gcaggagaaa atactagatc tttacgaagc tgataaaact 2820
   gatttaccat cacaaattga acattggaaa ctgatacgca tggagtgtgc tttattgtat 2880
   acagccaaac aaatgggatt ttcacattta tgccaccagg tggtgccttc tttgttagca 2940
   tcaaagacca aagcatttca agtaattgaa ctacaaatgg cattagagac attaagtaaa 3000
   tcacagtata gtacaagcca atggacattg caacaaacaa gcttagaggt gtggctttgt 3060
   gaaccaccaa aatgttttaa aaaacaagga gaaacagtaa ctgtgcaata tgacaatgac 3120
   aaaaaaaata caatggatta tacaaactgg ggtgaaatat atattataga ggaagataca 3180
   tgtactatgg ttacagggaa agtagattat ataggtatgt attatataca taactgtgaa 3240
   aaggtatatt ttaaatattt taaagaggat gctgcaaagt attctaaaac acaaatgtgg 3300
   gaagtacatg tgggtggtca ggtaattgtt tgtcctacgt ctatatctag caaccaaata 3360
   tccactactg aaactgctga catacagaca gacaacgata accgaccacc acaagcagcg 3420
   gccaaacgac gacgacctgc agacaccaca gacaccgccc agccccttac aaagctgttc 3480
   tgtgcagacc ccgccttgga caatagaaca gcacgtactg caactaactg cacaaacaag 3540
   cagcggactg tgtgtagttc taacgttgca cctatagtgc atttaaaagg tgaatcaaat 3600
   agtttaaaat gtttaagata cagattaaaa ccttataaag agttgtatag ttctatgtca 3660
   tccacctggc attggaccag tgacaacaaa aatagtaaaa atggaattgt aactgtaaca 3720
   tttgtaactg aacagcaaca acaaatgttt ttaggtaccg taaaaatacc acctactgtg 3780
   caaataagta ctggatttat gacattataa gtgtacatca caagccaata tgtgctgcta 3840
   attgtatata accatgatat ttgtttttgt attatgtttt atattgtttt tatgcttatc 3900
   cttattatta cgtcctttaa tactttccat ttctacctat gcttggttgc tggtgttggt 3960
   attgctgctt tgggtgtttg tgggatctcc tttaaaaatt tttttttgct atttgttgtt 4020
   tttatattta ccaatgatgt gtattaattt tcatgcacag catatgacac aacaagagta 4080
   atgtatatac atgtatatat tgtttgtata tatgtgcaca tggtggtgtt ttaacattgt 4140
   tgttgttatt ttagtttttt tttttttgta ttactaataa atacctttat attttagcag 4200
   tgtattatta tgagacacaa acgatctaca aggcgcaagc gtgcatctgc aacacaacta 4260
   taccaaacat gcaaggccac aggcacctgc ccacccgatg ttattcctaa agtggaagga 4320
   agtaccatag cagatcaaat tcttaaatat ggcagtttag gggttttttt tggtggttta 4380
   ggtattggca caggctctgg ttcaggtgga aggactggct atgtacctat tggtactgac 4440
   ccacctacag ctgcaatccc cttgcagcct atacgtcctc cggttactgt agacactgtt 4500
   ggacctttag actcgtctat agtgtcatta atagaagaaa caagttttat agaggcaggt 4560
   gcaccagccc catctattcc tacaccatca ggttttgatg ttactacatc tgcagatact 4620
   acacctgcaa ttattaatgt ttcatctgtt ggggagtcat ctattcaaac tatttctaca 4680
   catttaaatc ccacatttac tgaaccatct gtactacacc ctccagcgcc tgcagaagcc 4740
   tctggacatt ttatattttc ttcccctact gttagcacac aaagttatga aaacatacca 4800
   atggatacct ttgttgtttc cacagacagt agtaatgtaa catcaagcac gcccattcca 4860
   gggtctcgcc ctgtggcacg ccttggttta tatagtcgca atacccaaca ggttaaggtt 4920
   gttgaccctg cttttttaac atcgcctcat aaacttataa catatgataa tcctgcattt 4980
   gaaagctttg accctgaaga cacattacaa tttcaacata gtgatatatc acctgctcct 5040
   gatcctgact ttctagatat tattgcatta cataggcctg ctattacatc tcgtagacat 5100
   actgtgcgtt ttagtagagt aggtcaaaaa gccacactta aaactcgcag tggtaaacaa 5160
   attggagcta gaatacatta ttatcaggat ttaagtccta ttgtgccttt agaccacacc 5220
   gtgccaaatg aacaatatga attacagcct ttacatgata cttctacatc gtcttatagt 5280
   attaatgatg gtttgtatga tgtttatgct gacgatgtgg ataatgtaca caccccaatg 5340
   caacactcat acagtacgtt tgcaacaaca cgtaccagca atgtgtctat acctttaaat 5400
   acaggatttg atactcctgt tatgtctggc cctgatatac cttccccttt atttcccaca 5460
   tctagcccat ttgttcctat ttcgcctttt tttccttttg acaccattgt tgtagacggt 5520
   gctgactttg ttttacatcc tagttatttt attttacgtc gcaggcgtaa acgttttcca 5580
   tattttttta cagatgtccg tgtggcggcc tagtgaggcc acagtgtacc tgcctcctgt 5640
   acctgtatct aaagttgtca gcactgatga atatgtgtct cgcacaagca tttattatta 5700
   tgctggtagt tccagacttc ttgctgttgg ccatccatat ttttctatta aaaatcctac 5760
   taacgctaaa aaattattgg tacccaaagt atcaggcttg caatataggg tttttagggt 5820
   ccgtttacca gatcctaata aatttggatt tcctgacacc tccttttata accctgatac 5880
   acaacgatta gtatgggcat gtgtaggcct tgaaataggt agagggcagc cattaggcgt 5940
   tggcataagt ggtcatcctt tattaaacaa atttgatgac actgaaaccg gtaacaagta 6000
   tcctggacaa ccgggtgctg ataataggga atgtttatcc atggattata aacaaacaca 6060
   gttatgttta cttggatgta agcctccaac aggggaacat tggggtaaag gtgttgcttg 6120
   tactaatgca gcacctgcca atgattgtcc acctttagaa cttataaata ctattattga 6180
   ggatggtgat atggtggaca caggatttgg ttgcatggat tttaaaacat tgcaggctaa 6240
   taaaagtgat gttcctattg atatttgtgg cagtacatgc aaatatccag attatttaaa 6300
   aatgactagt gagccttatg gtgatagttt atttttcttt cttcgacgtg aacaaatgtt 6360
   tgtaagacac ttttttaata gggctggtac attaggagag gctgttcccg atgacctgta 6420
   cattaaaggt tcaggaacta ctgcctctat tcaaagcagt gctttttttc ccactcctag 6480
   tggatcaatg gttacttccg aatctcagtt atttaataag ccatattggc tacaacgtgc 6540
   acaaggtcat aataatggta tttgttgggg caatcaggta tttgttactg tggtagatac 6600
   cactcgcagt actaatatga ctttatgcac acaagtaact agtgacagta catataaaaa 6660
   tgaaaatttt aaagaatata taagacatgt tgaagaatat gatctacagt ttgtttttca 6720
   actatgcaaa gttaccttaa ctgcagaagt tatgacatat attcatgcta tgaatccaga 6780
   tattttagaa gattggcaat ttggtttaac acctcctcca tctgctagtt tacaggatac 6840
   ctataggttt gttacctctc aggctattac gtgtcaaaaa acagtacctc caaaggaaaa 6900
   ggaagacccc ttaggtaaat atacattttg ggaagtggat ttaaaggaaa aattttcagc 6960
   agatttagat cagtttcctt tgggacgcaa gtttttatta caggcaggtc ttaaagcaaa 7020
   acctaaactt aaacgtgcag cccccacatc cacccgcaca tcgtctgcaa aacgcaaaaa 7080
   ggttaaaaaa taacactttg tgtaattgtg ttatgttgtt gttttgttct gtctatgtac 7140
   tttgtgttgt tgtgttgtgt tgttgtttgt tttttgtgta tgtgttacaa tgtatgttat 7200
   gttgtatgtt actgtgtttg ttttatgtgt acttgtttgt gtgcatgttc tatgtacttg 7260
   tcagtttcct gtttgtgtat atgttaataa aacattgtgt gtatttgtta aactatttgt 7320
   atgtatgtta tgtatatggg tgtacctata tgagtaagga gttgtattgc ttgccctacc 7380
   ctgcattgca atgtacctac ctttatttcc ctatatttgt agtacctaca tgtttagtat 7440
   tgctttacct tttgacatac tagtgtccat attgtacaat ttcctccatt ttgtatgcct 7500
   aaccgttttc ggttacttgg catacatacc ctatgacatt ggcagaacag ttaatccttt 7560
   tctttcctgc actgtgtttg tctgtacttg ctgcattggc atacataccc tatgacattg     7620
   gcagaacagt taatcctttt ctttcctgca ctgtgtttgt ctgtacttgc tgcattgact     7680
   catatataca tgcagtgcaa ttgcaaaata cttaattgta ctaatagttt acacatgctt     7740
   ttaggcacat atttttactt tactttcaaa ccttaagtgc agttttggct tacacaattg     7800
   ctttgtatgc caaactatgc cttgtaaaag tgagtcacta cctgtttatt accaggtgtg     7860
   gactaaccgt tttaggtcat attggtcatt tataatcttt tatataata                 7909
<210> 24
   <211> 7858
   <212> DNA
   <213> Human Papillomavirus HPV45 (X74479.1)
<400> 24
   aatactttta acaattatac tacataaaaa agggtgtaac cgaaaacggt tgcaaccaaa      60
   aacggtgcat ataaaagctt tgtggaaaag tgcattacag gatggcgcgc tttgacgatc     120
   caaagcaacg accctacaag ctaccagatt tgtgcacaga attgaataca tcactacaag     180
   acgtatctat tgcctgtgta tattgcaaag caacattgga acgcacagag gtatatcaat     240
   ttgcttttaa agatttatgt atagtgtata gagactgtat agcatatgct gcatgccata     300
   aatgtataga cttttattcc agaattagag aattaagata ttattcaaac tctgtatatg     360
   gagagacact ggaaaaaata actaatacag agttgtataa tttgttaata aggtgcctgc     420
   ggtgccagaa accattgaac ccagcagaaa aacgtagaca ccttaaggac aaacgaagat     480
   ttcacagcat agctggacag taccgagggc agtgtaatac atgttgtgac caggcacggc     540
   aagaaagact tcgcagacgt agggaaacac aagtatagca ataagtatgc atggaccccg     600
   ggaaacactg caagaaattg tattgcattt ggaacctcag aatgaattag atcctgttga     660
   cctgttgtgt tacgagcaat taagcgagtc agaggaggaa aacgatgaag cagatggagt     720
   tagtcatgca caactaccag cccgacgagc cgaaccacag cgtcacaaaa ttttgtgtgt     780
   atgttgtaag tgtgacggca gaattgagct tacagtagag agctcggcag aggaccttag     840
   aacactacag cagctgtttt tgagcacctt gtcctttgtg tgtccgtggt gtgcaactaa     900
   ccaataatct acaatggcgg atccagaagg taccgacggg gagggaacgg ggtgtaatgg     960
   ctggttcttt gtagaaacaa ttgtagagaa aaaaacaggg gatgtaatat cagatgatga    1020
   ggatgaaact gcaacagata cagggtcgga tatggtagat tttattgaca cacaattatc    1080
   catttgtgaa caggcagagc aagagacagc acaggcattg ttccatgcgc aggaagttca    1140
   gaatgatgca caggtgttgc atcttttaaa acgaaagttt gcaggaggca gcaaggaaaa    1200
   cagtccatta ggggagcagc taagtgtgga tacggatcta agtccacggt tacaagaaat    1260
   ttcattaaat agtgggcaca aaaaagcaaa acgacggttg tttacaatat cagatagtgg    1320
   ctatggctgt tctgaagtgg aagctgcaga gactcaggta actgtaaaca ctaatgcgga 1380
   aaatggcggc agtgtacata gtacacaaag tagtggtggg gatagtagtg acaatgcaga 1440
   aaatgtagat ccgcattgca gtattacaga actaaaggag ctattacaag caagtaacaa 1500
   aaaggctgca atgctggcag tatttaaaga catatatggg ctgtcattta cggatttggt 1560
   tagaaatttt aaaagtgata aaacaacatg tacagattgg gtaatggcta tatttggagt 1620
   taatccaacg gtagcagaag gctttaaaac attaattaaa ccagcaacgt tatacgccca 1680
   tatccaatgt ttagattgta aatggggagt attaatatta gctttattaa gatataaatg 1740
   tggcaaaaat agactaactg ttgcaaaagg cttaagcaca ttgttgcacg tacctgaaac 1800
   atgtatgtta attgaaccac caaaattgcg aagtagtgtt gcagcattat actggtatag 1860
   aacaggtata tccaatatta gtgaagtaag tggagacaca cctgagtgga tacaaagact 1920
   gacaattatt caacatggta ttgacgatag taattttgat ttgtcagaca tggtgcaatg 1980
   ggcatttgat aatgacctta cagatgaaag tgatatggca tttcaatatg cccaattagc 2040
   agactgcaac agtaatgcag ctgcattttt aaaaagtaac tgccaagcca aatatttaaa 2100
   agattgtgct gtaatgtgta gacattataa aagagcacaa aaacgccaaa tgaatatgtc 2160
   tcaatggatt aaatatagat gttccaaaat agatgaaggt ggggattgga gacccatagt 2220
   acaattccta agatatcagg gagtagaatt tattagcttt ttaagggcac taaaggaatt 2280
   tcttaaagga acaccaaaaa aaaattgtat actgttatat ggacctgcaa atacaggaaa 2340
   atcgtatttt ggaatgagtt ttatacattt cctacaaggt gcaataatat catttgtaaa 2400
   ttcaaacagc catttttggt tagaaccgtt agcagatact aaggtagcca tgttggatga 2460
   tgccacacac acgtgttgga catattttga taattatatg agaaatgcat tagatggtaa 2520
   tcctataagt atagacagaa agcataaacc attattacag ctaaaatgtc ctccaatcct 2580
   attaacatcc aatattgatc cagcaaaaga taataaatgg ccatatttag aaagtagggt 2640
   gacggtattt acatttccac atgcatttcc atttgataaa aatggtaatc cagtatatga 2700
   aataaatgat aaaaattgga aatgtttttt tgaaaggaca tggtccagat tagatttgca 2760
   cgaggacgat gaagatgcag acaccgaagg aatccctttc ggaacgttta agtgcgttac 2820
   aggacaaaat actagaccac tatgaaaatg acagtaaaga cataaacagc caaataagtt 2880
   attggcaact tatacgtttg gaaaatgcaa tactatttac agcaagggaa catggtatta 2940
   ccaaactaaa ccaccaggtg gtgcctccta ttaacatttc aaaaagcaaa gcacataaag 3000
   ctattgaact gcaaatggcc ttaaagggcc ttgcacaaag caagtataac aatgaggaat 3060
   ggacactgca agatacatgc gaggaactat ggaatacaga accgtcgcag tgttttaaaa 3120
   aaggcggtaa aaccgtgcac gtatactttg atggcaacaa ggacaactgt atgaactatg 3180
   tagtatggga cagtatatat tatataactg agacagggat atgggacaaa acagcagcat 3240
   gtgttagcta ttggggtgta tattatataa aagatggaga taccacatat tatgtacaat 3300
   ttaaaagcga atgtgagaaa tatggaaata gtaatacgtg ggaagtacaa tatgggggca 3360
   atgtaattga ttgtaatgac tctatgtgca gtaccagtga cgacacggta tccgctactc 3420
   agattgttag acagctacaa cacgcctcca cgtcgacccc caaaaccgca tccgtgggca 3480
   ccccaaaacc ccacatccag acgccggcta ctaagcgacc tagacagtgt ggactcacag 3540
   agcagcacca cggacgtgtc aacacccacg tgcacaaccc gctcctgtgt tcaagtacaa 3600
   gtaacaacaa aagaaggaaa gtgtgtagtg gtaacactac gcctataata cacttaaaag 3660
   gtgacaaaaa cagtttgaaa tgtttaagat ataggctacg caaatatgca gaccattact 3720
   cagaaatatc ctccacctgg cattggacag gttgtaataa aaacactggt atattaactg 3780
   taacatataa tagtgaggta caaagaaata cctttttgga tgtagttact attcctaaca 3840
   gtgtacaaat ctcggtggga tacatgacta tatgaatctg tatattgtat acagtatgta 3900
   acattactat gctatcttta gtgtttttat tgtgcttttc tgtgtgcctt tatgtgtgct 3960
   gcaatgtccc gcttgtgcag tctgtctatg tgtgtgcttt tgcttggttg ttggtgtttc 4020
   tttttatagt tgttattaca tccccattaa cagcatttgc tgtatacatt tgttgctatt 4080
   tactacctat gtttgtatta catatgcatg ctttacacac catacaataa ttactataat 4140
   gtacagtaca gtgtaacata cctgtgatgt gcatgttgtt gtatttttgt atttttgtat 4200
   ttttgtattt ttgtatttta tatgtttaat aaaccatggt atcccaccgt gcagcacgtc 4260
   gcaagcgggc ctctgcaact gacttatata gaacatgtaa gcaatccggt acgtgccccc 4320
   ctgatgttat taacaaagtg gaaggcacaa ccttagctga taaaatttta cagtggtcta 4380
   gccttgggat atttttgggt ggccttggca ttggtaccgg cagtggttct ggaggccgta 4440
   cgggctatgt acccttaggg ggcaggtcta atactgttgt ggatgttggc cccactaggc 4500
   cacctgtggt tattgaacct gtagggccta ctgatccatc tattgttacg ttggtagagg 4560
   attccagtgt tgttgcctct ggtgctccgg ttcccacatt taccggaacc tctgggtttg 4620
   aaattacgtc ttctggtact accacaccag ctgtgttgga catcacacct accgtggact 4680
   ctgtttctat ttcgtcaact agttttacaa atcctgcatt ttctgatccc tctattattg 4740
   aggtgcccca aacaggggag gtatcaggta atatatttgt tggtacacca acatcgggca 4800
   gccatggata tgaggaaata cctttacaaa catttgcatc ttctgggtca ggtacggaac 4860
   ccattagtag tacccccctc cctactgtgc ggcgggtacg gggtccccgc ctgtatagta 4920
   gggctaatca acaggtccgt gtgtccacct cacagttttt aacacatccc tcatcgttgg 4980
   ttacatttga taatccagct tatgagcccc tggacaccac actatccttt gagcctacca 5040
   gtaatgttcc tgattccgat tttatggata ttattcgttt gcataggcca gcattatcct 5100
   ctagacgtgg cactgttaga tttagtagat tgggtcaaag ggcaaccatg tttacacgta 5160
   gtggtaaaca aatagggggt agggtacatt tttaccatga tataagcccc attgctgcta 5220
   cagaggaaat tgaattgcag cctttaatta gtgctacaaa tgatagtgac ctgtttgatg 5280
   tatatgcaga cttcccacct cctgcgtcca ctacacctag cactatacac aaatcattta 5340
   catatccaaa gtattccttg accatgcctt ctactgctgc atcctcttac agtaatgtta 5400
   cagtaccatt aacatctgca tgggatgtac ctatatatac tggcccggac attatattgc 5460
   catcccatac tcctatgtgg cctagtacat ctcctaccaa tgcttccacc accacctata 5520
   taggtattca tggcacacaa tattatttat ggccatggta ttattatttt cctaaaaaac 5580
   gtaaacgtat tccctatttt tttgcagatg gctttgtggc ggcctagtga cagtacggta 5640
   tatcttccac caccttctgt ggccagagtt gtcagcactg atgattatgt gtctcgcaca 5700
   agcatatttt atcatgcagg cagttcccga ttattaactg taggcaatcc atattttagg 5760
   gttgtaccta atggtgcagg taataaacag gctgttccta aggtatccgc atatcagtat 5820
   agggtgttta gagtagcttt acccgatcct aataaatttg gattacctga ttctactata 5880
   tataatcctg aaacacaacg tttggtttgg gcatgtgtag gtatggaaat tggtcgtggg 5940
   cagcctttag gtattggcct aagtggccat ccattttata ataaattgga tgatacagaa 6000
   agtgctcatg cagctacagc tgttattacg caggatgtta gggataatgt gtcagttgat 6060
   tataagcaaa cacagctgtg tattttaggt tgtgtacctg ctattggtga gcactgggcc 6120
   aagggcacac tttgtaaacc tgcacaattg caacctggtg actgtcctcc tttggaactt 6180
   aaaaacacca ttattgagga tggtgatatg gtggatacag gttatggggc aatggatttt 6240
   agtacattgc aggatacaaa gtgcgaggtt ccattagaca tttgtcaatc catctgtaaa 6300
   tatccagatt atttgcaaat gtctgctgat ccctatgggg attctatgtt tttttgccta 6360
   cgccgtgaac aactgtttgc aagacatttt tggaataggg caggtgttat gggtgacaca 6420
   gtacctacgg acctatatat taaaggcact agcgctaata tgcgtgaaac ccctggcagt 6480
   tgtgtgtatt ccccttctcc cagtggctct attattactt ctgattctca attatttaat 6540
   aagccatatt ggttacataa ggcccagggc cataacaatg gtatttgttg gcataatcag 6600
   ttgtttgtta ctgtagtgga cactacccgc agtactaatt taacattatg tgcctctaca 6660
   caaaatcctg tgccaagtac atatgaccct actaagttta agcagtatag tagacatgtg 6720
   gaggaatatg atttacagtt tatttttcag ttgtgcacta ttactttaac tgcagaggtt 6780
   atgtcatata tccatagtat gaatagtagt atattagaaa attggaattt tggtgtccct 6840
   ccaccaccta ctacaagttt ggtggataca tatcgttttg tgcaatcagt tgctgttacc 6900
   tgtcaaaagg atactacacc tccagaaaag caggatccat atgataaatt aaagttttgg 6960
   actgttgacc taaaggaaaa attttcctcc gatttggatc aatatcccct tggtcgaaag     7020
   tttttagttc aggctgggtt acgtcgtagg cctaccatag gacctcgtaa gcgtcctgct     7080
   gcttccacgt ctactgcatc tactgcatct aggcctgcca aacgtgtacg tatacgtagt     7140
   aagaaataat atgttagcac atatatgtat gtttgtatgt atggttttgt atgttgtatg     7200
   tatgtatgta tttgtgtgat atattactgt attttgtttg tttgcgtgcg tgtatgtatg     7260
   aatgtgcctt gtggcatgta tggtgttact gtacataatt gtggtattaa ataaagtatg     7320
   ctaatagtgt tgtgtagggt tgcacccttg tgagtaacaa tactatttgt gtgtatgtgt     7380
   attgctttgt accctatatt ctttcctgta tttcaagtta taaacttgca tactacacag     7440
   catccatttt acttataatc ctccattttg ctgtgcaacc gatttcggtt gcctgtggct     7500
   tatatgtgac cttttaaaca taatacctaa actggcacat ttacaacccc tacatagttt     7560
   aacctactgg cgcgccttct tggcgtacat gtggcacacc tggtattagt cattttcctg     7620
   tccaggtgta ctaaaacaat ggcttgcaca actgtatcca caccctatgt aataaaactg     7680
   cttttaggca catattttag tctgttttta cctgtgctaa ttgtataatt ggcgtgtaga     7740
   accactttct tatccaacaa tctgtctact tgttacataa actataaact gactcactta     7800
   tacatacata gtttatgcaa ccgaaaaagg ttgggcccta taacacatac cttttctt       7858

## Description

The invention relates to a method for easily, rapidly and accurately detecting rearrangements of a human papilloma virus (HPV) DNA in a viral host cell, tissue or biological fluid obtained from a subject or patient using Molecular Combing and/or DNA stretching in combination with specially designed probes for the HPV DNA sequence.

The disclosure describes a method for monitoring *in vitro* the effects of anti-viral treatment by following the presence of genomic viral polynucleotides in a virus-infected cell, tissue or a fluid of a patient to be tested as infected by a virus.

The invention relates to the detection of a HPV based on DNA hybridization and the use of a kit comprising the probes used to carry out a method of the invention. The disclosure describes a composition comprising said probes.

### Description of the Related Art

### Molecular Combing

Molecular Combing technology has been disclosed in various patents and scientific publications, for example in U.S. 6,303,296, WO 9818959, WO 0073503, U.S. 2006/257910, U.S.2004/033510, U.S. 6,130,044, U.S. 6,225,055, U.S. 6,054,327, WO 2008/028931, WO 2010/035140, and Michalet, Ekong et al. 1997, Herrick, Michalet et al. 2000, Herrick, Stanislawski et al. 2000, Gad, Aurias et al. 2001, Gad, Caux-Moncoutier et al. 2002, Gad, Klinger et al. 2002, Herrick, Jun et al. 2002, Pasero, Bensimon et al. 2002, Gad, Bieche et al. 2003, Lebofsky and Bensimon 2003, Herrick, Conti et al. 2005, Lebofsky and Bensimon 2005, Lebofsky, Heilig et al. 2006, Patel, Arcangioli et al. 2006, Rao, Conti et al. 2007 and Schurra and Bensimon 2009 (see citations below)..

Bensimon, et al., U.S. Patent No. 6,303,296, discloses DNA stretching procedures, Lebofsky, et al., WO 2008/028931 discloses detections of genomic sequences, and Lebofsky, et al., WO 2010/035140 A1, discloses a method for analysis of D4Z4 tandem repeat arrays on human chromosomes 4 and 10 based on stretching of nucleic acid and on Molecular Combing.

Molecular Combing is a technique enabling the direct visualization of individual nucleic acid molecules and has been successfully used for the location of *myc* and certain Human Papillomavirus (HPV) sequences in cervical cancer cell lines as well as for the study of replication kinetics on previously characterized MYC/papillomavirus (HPV18) amplicon and particularly on its origin of replication (Herrick, et al., Cancer Res. 65(4): 1174-1179 2005). Nucleic acid rearrangements and amplifications in tumor cells were correlated with cancer outcome using this technique. Herrick, et al. probed combed nucleic acids from cultured tumor cell lines IC1, IC2, IC4 and IC5 with probes specific to *N-myc* and *c-myc* genes and probes to detect HPV nucleic acids containing full-length HPV-18 and HPV-44 (∼ 7.8 kb). Conti, et al., Genes, Chromosomes & Cancer 46:724-734 (2007) studied origin activation in an HPV/MYC amplicon containing certain HPV 18 nucleic acid sequences and analyzed origin activity and fork movement on DNA stretched by Molecular Combing from the IC1 tumor cell line. Conti identifies gene rearrangements and amplifications as hallmarks of cancer cells focusing on the mechanisms involving both HPV and MYC nucleic acid sequences. Both these studies used cultured tumor cells in which HPV nucleic acid sequences act as oncogenes but not as infectious genomic viral nucleic acids.

Episomal and integrated Epstein-Barr virus DNA obtained from cultured Burkitt's lymphoma cell lines was characterized by FISH on combed DNA, (Reisinger, et al., Int. J. Cancer 118: 1603-1608 (2006). Reisinger's objective was to study the genomic organization of viral genomes and understand and attempt to discriminate between the episomal and integrated forms of EBV in infected eukaryotic cells. DNA from cultured Burkitt's lymphoma cell lines was stretched by Molecular Combing and probed with EBV-specific DNA probes that cover genomic sequences but not the region between the terminal repeat (TR) and the internal repeat 1 (IR1) sequences.

Reisinger *et al.* and Herrick *et al.* also do not describe methods for detecting rearrangements of viral genomes using probe sets tagged with different haptens that can generate a different color-fluorescence array that will allows the identification of rearrangements within this specific region of the viral DNA. These two references describe the detection of viral DNA in cultured cells but not in tissue or in biological fluids.

In contrast, in the present invention, the standard extraction procedure is modified to allow the extraction of viral DNA from viral particles in order to analyze by Molecular Combing the structure of the viral DNA in the infectious particles. Moreover, the invention develops a methodology of extraction of high molecular weight DNA from solid tissues such as cornea that is compatible with the analysis by Molecular Combing and that allows the detection of the presence of the infectious forms of HSV DNA in an infected cornea. These two methodologies have never been described before this invention.

Stretching nucleic acid, in particular viral or genomic DNA provides immobilized nucleic acids in linear and parallel strands and is preferably performed with a controlled stretching factor on an appropriate surface (e.g. surface-treated glass slides). After stretching, it is possible to hybridize sequence-specific probes detectable for example by fluorescence microscopy (Lebofsky, Heilig et al. 2006). Thus, a particular sequence may be directly visualized on a single molecule level. The length of the fluorescent signals and/or their number, and their spacing on the slide provides a direct reading of the size and relative spacing of the probes. However, up to now, it was not shown that this technology can be used to detect the infectious forms of viruses in an infected eukaryotic cell or viral sequences or genome of a derived mutated viral sequence infecting a cell or tissue or present in a fluid of mammals.

EP1331277 discloses a method for DNA sequencing and gene identification that allows identification of a single molecule of a target DNA wherein an optically distinguishable material is attached to a DNA recognition unit and the detection is performed in a sequential manner after the hybridization complex between the DNA sequence hybridization unit and the target DNA molecule has been starched to adopt a substantially linear configuration.

### Virus Structure and Genome

The viruses that cause human diseases are placed into seven groups based upon the existence of an envelope, the type of the capsid, the nature of the genome (DNA or RNA, double-stranded or single-stranded), size, and target cells (International Committee on Taxonomy of Viruses, http://_www.ictvonline.org; last accessed April 7, 2011). The enveloped DNA viruses include the poxviruses, herpesviruses, and hepadnaviruses. The nonenveloped group includes the adenoviruses, papovaviruses, and parvoviruses.

An enormous variety of genomic structures can be seen among viral species. Indeed, there are millions of different types of viruses (Breitbart and Rohwer 2005), though only about 5,000 of them have been described in detail (Dimmock, Easton et al. 2007). A viral genome is either single-stranded or double-stranded. Single-stranded genomes consist of an unpaired nucleic acid, analogous to one-half of a ladder split down the middle. All RNA viruses are single-stranded except for the double-stranded reoviruses. Double-stranded genomes consist of two complementary paired nucleic acids, analogous to a ladder. All DNA viruses are double-stranded except for the parvoviruses, which have single-stranded DNA. Some viruses, such as those belonging to the Hepadnaviridae, contain a genome that is partially double-stranded and partially single-stranded (Collier and Oxford 2006)

Viral genomes are circular, as in the polyomaviruses, or linear, as in the adenoviruses (Fields, Knipe et al. 2007). The type of nucleic acid is irrelevant to the shape of the genome. Among RNA viruses, the genome is often divided up into separate parts within the virion and is called segmented. Each segment often codes for one protein and they are usually found together in one capsid. Every segment is not required to be in the same virion for the overall virus to be infectious, as demonstrated by the brome mosaic virus (Collier and Oxford 2006).

For viruses with RNA or single-stranded DNA, the strands are said to be either positive-sense (called the plus-strand) or negative-sense (called the minus-strand), depending on whether it is complementary to the viral messenger RNA (mRNA) (Fields, Knipe et al. 2007). Positive-sense viral RNA is identical to viral mRNA and thus can be immediately translated by the host cell. Negative-sense viral RNA is complementary to mRNA and thus must be converted to positive-sense RNA by an RNA polymerase before translation. DNA nomenclature is similar to RNA nomenclature, in that the coding strand for the viral mRNA is complementary to it (-), and the non-coding strand is a copy of it (+). Some single-stranded RNA viruses called retrovirus (which includes HIV) replicate in the host cell via the enzyme reverse transcriptase to produce DNA from its RNA genome. The DNA is then incorporated into the host's genome by an integrase enzyme. The virus genome, or provirus, remains in the genome of the infected cell and thereafter replicates as part of the host cell's DNA.

Genome size varies greatly amongst different kinds or species of viruses ranging from 1.7 kb for the hepatitis delta virus (HDV) to 309 kb for the Poxvirus. RNA viruses generally have smaller genome sizes than DNA viruses because of a higher error-rate when replicating, and have a maximum upper size limit. Beyond this limit, errors in the genome when replicating render the virus useless or uncompetitive. To compensate for this, RNA viruses often have segmented genomes where the genome is split into smaller molecules, thus reducing the chance of error. In contrast, DNA viruses generally have larger genomes because of the high fidelity of their replication enzymes (Pressing and Reanney 1984).

Viruses can undergo genetic changes that occur through different mechanisms. The first process is called genetic drift where individual bases in the DNA or RNA mutate to other bases. Most of these point mutations are "silent" meaning that they do not change the protein that the gene encodes but others can confer evolutionary advantages such as resistance to antiviral drugs. Antigenic shift occurs when there is a major change in the genome of the virus (Pan, Li et al. 2007). This can be a result of recombination or reassortment. When this happens with influenza viruses (Hampson and Mackenzie 2006), pandemics might result. RNA viruses often exist as quasispecies or swarms of viruses of the same species but with slightly different genome nucleoside sequences. Such quasispecies are a prime target for natural selection (Metzner 2006). Secondly, segmented genomes confer also evolutionary advantages; different strains of a virus with a segmented genome can shuffle and combine genes and produce progeny viruses that have unique and new characteristics. This is called reassortment or viral sex (Goudsmit 1998). Finally, genetic recombination which is the process by which a strand of DNA is broken and then joined to the end of a different DNA molecule can arise. This can occur when viruses infect cells simultaneously and studies of viral evolution have shown that recombination has been rampant in the species studied (Worobey and Holmes 1999). Recombination is common to both RNA and DNA viruses (Umene 1999; Lukashev 2005).

The HSV are among the larger viruses with a diameter ranging from about 150 nm to 200 nm. They are enclosed within a loosely fitting envelope that contains glycoprotein spikes (Wildy, Russell et al. 1960). Like other enveloped viruses, herpes viruses are prone to deactivation by organic solvents or detergents and are unstable outside the host's body. The icosahedral capsid houses a core of double-stranded DNA that winds around a proteinaceous spindle in some viruses. The genome sequence of HSV-1 strain 17, first published in 1988, contains 152,261 residues in each strand (Genbank accession number: NC_001806.1; last accessed April 7, 2011) (McGeoch, Dalrymple et al. 1988). The HSV genome is regarded as being composed of two covalently joined segments, the long (L) and short (S) regions (Roizman 1979). The L region consists of a unique sequence (U_{L}) flanked by a pair of oppositely oriented repeat elements (termed R_{L}, with the terminal and internal copies specifically referred to as TR_{L} and IR_{L}) (Wadsworth, Jacob et al. 1975). The S region similarly consists of IRs, Us, and TRs. The sequences of the R_{L} and Rs elements are distinct, except that there is a 400 base pairs (bp) direct repeat at the genome termini, termed the a sequence, and at least one further copy of this is found at the junction of L and S regions, in the orientation opposite to the terminal copies (Roizman 1979; Davison and Wilkie 1981). Each terminus possesses one overhanging residue, with the 3'-hydroxyl group free (Mocarski and Roizman 1982). Preparations of HSV DNA contain equimolar amounts of four sequence-orientation isomers, in which U_{L} and Us each lie independently in one of two possible orientations with respect to the joint between L and S sequences (Hayward, Jacob et al. 1975; Bataille and Epstein 1997). One isomer is defined as the prototype (Roizman 1979). HSV-1 DNA has a base composition of 68.3% G+C (McGeoch, Dahymple et al. 1988). The G+C content is not constant throughout the genome; the 6.6-kbp R_{S} elements deviate most notably from the mean, with a G+C content of 79.5% (McGeoch, Dolan et al. 1986). The genome of HSV-2 has not been completely sequenced, but it closely resembles that of HSV-1, albeit with a slightly higher G+C content.

The genomic polynucleotide sequences of numerous viruses are well-known to those of skill in the art and are described in Fields, B. N., D. M. Knipe, et al. (2007), Fields' Virology. Philadelphia, Wolters kluwer/Lippincott Williams & Wilkins; and in the NCBI Virus Genomes database accessible at http:// www._ ncbi.nlm.nih.gov/genomes/genlist.cgi?taxid=10239&type=5&name=Viruses (last accessed April 7, 2011).

### Viral Pathogenesis

Many types of viral pathogens are recognized. For a recent review on viral infection, one can refer to Fields, *et al.* (Fields, Knipe et al. 2007) and references therein. A viral infection occurs when a virus enters the body through such processes as breathing air contaminated with a virus, eating contaminated food, or by having sexual contact with a person who is infected with a virus. A viral infection may also be caused by an insect bite. In a viral infection, the virus invades the inside of the body's cells in order to reproduce. A virus then spreads to other cells and repeats the process. This process of viral infection results in a variety of symptoms that vary in character and severity depending on the type of viral infection and individual factors. Common symptoms of a viral infection include fatigue, flu-like symptoms and fever.

Many types of viral infections, such as a common cold, are self limiting in generally healthy people. This means that the viral infection causes illness for period of time, then it resolves and symptoms disappear. However, some people are at risk for developing serious complications of viral infection. In addition, certain types of viral infections, such as HIV/AIDS, are not self limiting and cause serious complications and/or can eventually be fatal. As an example of serious damage, some forms of measles virus infection lead to brain's inflammation, and consequently permanent brain injury.

There are many types of viruses that cause a wide variety of viral infections or viral diseases. For example, there are over 200 different viruses that can cause a cold or an upper respiratory infection. Other common viruses include the influenza virus which causes influenza or the flu. The Epstein-Barr virus and the cytomegalovirus cause infectious mononucleosis, the varicella zoster virus (VZV) causes shingles and chickenpox, and HIV causes AIDS.

Viruses have different mechanisms by which they produce disease in an organism, which largely depends on the viral species. Mechanisms at the cellular level primarily include cell lysis, the breaking open and subsequent death of the cell. In multicellular organisms, if enough cells die the whole organism will start to suffer the effects. Although viruses cause disruption of healthy homeostasis, resulting in disease, they can also exist relatively harmlessly within an organism. An example would include the ability of the herpes simplex virus (HSV), which causes cold sores, to remain in a dormant state within the human body. This is called latency (Margolis, Elfman et al. 2007) and is a characteristic of the all herpes viruses including the EBV, which causes glandular fever, and the VZV. Most people have been infected with at least one of these types of HSV (Whitley and Roizman 2001). However, these latent viruses might sometimes be beneficial, as the presence of the virus can increase immunity against bacterial pathogens, such as *Yersinia pestis* (Barton, White et al. 2007). On the other hand, latent chickenpox infections return in later life as the disease called shingles.

Some viruses can cause life-long or chronic infections, where the viruses continue to replicate in the body despite the host's defense mechanisms (Bertoletti and Gehring 2007). This is common in hepatitis B and C virus infections. People chronically infected are known as carriers, as they serve as reservoirs of infectious virus. In populations with a high proportion of carriers, the disease is said to be endemic (Rodrigues, Deshmukh et al. 2001). In contrast to acute lytic viral infections this persistence implies compatible interactions with the host organism.

HSV-1 infection is one example of a viral infection. HSV was named for the tendency of some herpes infections to produce a creeping rash (Taylor, Brockman et al. 2002). It is the common name for a large family whose members include herpes simplex 1 and 2, the cause of fever blisters and genital infections; VZV, the cause of chickenpox and shingles; cytomegalovirus (CMV), which affects the salivary glands and other viscera; EBV, associated with infection of the lymphoid tissue; and some recently identified viruses (HSV-6, -7, and -8). Prominent features of the family are its tendency toward viral latency and recurrent infections.

HSV infections, often colloquially called herpes, usually target the mucous membranes. The virus enters cracks or cuts in the membrane surface and then multiplies in basal and epithelial cells in the immediate vicinity. This results in inflammation, edema, cell lysis, and formation of a characteristic thin-walled vesicle. The main diseases of HSV are facial herpes (oral, optic, and pharyngeal), genital herpes, neonatal herpes, and disseminated disease. Herpes labialis otherwise known as fever blisters or cold sores is the most common recurrent HSV-1 infection. Vesicles usually crop up on the muco-cutaneous junction of the lips or on adjacent skin. Herpetic keratitis (also called ocular herpes) is an infective inflammation of the eye in which a latent virus travels into the ophthalmic rather than the mandibular branch of the trigeminal nerve. Preliminary symptoms are a gritty feeling in the eye, conjunctivitis, sharp pain, and sensitivity to light. Some patients develop characteristic branched or opaque corneal lesions as well. In 25% to 50% of cases, keratitis is recurrent and chronic and can interfere with vision.

The primary infection, asymptomatic in more than 90 % of cases (Liesegang 1989) takes place in the oral mucosae, as a consequence of contact with infected particles of saliva. Soon after infection, the linear viral genome circularizes and DNA replication initiates at an origin (for review:(Boehmer and Lehman 1997). DNA replication initially proceeds by a theta mechanism and subsequently switches to a sigma or rolling-circle mode to yield long head-to-tail concatemers. Multiple DNA replication forks that arise from homologous recombination, *a* sequence-mediated genome isomerization, and other events, lead to the formation of an extensive network of branched DNA intermediates. Finally, these structures are resolved into unit-length genomes and packaged into preassembled capsids. Nevertheless, DNA molecules shorter than the full-length standard HSV-1 viral DNA can become encapsidated within nuclear capsids provided they contain the cleavage/packaging signal (Vlazny, Kwong et al. 1982). However, capsids containing HSV-1 genome significantly shorter than standard viral genome are not infective.

After replication in epithelial tissues, viruses propagate in neurons before becoming latent. Type 1 HSV enters primarily the trigeminal, or fifth cranial, nerve, which has extensive innervations in the oral region while type 2 HSV usually becomes latent in the ganglion of the lumbo-sacral spinal nerve trunk. Following various triggering factors such as fever, UV radiation, stress, or mechanical injury, the virus migrates to the body surface and produces a local skin or membrane lesion, often in the same site as a previous infection. Since the principal location of latent HSV-1 is the trigeminal ganglion, responsible for sensory innervation of the face, most recurrences are located in the eyes or the lips. The seroprevalence of HSV-1 in the general population ranges from 24.5% to 67%, with 30 to 70% of positive subjects experiencing recurrent herpes labialis (Liesegang 2001). Indeed, almost everyone may potentially exhibit an HSV-1 infection event since recent studies using Polymerase Chain Reaction (PCR) on post-mortem tissues showed that nearly 100% of people at least 60 years of age have several HSV-1 genome copies present in the trigeminal ganglion (Wang, Lau et al. 2005). The eye, and particularly the cornea, is the second most frequent location of HSV-1 infection. The prevalence of herpes keratitis is 149/100,000 (Liesegang, Melton et al. 1989), with more than 30 new events per 100,000 inhabitants annually (Labetoulle, Auquier et al. 2005). Once the eye has been clinically affected by an herpetic infection, the rate of relapse is 23% within 2 years and 40% within 5 years of follow-up (Wilhelmus, Coster et al. 1981; Liesegang 1989). The visual prognosis is poor, especially in deep corneal infections (stromal keratitis), with up to 60% of affected eyes reaching a visual acuity of less than 20/40 after 5 years of follow-up (Kabra, Lalitha et al. 2006). As a consequence, HSV-1 is a leading cause of blindness throughout the world. In less developed countries, HSV-1 ocular infection accounts for about 10% of patients attending corneal clinics (Pramod, Rajendran et al. 1999), and despite the use of topical or oral antiviral agents in the last three decades, herpes simplex keratitis also remains the most frequent cause of infectious corneal opacities in the most developed areas of the world (group 1998), accounting for about 10% of patients undergoing corneal transplantation (Leger, Larroque et al. 2001). Moreover, the natural risk of HSV-1 reactivation in recently grafted cornea is about 25% in the first year following surgery (Lomholt, Baggesen et al. 1995), and about 33% of primary graft failure (no clearing of the graft) have shown to be associated with the presence of the HSV-1 genome in the cornea (Cockerham, Bijwaard et al. 2000). These data explain why HSV-1 remains a major cause of corneal graft failure, accounting for about 22% of all cases of re-grafting.

The clinical complications of latency and recurrent infections become more severe with advancing age, cancer chemotherapy, or other conditions that compromise the immune defenses. The HSV are among the most common and serious opportunists among AIDS patients (Ramaswamy and Geretti 2007). Nearly 95% of this group will experience recurrent bouts of skin, mucous membrane, intestinal, and eye disease from these viruses.

Human immunodeficiency virus (HIV) is a lentivirus (a member of the retrovirus family) that causes *acquired immunodeficiency syndrome* (AIDS) (Weiss 1993; Douek, Roederer et al. 2009), a condition in humans in which the immune system begins to fail, leading to life-threatening opportunistic infections. Infection with HIV occurs by the transfer of blood, semen, vaginal fluid, pre-ejaculate, or breast milk. Within these bodily fluids, HIV is present as both free virus particles and virus within infected immune cells. The four major routes of transmission are unsafe sex, contaminated needles, breast milk, and transmission from an infected mother to her baby at birth (vertical transmission). Screening of blood products for HIV has largely eliminated transmission through blood transfusions or infected blood products in the developed world.

HIV infection in humans is considered pandemic by the World Health Organization (WHO). From its discovery in 1981 to 2006, AIDS killed more than 25 million people. HIV infects about 0.6% of the world's population. In 2005 alone AIDS claimed an estimated 2.4-3.3 million lives of which more than 570,000 were children. Antiretroviral treatment reduces both the mortality and the morbidity of HIV infection.

HIV infects primarily vital cells in the human immune system such as helper T cells (to be specific, CD4⁺ T cells), macrophages, and dendritic cells. HIV infection leads to low levels of CD4⁺ T cells through three main mechanisms: first, direct viral killing of infected cells; second, increased rates of apoptosis in infected cells; and third, killing of infected CD4⁺ T cells by CD8 cytotoxic lymphocytes that recognize infected cells. When CD4⁺ T cell numbers decline below a critical level, cell-mediated immunity is lost, and the body becomes progressively more susceptible to opportunistic infections.

Most people infected with HIV-1 left untreated eventually develop AIDS and eventually die from opportunistic infections or malignancies associated with the progressive failure of the immune system (Lawn 2004). HIV progresses to AIDS at a variable rate affected by viral, host, and environmental factors; most will progress to AIDS within 10 years of HIV infection: some will have progressed much sooner, and some will take much longer (Buchbinder, Katz et al. 1994; 2000). Treatment with anti-retroviral drugs increases the life expectancy of people infected with HIV and even after HIV has progressed to diagnosable AIDS, the average survival time with antiretroviral therapy was estimated to be more than 5 years as of 2005 (Schneider, Gange et al. 2005). Without antiretroviral therapy, someone who has AIDS typically dies within a year (Morgan, Mahe et al. 2002).

### Diagnoses of and Treatment of Viral Infections

Viral diseases are diagnosed by a variety of clinical and laboratory methods. These include a clinical assessment of symptoms, viral isolation in cell or animal culture, and serological testing for antibodies to a virus. Making a diagnosis of a viral infection begins with taking a thorough personal and family medical history, including symptoms, and completing a physical examination. Diagnosing some viral infections, such as seasonal influenza, may be made based on a history and physical. Blood tests, such as a complete blood count may be done. A complete blood count measures the numbers of different types of blood cells, including white blood cells (WBCs). Different types of WBCs increase in number in characteristic ways during an infectious process, such as viral infection. A culture test may also be performed. This test requires taking a small sample from the body area that is suspected to be infected with a virus and grows the sample in a lab to determine the type of microorganism causing illness. Common samples tested with a culture include those from the throat, blood, and sputum from the lungs. Diagnostic tests may also include a lumbar puncture, also called a spinal tap, which involves withdrawing a small sample of cerebrospinal fluid (CSF) from the spine with a needle. The sample of CSF is tested for white blood cells and other indications of viral infection that may be in the spine or brain, such as viral meningitis. X-rays may be performed to assist in the diagnosis of some viral infections. This may include taking a chest X-ray for suspected cases of viral pneumonia. Additional tests may be performed in order to rule out or confirm other diseases that may accompany viral infection or cause similar symptoms, such as a secondary bacterial infection.

HSV infections are diagnosed by a variety of clinical and laboratory methods, including a clinical assessment of symptoms, isolation in cell or animal culture, and serological testing for antibodies to HSV. Small, painful, vesiculating lesions on the mucous membranes of the mouth or genitalia, lymphadenopathy, and exudate are typical diagnostic symptoms of herpes simplex. Further diagnostic support is available by examining scrapings from the base of such lesions stained with Giemsa, Wright (also called Tzanck preparation), or Papanicolaou (Pap) methods. The presence of multinucleate cells, giant cells, and intranuclear eosinophilic inclusion bodies can help establish herpes infection. This method, however, will not distinguish among HSV-1, HSV-2, or other herpesviruses, which require more specific subtyping. Moreover, laboratory culture and specific tests are essential for diagnosing immunosuppressed and neonatal patients with severe, disseminated herpes infection. A specimen of tissue or fluid is introduced into a primary cell line such as monkey kidney or human embryonic kidney tissue cultures and is then observed for cytopathic effects within 24 to 48 hours. Direct tests on specimens or cell cultures using fluorescent antibodies or detection of DNA using specific probes or amplification by Polymerase Chain Reaction (PCR) can differentiate among HSV-1, HSV-2, and closely related HSV, but are not useful tools to confirm that the genomes of said viruses found in a sample are intact or infectious. While serological analysis is useful for primary infection, it is inconclusive for recurrent illness because the antibody titer to HSV upon recurrence of the infection usually does not increase.

Several agents are available for treatment of HSV infections. Acyclovir is the most effective therapy developed to date that is nontoxic and highly specific to HSV. Famciclovir and valacyclovir are alternate drugs. Topical medications applied to genital and oral lesions cut the length of infection and reduce viral shedding. Systemic therapy is available for more serious complications such as herpes keratitis and disseminated herpes. New evidence indicates that a daily dose of oral acyclovir taken for a period of 6 months to one year can be effective in preventing recurrent genital herpes. Over-the-counter cold sore medications containing menthol, camphor, and local anesthetics lessen pain and may protect against secondary bacterial infections, but they probably do not affect the progress of the viral infection. Some protection in suppressing cold sores can be obtained from the amino acid lysine, taken orally in the earliest phases of recurrence. However, current treatments do not reduce the load of the DNA matrix, and thus are unable to reduce the risk of further viral reactivation. Ideally, an ultimate weapon against HSV-1 infection should be durably present in the cells, avoid questions of sensitivity and, if possible, reduce the load of viral genomes. New antiviral treatments with the aim to eliminate the virus DNA by the action of endonucleases are in development.

Virus DNA detection of HSV in cornea have been performed from tear fluid (Fukuda, Deai et al. 2008), corneal scrapings (E1-Aa1, E1 Sayed et al. 2006) and corneal explants button from healthy and diseased patients (Crouse, Pflugfelder et al. 1990) using PCR-based methods (Llorente, Hidalgo et al. 1998; Gonzalez-Villasenor 1999; Kessler, Muhlbauer et al. 2000; O'Neill, Wyatt et al. 2003; Kimura, Ihira et al. 2005; Namvar, Olofsson et al. 2005; Strick and Wald 2006; Susloparov, Susloparov et al. 2006; Engelmann, Petzold et al. 2008; Sugita, Shimizu et al. 2008; Tanaka, Kogawa et al. 2009; Wada, Mizoguchi et al. 2009; Yu, Shi et al. 2009) or DNA/DNA hybridization on infected cells (Nago, Hayashi et al. 1988; Kotronias and Kapranos 1998). However, none of these methods allowed for both the detection and the nature of an infectious HSV genome in infected cells in a single analysis.

HSV is one type of viral disease for which a large spectrum of technologies for the diagnosis of HSV infection has been disclosed in various patents and publications as for example in EP0139416, EP0263025, WO0202131, WO2004036185, and in Matsumoto, Yamada et al. 1992, Kotronias and Kapranos 1998, Kessler, Muhlbauer et al. 2000, Namvar, Olofsson et al. 2005, Susloparov et al. 2006, Sugita, Shimizu et al. 2008, and Yu, Shi et al. 2009.

The most reliable method of diagnosis of the HSV infectious disease is to isolate the virus for determination, but this method required culture cells and requires several days for the determination. There are also immunological methods but they are mostly unreliable and difficult to perform, especially during the latency phase. A large number of PCR-based methods have been develop for enhanced sensitivity and faster time to result than is possible by conventional means but these methods do not allow analysis of the whole HSV genome and cannot predict the infectivity of the sample containing nucleotide sequences of the virus because the complete genome of the said virus is not tested. Direct methods of detection of the HSV genome in cells *in situ* hybridization (Nago, Hayashi et al. 1988; Kotronias and Kapranos 1998) or dot blot DNA-DNA hybridization (Matsumoto, Yamada et al. 1992) have been reported but these methods are not able to determine the type of HSV. There are instances in which rapid, sensitive, and specific diagnosis of HSV disease is imperative. There is therefore, a clinical need to develop a rapid and sensitive tool to aid in the diagnosis of HSV. There also remains a need for a tool for the typing of the HSV infection. Rapid identification of the specific etiological agent involved in a viral infection provides information which can be used to determine appropriate therapy within a short period of time.

Analysis of the configuration of HSV-1 genome following lytic or latent infection has been performed by the gel electrophoresis system of Gardella *et al.* (Gardella, Medveczky et al. 1984). In these gels circular molecules characteristically exhibit lower mobilities than the corresponding linear forms do and this has allowed the detection of episomal forms of latent herpes virus genomes. However, this technique does not discriminate between the isomer of HSV genome in infected cells or tissues. Other techniques like pulse-field gel electrophoresis (PFGE) have been used to provide insights into the mechanism of HSV DNA replication (Severini, Morgan et al. 1994). This family of tests is highly time-consuming and in all cases requires southern blotting, implying manipulation of radioactivity, long migration and/or exposure times.

Another virus for which numerous detection and analytic methods have been reported is Human Immunodeficiency Virus, or HIV, including HIV-1 and HIV-2. HIV-1 load in blood plasma, as measured by the number of copies of HIV-1 RNA, is a major laboratory marker widely used in clinical practice. Higher virus loads are directly linked to more rapid progression to AIDS in HIV-1-infected individuals. The effectiveness of highly active antiretroviral therapy (HAART) is also assessed by measuring the HIV-1 load in plasma. A patient is considered to be successfully treated by HAART when HIV-1 load in plasma stays below the detection limit of commercial assays which is currently 50 copies of HIV-1 RNA per ml of plasma. However, in spite of its clinical success, HAART cannot eradicate the virus, mainly due to the persistence of various viral reservoirs including latently infected resting CD4⁺ cells (Hermankova, Siliciano et al. 2003; Siliciano, Kajdas et al. 2003). Recent studies demonstrated that both virus replication and evolution do continue in some patients even when HIV-1 RNA in plasma is undetectable and therapy is otherwise considered to be successful (Frenkel, Wang et al. 2003; Havlir, Strain et al. 2003; Ramratnam, Ribeiro et al. 2004; Chun, Nickle et al. 2005; Tobin, Learn et al. 2005). HAART failure as a result of development of drug-resistant HIV-1 strains is a common problem (del Rio 2006). Thus, special attention should be given to characterizing HIV-1 residual replication by studying its molecular markers in peripheral blood mononuclear cells (PBMC). In particular, the amounts of cell-associated HIV-1 RNA and proviral DNA should be quantified. Of these, the amounts of proviral DNA may reflect the size of the pool of latently infected cells. However, systematic studies of the relationships between the cellular HIV-1 RNA/DNA levels and therapy outcome are hindered by the extremely low copy numbers of HIV-1 RNA/DNA in PBMC under HAART. Therefore, development of highly sensitive methods for quantification of cellular forms of HIV-1 RNA/DNA is essential.

Real-time reverse transcription-PCR (RT-PCR) is currently the preferred method for quantification of HIV-1 RNA/DNA in cells (Douek, Brenchley et al. 2002; Fischer, Joos et al. 2004). However, despite their accuracy and specificity, single-step real-time RT-PCR methods using the TaqMan detection chemistry are unable to reliably quantify <100 copies of HIV-1 RNA/DNA target per reaction in the context of total cellular RNA/DNA (Espy, Uhl et al. 2006). This evokes the possibility of yielding false-negative results when PBMC material from patients under HAART is studied, especially when limited amounts of clinical material are available for analysis. Methods that use SYBR green-based detection chemistry to detect HIV-1 RNA/DNA may be more sensitive (Espy, Uhl et al. 2006) but are prone to false-positive results because DNA binding dyes do not bind in a sequence-specific manner. With a theoretical detection limit of one molecule per reaction, nested PCR is considered a more sensitive method than real-time PCR. However, only semi-quantitative data can be produced with this method. In addition, it requires labor-intensive and time-consuming experimental procedures. In contrast as described below the inventors have discovered that Molecular Combing allows the detection of unique events and can be used as a highly sensitive method for the detection and quantification of the HIV proviral DNA in the infected cells of patients.

To diagnose viral infection, the use of antigen detection assays is on the increase, especially those based on monoclonal antibodies. Rapid nucleic acid detection using specific probes directed against DNA or RNA or amplification methods are other options for several viruses. These have the advantage of being so sensitive that they could conceivably detect the viral nucleic acid in a single infected cell. For example, PCR technique which allows the enzymatic amplification of minute quantities of DNA often undetectable by other methods, has been widely used for detection of part of genome of several viral agents such as HSV (Rowley, Whitley et al. 1990), human immunodeficiency virus (HIV) (Ou, Kwok et al. 1988) and human papilloma viruses (HPV) (Shibata, Arnheim et al. 1988).

A significant limitation of PCR is that it does not allow one to confirm the integrity of the complete genome detected and consequently cannot be a standard of characterization of complete infectious viral genome found in a tested sample. PCR thus also lacks the specificity required for testing the efficiency of an antiviral treatment against infectious virus particles because its results do not indicate whether the viral polynucleotides detected are infectious. Serological methods do not directly detect infectious viral polynucleotides, such as chromosomally-integrated viral genomic DNA, and require that a subject mount an immune response to a virus prior to detection or that a sufficient amount of viral antigen be present in a sample.

### Detection of viral oncogenes and activation of proto-oncogenes by virus

Many cancers originate from a viral infection; this is especially true in animals such as birds, but also in humans. Worldwide, the WHO International Agency for Research on Cancer estimated that in the year 2002 20% of human cancers were caused by infection of which 10-15% are caused by one of seven different viruses (Carrillo-Infante, Abbadessa et al. 2007). However, only a minority of persons or animals will go on to develop cancers after infection. Tumor viruses come in a variety of forms: viruses with a DNA genome, such as HPV (cervical cancer), Hepatitis B virus (liver cancer), and EBV (a type of lymphoma), and viruses with an RNA genome, like the Hepatitis C virus (HCV) can cause cancers, as can retroviruses having both DNA and RNA genomes (Human T-lymphotropic virus and hepatitis B virus, which normally replicates as a mixed double and single-stranded DNA virus but also has a retroviral replication component).

A direct oncogenic viral mechanism involves either insertion of additional viral oncogenic genes into the host cell (acutely-transforming virus) or to enhance already existing oncogenic genes (proto-oncogenes) in the genome (slowly-transforming virus) (for review, (Parsonnet 1999)). In acutely-transforming viruses the viral particles carry a gene that encodes for an overactive oncogene called viral-oncogene (v-onc) and the infected cell is transformed as soon as v-onc is expressed. In contrast, in slowly-transforming viruses, the virus genome is inserted, especially as viral genome insertion is obligatory part of retroviruses, near a proto-oncogene in the host genome. The viral promoter or other transcription regulation elements, in turn, cause over-expression of that proto-oncogene, which, in turn, induces uncontrolled cellular proliferation. Because viral genome insertion is not specific to proto-oncogenes and the chance of insertion near that proto-oncogene is low, slowly-transforming viruses have very long tumor latency compared to acutely-transforming virus, which already carries the viral-oncogene.

Some viruses are tumorigenic when they infect a cell and persist as circular episomes or plasmids, replicating separately from host cell DNA (Epstein-Barr virus and Kaposi's sarcoma-associated herpesvirus). Indirect viral oncogenicity also exists and involves chronic nonspecific inflammation occurring over decades of infection, as is the case for HCV-induced liver cancer. These two mechanisms differ in their biology and epidemiology: direct tumor viruses must have at least one virus copy in every tumor cell expressing at least one protein or RNA that is causing the cell to become cancerous. Foreign virus antigens are expressed in these tumors, consequently persons who are immunosuppressed such as AIDS or transplant patients are at higher risk for these types of cancers.

### Gene or cellular therapy based on the use viral vectors

Gene therapy involves the insertion of genes into an individual's cells and tissues to treat diseases, such as hereditary diseases where deleterious mutant alleles of a gene are replaced with functional ones. Although the technology is still in its infancy, it has been used with some success. Viral vectors are a tool commonly used by scientific to deliver such genetic material into tissues (gene therapy) or cells (cellular therapy). These viral vectors are mainly derived from lentiviruses, retroviruses, adenoviruses or herpes viruses (for review, (Thomas, Ehrhardt et al. 2003)).

The genetic material in lentiviruses or retroviruses is in the form of RNA molecules, while the genetic material of their hosts is in the form of DNA. The genome of these classes of viruses can be modified by inserting a gene sequence of interest to be transferred in the infected tissue or cells. As the wild type virus, the recombinant viruses will introduce its RNA together with some enzymes, namely reverse transcriptase and integrase, into the cell. This RNA molecule from the recombinant virus must produce a DNA copy from its RNA molecule before it can be integrated into the genetic material of the host cell. After this DNA copy is produced and is free in the nucleus of the host cell, it must be incorporated into the genome of the host cell. That is, it must be inserted into the chromosomes in the cell by another enzyme carried in the virus called integrase. If this host cell divides later, its descendants will all contain the new genes.

One of the problems of gene therapy that can result from the use of lentiviruses or retroviruses is that the integrase enzyme can insert the genetic material of the virus into any arbitrary position in the genome of the host; it randomly shoves the genetic material into a chromosome. If genetic material happens to be inserted in the middle of one of the original genes of the host cell, this gene will be disrupted (insertional mutagenesis). If the gene happens to be one regulating cell division, uncontrolled cell division (i.e., cancer) can occur. This problem has recently begun to be addressed by utilizing single-chain homing endonucleases (Grizot, Smith et al. 2009) or by including certain sequences such as the locus control region to direct the site of integration to specific chromosomal sites (Zhou, Zhao et al. 2007).

Adenoviruses are viruses that carry their genetic material in the form of double-stranded DNA. They cause respiratory, intestinal, and eye infections in humans (especially the common cold). When these viruses infect a host cell they introduce their DNA molecule into the host. The genetic material of the adenoviruses is not incorporated into the host cell's genetic material. The DNA molecule is left free in the nucleus of the host cell and the instructions in this extra DNA molecule are transcribed just like any other gene. The only difference is that these extra genes are not replicated when the cell is about to undergo cell division so the descendants of that cell will not have the extra gene. Adeno-associated viruses (AAV) from the parvovirus family are small viruses with a genome of single stranded DNA. The wild type AAV can insert genetic material at a specific site on chromosome 19 with near 100% certainty (Huser and Heilbronn 2003). But the recombinant AAV, which does not contain any viral genes and only the therapeutic gene, does not integrate into the genome. As a result, treatment with either adenoviral vector or AAV will require readministration in a growing cell population although the absence of integration into the host cell's genome should prevent the development of cancer (Douglas 2007).

Herpes viruses are currently used as gene transfer vectors due to their specific advantages over other viral vectors. Among the unique features of HSV derived vectors are the very high transgenic capacity of the virus particle allowing to carry long sequences of foreign DNA, the genetic complexity of the virus genome, allowing to generate many different types of attenuated vectors possessing oncolytic activity, and the ability of HSV vectors to invade and establish lifelong non-toxic latent infections in neurons from sensory ganglia from where transgenes can be strongly and long-term expressed. Three different classes of vectors can be derived from HSV: replication-competent attenuated vectors, replication-incompetent recombinant vectors and defective helper-dependent vectors known as amplicons. Replication-defective HSV vectors are made by the deletion of one or more immediate-early genes, e.g. ICP4, which is then provided in trans by a complementing cell line. Oncolytic HSV vectors are promising therapeutic agents for cancer. Such HSV based vectors have been tested in glioma, melanoma and ovarian cancer patients.

The inventors have surprisingly discovered that Molecular Combing and stretching techniques in combination with specially designed probes or sets of probes can be used to diagnose, detect or monitor subjects carrying infectious viral DNA and other pathogenic genes by means of Molecular Combing and/or DNA stretching techniques. These diagnostic applications are unknown in the prior art which generally performed Molecular Combing on DNA samples easily obtained from known cultured cell lines. While Molecular Combing has been previously applied for chromosomal analysis, it was not previously recognized that this methodology or adaptations of this methodology could be usefully applied to diagnosing viral infection or detecting other pathogenic polynucleotides in a sample. On the other hand, the inventors have surprisingly found that DNA obtained directly from biological samples from a virus-infected subject can be evaluated by Molecular Combing procedures and active, infectious viral DNA detected in these samples. These methods overcome many of the problems associated with existing diagnostic methods for virus infection and provide a convenient, rapid and accurate method for detection and monitoring of infectious viral polynucleotides in a virus-infected subject or patient.

### BRIEF SUMMARY OF THE INVENTION

The invention provides a reliable, simple, fast, and inexpensive way to detect rearrangements of a human papilloma virus (HPV) DNA, in a sample from a subject or patient using a Molecular Combing and/or DNA stretching techniques in combination with specific probes which are able to bind with at least 5%, 10%, 25%, 50%, 75%, especially sets of probes that bind to 80-100% of the HPV genome. The method of the invention is carried out *in vitro.*

The inventors have discovered that Molecular Combing is a powerful technique (i) to detect and quantify viral DNA or viral origin-DNA in infected mammalian cells, tissues or biological fluids, such as infectious herpes simplex virus DNA in infected cornea, (ii) follow viral replication and viral genomic rearrangements that occur in the infected mammalian cells tissues or biological fluids, and (iii) identify infectious virus polynucleotides or infectious virus polynucleotides present in infected cells, tissues or biological fluids.

Unlike prior art techniques for detecting virus in infected cells or tissues, the Molecular Combing techniques of the invention can be performed simultaneously and do not require multiple time consuming and expensive procedures. The methods of the invention are easily applicable as diagnostic tools for detecting viral contamination or infection in cells or tissues, useful for determining the efficacy of antiviral treatments by quantifying or otherwise evaluating the effects or efficacies of such treatments on the quantity of infectious virus or infectious viral polynucleotides or viral replication in infected mammalian cells or tissues. The inventors have found that Molecular Combing overcomes many of the problems associated with past methods of detecting or diagnosing a viral infection, including PCR-based methods, and offers an easy and rapid way to do so. Specific embodiments of the invention include the following.

A method for detecting rearrangements of a HPV DNA in a biological sample comprising extracting a polynucleotide from said sample; Molecular Combing said HPV DNA to form a stretched polynucleotide; contacting the stretched polynucleotide with a set of labelled probes specific for HPV DNA that recognize a HPV sequence; detecting hybridization of the probes to the combed HPV DNA, thereby detecting rearrangements of the HPV DNA. The polynucleotide may be infectious genomic viral DNA or infectious non-genomic viral DNA, an oncogene or a protooncogene and may be integrated into a chromosome of a subject or may be episomal or transgenic DNA. Detection of an entire viral genome or entire infectious viral DNA is importantly correlated with infection. As disclosed herein, regarding the HSV causative agents of herpetic keratitis, it is essential to be able to show the presence of complete infectious genomes in the cells which can then be partially or totally generated in the infected cells.

The biological sample is generally obtained directly from a subject or patient and may constitute tissue, a cell sample, or blood, CSF, or synovial fluid sample or other fluid biological sample. A DNA molecule or fiber for stretching or for Molecular Combing may be extracted from the biological sample. A sample is obtained from a living or non-living subject. Subjects include animals susceptible to viral diseases including humans, non-human mammals, such as cattle, bovines, sheep, goats, horses, pigs, dogs, cats and non-human primates; birds, such as chicken, turkey, duck, goose, ostrich, emu, or other birds; reptiles, amphibians and other animals.

A stretched or combed DNA molecular is contacted with a set of labelled probes specific for HPV DNA that recognize a HPV sequence. For detection of a HPV DNA, a set of probes covering 80-100% or any intermediate subrange or value of the HPV genome are employed. The HPV DNA may be one that is single or double stranded. As disclosed herein, representative DNA viruses include herpes virus, such as Herpes Simplex Virus (HSV) types 1 and 2, papillomaviruses, and hepatitis viruses, such as hepatitis B virus. Probes that bind to retroviruses, such as HIV, preferably a retrovirus integrated as a provirus into a host chromosome are also selected in a similar manner and preferably a set of probes covering 80-100% of the proviral genome are employed.

The probes may correspond to at least 5% and preferably 80-100% of the genome of a HPV strain already known to be infectious or may be designed to encompass the combination of genes known to be essential for replication, reproduction, or pathogenicity of a particular kind of virus.

Said probes can correspond to HPV16 (NC_001526.2)(SEQ ID NO: 20), HPV18 (XO5015.1)(SEQ ID NO: 21), HPV31 (J04353.1)(SEQ ID NO: 22), HPV33 (M12732.1)(SEQ ID NO: 23) and HPV45 (X74479.1)(SEQ ID NO: 24).

The probe set may comprise two, three or more different subsets of probes tagged with different labels. Preferably, such probes are used to determine the configurations of a genomic or infectious HPV DNA or to identify different portions or segments of a HPV genome or infectious HPV DNA molecule.

The disclosure also describes a method for detecting or identifying an infectious or genomic viral polynucleotide sequence in a mammalian cell, tissue or biological fluid comprising using Molecular Combing to detect the presence or the quantity of infectious viral polynucleotide or genomic viral polynucleotide in a cell, tissue or biological fluid. The disclosure describes a method for quantifying an infectious or genomic viral polynucleotide sequence comprising using Molecular Combing to detect quantity of infectious viral polynucleotide or genomic viral polynucleotide in a cell, tissue or biological fluid compared to an uninfected control sample or an otherwise similar sample obtained at a different point in time.

As described herein, methods for detecting the presence of a virus or detecting replication of a virus in a biological sample are also contemplated, as well as methods for longitudinally following virus replication or the rearrangement of a viral genome or infectious viral DNA especially in mammalian cells, tissue or biological fluid. Such methods may be performed by Molecular Combing DNA obtained from appropriate samples or samples taken over a specific time period for the presence of latent or replicating viral DNA or rearranged viral DNA in a cell or tissue.

The disclosure also describes a method for evaluating the efficacy of anti-viral treatment using Molecular Combing to detect the presence, arrangement or quantity of infectious or genomic viral DNA in a sample obtained from a subject, treating the subject with an anti-viral agent or performing other anti-viral therapy, and monitoring or re-evaluating the presence, arrangement or quantity of infectious or genomic viral DNA after treatment using Molecular Combing techniques of described herein.

The invention also relates to the use in the method of the invention of a kit comprising reagents, comprising 0.1% SDS for the lysis of viral particles in low-melting agarose plugs and one or more probes that bind to an infectious viral polynucleotide for detecting or identifying genomic viral HPV DNA, in a combed DNA molecule. In some embodiments the kits will contain a set of probes for detecting or identifying genomic viral DNA in a combed DNA molecule covering at least 5% of a HPV genome, especially 80, 85, 90, 95, 99 or 100%. The minimal length of the probe or set probe is 1 kb. The kit may further contain software for detecting or classifying the infectious sequences and the kit may additionally comprise instructions for detecting a HPV genome according to the methods of the invention.

### BRIEF DESCRIPTION OF THE DRAWINGS

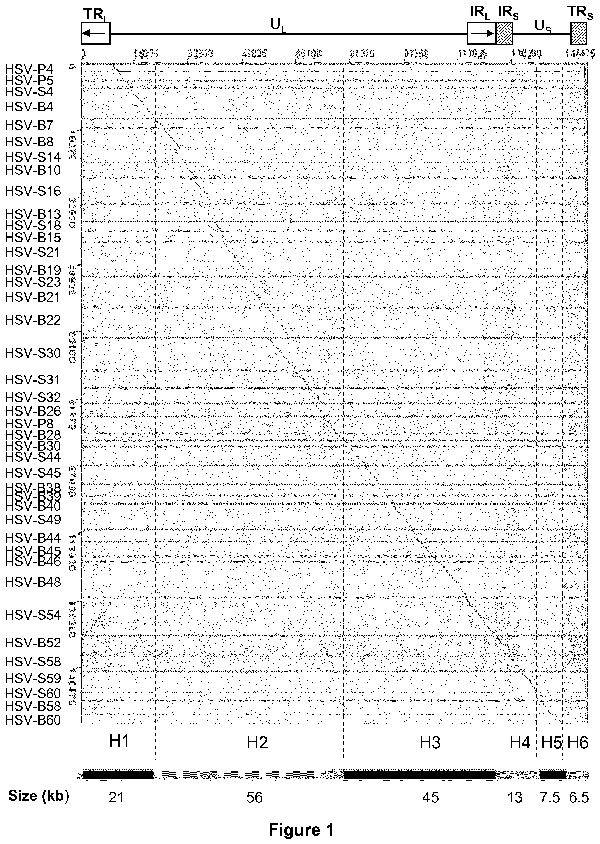

Figure 1. Example of HSV-1 specific-probes that can be used according to the invention. The dot plot represents the comparison between the HSV-1 genome sequence (horizontal axe) and the HSV-1 specific probes (vertical axe) to map the different probes to each other. Some of the HSV-1 fragments (HSV-S54, -B52 and - S58) hybridize with the inverted repeated sequences and consequently are present in two copies. The HSV-1 fragments listed in Table A can be associated and revealed in different colours to detect the HSV-1 genome. In this example, the association of 41 probes (on the 63 probes available, Table A) as follow allows covering 99% of the HSV-1 genome. The HSV-S54, -P4, -P5, -S4 and -B4 fragments correspond to the apparent H1 21kb probe. The apparent H2 56 kb probe consists of 19 overlapping probes (HSV-B7, -B8, -S14, -B10, -S16, -B13, - S18, -B15, -S21, -B19, -S23, -B21, -B22, -S30, -S31, -S32, -B26, -P8, and -B28 fragments). The 45 kb H3 probe is composed of the HSV-B30, -S44, -S45, -B38, -B39, -B40, -S49, -B44, -B45, -B46, -B48 and -S54 fragments. Due to the presence of the inverted repeated sequences, both H4 (13 kb) and H6 (6.5 kb) probes are composed of HSV-B52 and -S58 fragments; the HSV-S59 fragment is also present in the H4 probe. Finally, 3 overlapping fragments (HSV-S60, -B58 and -B60) formed the H5 7.5 kb probe. The localization probes which form a signature specific for a specific region of the HSV-1 genome is in red (digoxygenin labelling, black boxes) for H1, H3 and H5 probes and in green (biotin labelling, grey boxes) for the H2, H4 and H6 probes. The sizes (rounded to the nearest kb) of the localization probes are indicated respectively below each boxes.

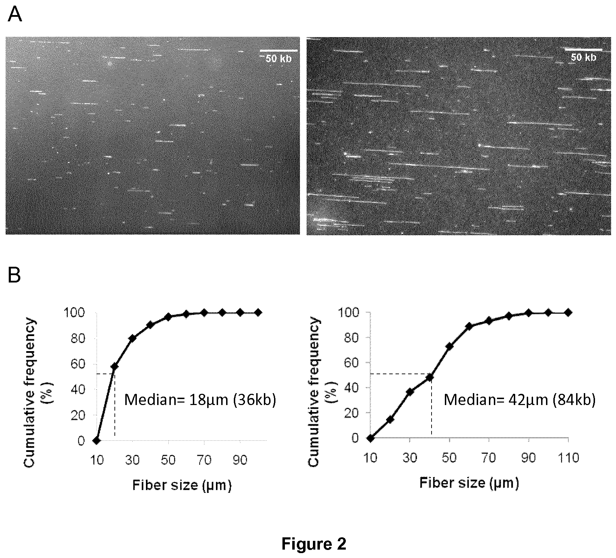

Figure 2. Comparison of two methods of HSV-1 DNA extraction from viral particles. A) Example of combed HSV-1 DNA on silanized surfaces. After combing of the HSV-1 DNA solution extracted with either the standard phenol chloroform (left picture) or the modified extraction protocol from agarose plug-embedded HSV-1 (right picture), the fibers are revealed with the intercalating agent YOYO-1 and observed on a epifluorescence microscope equipped with a camera. The figure shows two pictures that are characteristic of each extraction method. Scale is indicated as a bar. B) Histogram showing the cumulative frequency of the number DNA fiber sizes within a given length interval which were contained on genomic DNA isolated from HSV-1 particles. Interval width is 10 µm. Thus, for example, the third point represents the number of measures in the [30-40 µm] interval. Left panel shows the length of every HSV-1 DNA fibers that was recorded (654 measures) in the DNA solution extracted with the standard phenol:chloroform extraction. Median size of the fibers is 18 µm that is, 36 kb. The right panel show the distribution of the length of DNA fibers (2322 measures) obtained with the alternative method. In that case, the median size of the distribution is 42 µm that is, 84 kb.

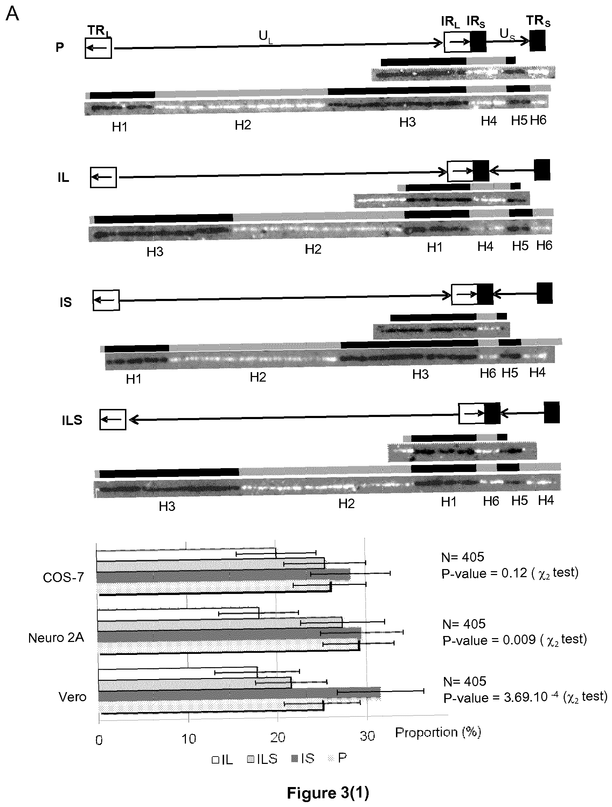

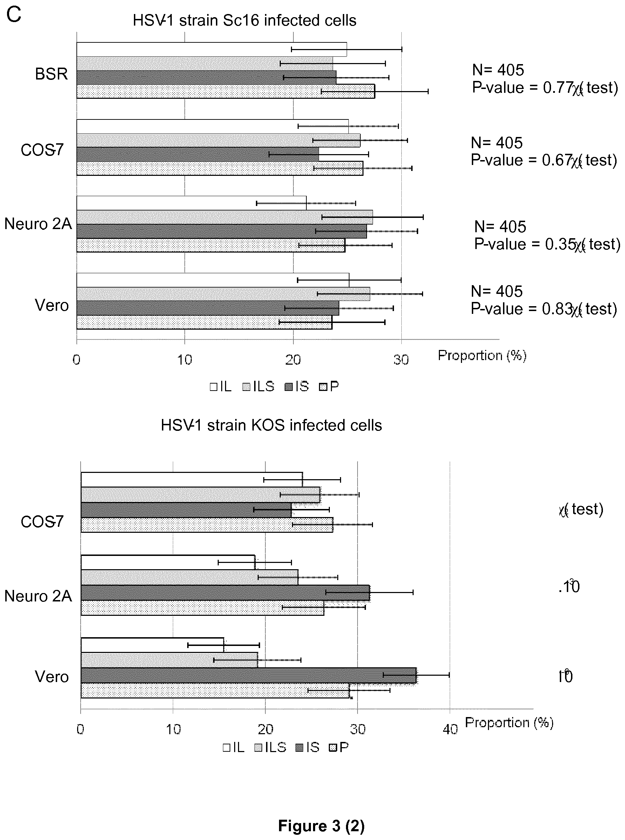

Figure 3. Detection of isomers of the HSV-1 genome in DNA solution extracted from viral particles and infected cells. A) Examples of FISH on combed HSV-1 DNA. Schematic representation of the different possible organization of the hybridization patterns corresponding to the different isomers of the HSV-1 genome are indicated (digoxygenin labelled-H1, H3 and H5 probes are represented in black boxes; biotin-labelled-H2, H4 and H6 probes are depicted in grey boxes). The minimal requirement hybridization patterns as defined in the "Analysis of HSV-1 detected signals" section are also indicated just above the complete signal. Four representative linear hybridization chains showing each complete isomer of HSV-1 genome (White: Texas Red/Alexa 594-fluorescence: H1, H3 and H5; Black: green Alexa 488-fluorescence: H2, H4 and H6). Schematic representations of each HSV-1 genome isomer are shown above the corresponding pictures. (B) Histogram of the distribution genome isomers of the HSV-1 KOS strain in viral particles produced in Vero, COS7 and Neuro2A cell lines. Hybridization signals were selected and analyzed as described in the "Examples" section. In this example, a total of 405 hybridization signals for each experiment were identified and classified. Each bar represents the number of each isomer of the HSV-1 genome. In this example, the distribution of the HSV-1 KOS strain isomers are equivalently distributed in viral particles from COS7 cells whereas the P and IS isomers are the more frequent isomer in the viral particles produced from the Neuro2A and Vero cell lines. This latter distribution is statistically different from an equimolar distribution (Chi-2 test). (C) Histograms of the distribution of the genome isomers of HSV-1 strains Sc16 and KOS in different infected cells (BSR, COS-7, Neuro 2A and Vero cells). The hybridization signals were selected and analyzed as described in the "Examples" section. In these examples, 405 signals from each production were selected and classified. The distribution of the HSV-1 strain Sc16 produced in BSR, COS-7, Neuro 2A and Vero cells and of the HSV-1 strain KOS produced in COS-7 is statistically equivalent to an equimolar distribution (Chi-2 test). In Neuro 2A and Vero infected cells, the P and IS isomers are more frequent that the IL and ILS isomers.

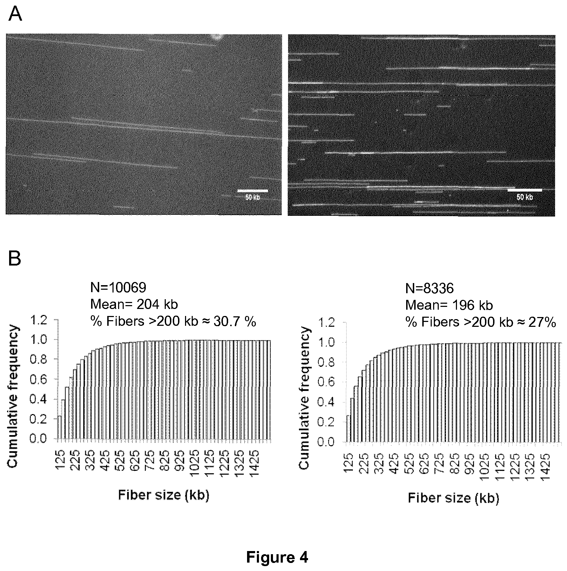

Figure 4. Method of extraction of genomic DNA from mouse and rabbit cornea. A) Example of combed genomic DNA on silanized surfaces. After extraction from cornea with the protocol described in Examples section, the genomic DNA solution is combed and revealed with the intercalating agent YOYO-1 before observation on an epifluorescence microscope equipped with a camera. Left pictures show a representative picture of combed DNA extracted from a healthy mouse cornea and the right picture from a healthy rabbit cornea. Molecular Combing is performed at low density to allow measurement of the length of the genomic DNA fiber. Scale is indicated as bar. B) Histogram showing the cumulative frequency of the number DNA fiber sizes within a given length interval which were contained on genomic DNA isolated from HSV particles. Interval width is 25 kb. Thus, for example, the fifth bar represents the number of measures in the [225-250 kb] interval. Left panel shows the length of every DNA fibers that was recorded (10069 measures) in the DNA solution extracted from the mouse cornea. Median size of the fibers is 204 kb. In this sample, around 31% of the genomic DNA fiber exhibited a size above 200 kb. The right panel show an example of the distribution of the length of DNA fibers (8336 measures) obtained from the rabbit cornea. In that case, the median size of the distribution is 196 kb with a proportion of fibers above 200 kb in length of 27%.

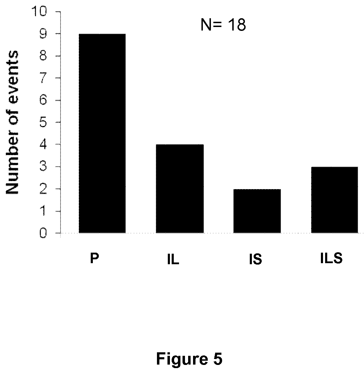

Figure 5. Histogram of the distribution of isomers of HSV-1 genome in infected mouse cornea. Total DNA from HSV-1 infected mouse cornea was extracted, combed and hybridized with the HSV-1-specific probes. In this example, a total of 18 hybridization signals were identified and classified. Each bar represents the number of each isomer of the HSV-1 genome.

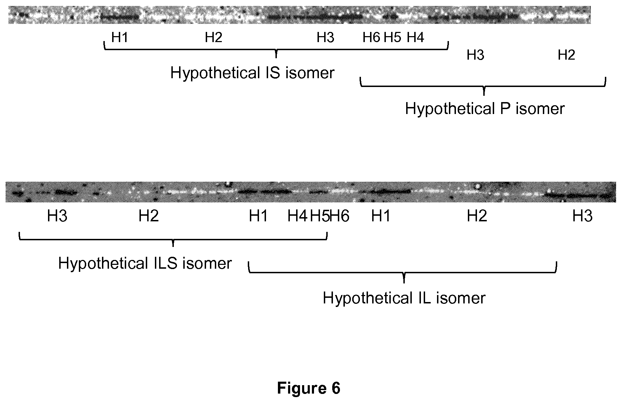

Figure 6. Detection of replication intermediate of the HSV-1 in HSV-1 infected mouse cornea. The figure shows two examples of complex HSV-1 genome DNA corresponding to replication concatemers (White signal: Texas Red/Alexa 594-fluorescence: H1, H3 and H5; Black signal: green Alexa 488-fluorescence: H2, H4 and H6).The upper picture depicted a signal compose of at least two hypothetical overlapping HSV-1 genomes composed of an IS and a P isomer while the lower picture show a HSV-1 concatemer consisting of at least hypothetical ILS and IL signals.

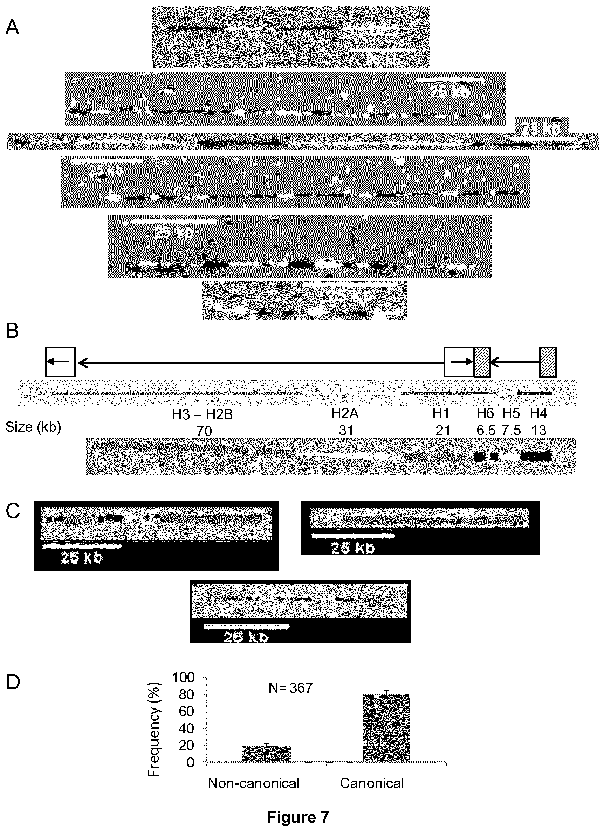

Figure 7. Detection of non canonical HSV-1 genomes in HSV-1 infected mouse cornea. A) Examples of non canonical HSV-1 from infected mouse cornea. The figure shows representative examples of hybridization patterns that do not correspond to one of the canonical isomers of the HSV-1 genome (White signal: Texas Red/Alexa 594-fluorescence: H1, H3 and H5; Black signal: green Alexa 488-fluorescence: H2, H4 and H6). Scale bar is indicated Canonical hybridization pattern corresponding to the ILS isomer (biotin-labelled H1, H2B and H3 probes are represented in grey boxes and signal; digoxygenin-labelled H2A and H5 are depicted in white boxes and signals, and Alexa488-labelled H4 and H6 probes are depicted in black boxes and signals) obtained on combed DNA extracts of HSV-1 strain Sc16-infected Vero cells. This example shows HSV-1 specific probes H1 to H6 labelled to evaluate the proportion of non-canonical structures in the H4/H6 region. C) Non canonical H4/H6 on HSV-1 strain Sc16 in Vero cell extracts. The first hybridization pattern shows alternation of Alexa 488 fluorescence signal (black signals) of various sizes corresponding to the H4/H6 probes and Alexa 594 fluorescence (grey signal) corresponding to fragments H1, H2B or H3 probes or AMCA/ Alexa 350 fluorescence signal (white signal) corresponding to part of H2A or H5 probes of a maximum of 10kb. The second example exhibit an alternation between Alexa 488 fluorescence signal (black signals) of various sizes and AMCA/ Alexa 350 fluorescence signal (white signal) that is surrounded by Alexa 594 fluorescence signal (grey signal). The third example shows a unique repetition of an Alexa 488 fluorescence signal (black signals) surrounded by Alexa 594 fluorescence signal (grey signal). Scale bar is indicated. D) Histograms of the distribution between canonical and non canonical structure in the H4/H6 regions of HSV-1. 367 hybridization signals were selected and analysed as described in the "Examples" section. In this example, 80% of the H4/H6 probes correspond to the theoretical structure.

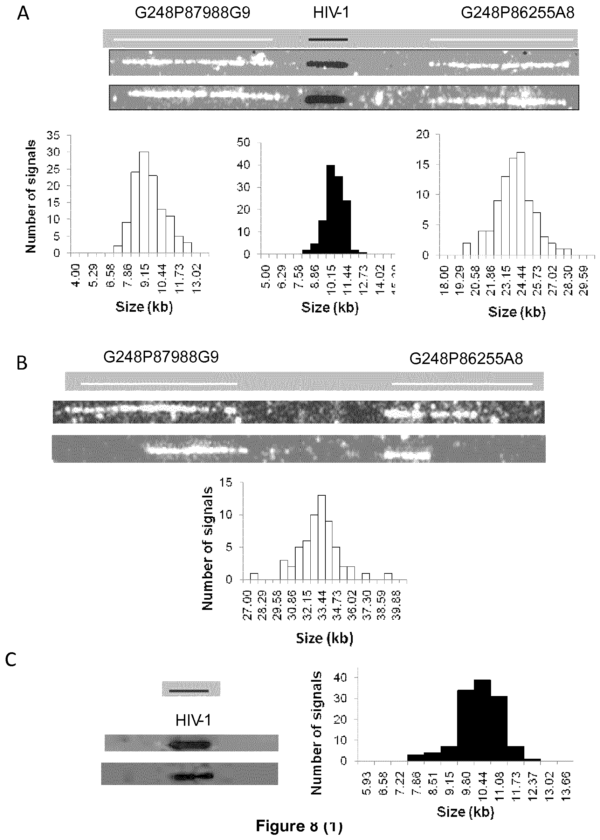

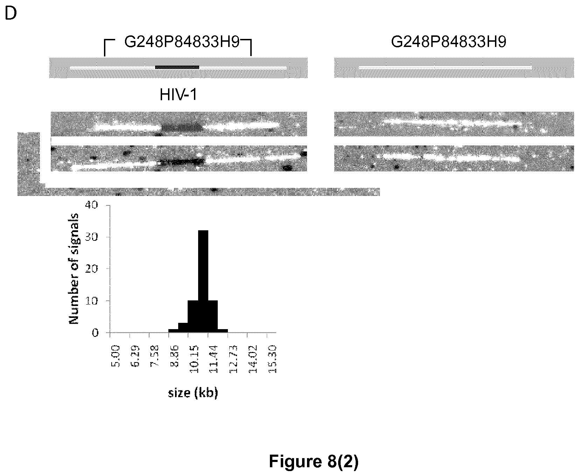

Figure 8. Detection of proviral HIV-1 DNA in ACH-2 cells culture. All histograms are showing number of signals within a given length interval (0.644 kb/class) in function of one FISH signal length or gap size between two FISH signals, in kilobases. Thus, for example, the second bar of the first histogram represents the number of measures in the [7.86-8.50 kb] interval. A) Examples of proviral HIV-1 DNA located at chromosome 7p15 using the G248P87988G9 and G248P86255A8 fosmids. Schematic representation of organization of the hybridization pattern corresponding to the integrated proviral HIV-1 DNA is indicated (digoxygenin labelled-HIV-1 probes are represented in a black box and signal; biotin-labelled- fosmids are depicted in white boxes and signals). Histograms on the left and right showing the distribution of the gap size between fosmid G248P87988G9 green Alexa 488-fluorescence signal and HIV-1 Texas Red/Alexa 594-fluorescence signal, and between HIV-1 Texas Red/Alexa 594-fluorescence signal and fosmid G248P86255A8 green Alexa 488-fluorescence signal, respectively. Middle histogram shows the distribution of the HIV-1 Texas Red/Alexa 594-fluorescence signal size. B) Examples of normal allele of the 7p15 locus. Schematic representation of organization of the hybridization pattern corresponding to the normal allele is indicated (biotin-labelled-fosmids are depicted in grey boxes and signals). Histogram showing the distribution of the gap size between fosmid G248P87988G9 green Alexa 488-fluorescence signal and G248P86255A8 green Alexa 488-fluorescence signal. C) Isolated proviral HIV-1 DNA. Schematic representation of organization of the hybridization pattern corresponding to the isolated form of HIV-1 is indicated (digoxygenin labelled- HIV-1 probes are represented in a black box and signal). Histogram shows the distribution of HIV-1 Texas Red/Alexa 594-fluorescence signal size. D) Examples of proviral HIV-1 DNA located at chromosome 7p15 using the G248P84833H9 fosmids. Schematic representations of organization of the hybridization patterns corresponding to the integrated proviral HIV-1 DNA (digoxygenin labelled- HIV-1 probes are represented in a black box and signal; and the wild type locus (biotin-labelled-G248P84833H9 fosmids are depicted in white boxes and signals) are indicated. The histogram shows the distribution of the HIV-1 Texas Red/Alexa 594-fluorescence signal size.

### DETAILED DESCRIPTION OF THE INVENTION

The invention enables a rapid, specific and sensitive detection of rearrangements of a HPV DNA in a sample that avoids the significant constraints imposed by amplification methods like PCR or serological tests such as ELISA.

In contrast to prior art methods, the Molecular Combing techniques of the invention permit the successful detection of complete viral genomes or infectious or virulent portions of viral polynucleotide sequences leading to improved diagnosis of acute or latent viral infections.

The method disclosed herein enables to detect the type of HSV and the structure of its genome reliably, in a time- and cost-effective fashion, and with none of the constraints of manipulating radioactivity. Moreover, the method disclosed herein enables to follow the presence of the viral infection after antiviral treatment, whatever the type of treatment considered.

The disclosure describes a method for detecting *in vitro* the presence of a genome of DNA viruses in infected cells of eukaryotic cells, in particular the detection of the HSV genome. Said method comprises a hybridization step of nucleic acid representative of given virus with at least a probe or a set of probes which is (are) specific for HSV DNA.

Molecular Combing techniques which may be used in accordance with the invention are disclosed by Bensimon, et al., U.S. Patent No. 6,303,296 and by Lebofsky, et al., WO 2008/028931. The inventors recognized that no technique can detect the presence of a complete or non-rearranged genome of infectious form of a virus except by traditional culture techniques and thus sought to apply and adapt these techniques to detection of infectious viral DNA. The combing method was never tested as an efficient tool to detect such viral DNA forms but only for the location of long sequences of DNA inserted in a cellular genome in view of understanding the genetic influence of such sequence in a specific genetic environment. Non-limiting examples of detection of infectious viral (HSV) DNA by the modified methods developed by the inventors are shown below.

The inventors modified the standard extraction protocol to isolate viral genomic DNA from viral particles. Generally, 0.1% SDS is used for the lysis of viral particles in the low-melting agarose plugs instead of 0.1% sarkosyl in the standard protocol. A methodology was also developed to extract genomic DNA from cornea to investigate the presence of HSV DNA in infected cornea for purposes of diagnosis.

The present invention relates to a method for detecting rearrangements of a HPV DNA contained on nucleic acid of infected cells, tissue or biological fluid. Said method comprises a hybridization step of nucleic acid representative of said HPV DNA with a set of labelled probes that cover the entire said HPV DNA and that permit to identify rearrangements within the nucleic acid representative of said HPV DNA.

The term "nucleic acid" and in particular "nucleic acid representative of viral DNA" as used herein designates one or several molecules of any type of nucleic acid capable of being attached to and stretched on a support as defined herein, and more particularly stretched by using Molecular Combing technology; nucleic acid molecules include DNA (in particular genomic DNA, especially viral DNA, or cDNA) and RNA (in particular mRNA). A nucleic acid molecule can be single-stranded or double-stranded.

"Nucleic acid representative of said HPV DNA" means that said nucleic acid contains the totality of the genetic information or the essential information with respect to the purpose of the invention, which is present on said HPV DNA. This term includes genomic HPV DNA, such as that integrated into a host chromosome and which can produce infectious virus, infectious HPV DNA which may lack certain genomic elements but can be infectious when expressed in particular host cells, and viral genes which exert a pathogenic effect on host cells, such as viral oncogenes.

A proto-oncogene includes those normal genes, which when altered by mutation can convert into oncogenes that causes a cell to grow or divide in an unregulated manner. Proto-oncogenes have diverse cellular functions, some provide signals for cell division, and others may play roles in apoptosis. Functional and structural characteristics of oncogenes, including their nucleic acid sequences, are well-known in the art and are described in Human Cancer Viruses: Principle of Transformation and Pathogenesis by J. Nicolas, et al., Karger Publishers (2010), ISBN3805585764, 9783805585767 (244 pages).

An oncogene encompasses a defective version of a proto-oncogene. A single copy of an oncogene can cause uncontrolled cell growth. Representative oncogenes include *ras, myc, src,* Her-2/neu, hTERT, and Bcl-2. Functional and structural characteristics of oncogenes, including their nucleic acid sequences, are well-known in the art and are described in Oncogene: Gene, Mutation, Tumor, Apoptosis, Gene Expression, Protein, Cell Growth, Cellular Differentiation, by L. M. Surhone, et al., Betascript Publishers (2010), ISBN6130361599, 9786130361594 (172 pages). Further description and identification of oncogenes is described in http://_www.cancerquest.org/index.cfm?page=780 (last accessed April 11, 2011) and in Cooper G. Oncogenes. Jones and Bartlett Publishers, 1995 and Vogelstein B, Kinzler KW; The Genetic Basis of Human Cancer. McGraw- Hill: 1998. The process of activation of proto-oncogenes to oncogenes can include viral transduction or viral integration, point mutations, insertion mutations, gene amplification, chromosomal translocation and/or protein-protein interactions. Viruses that can induce activation of proto-oncogenes include HBV and HCV (hepatocellular carcinoma), HTLV (leukaemia), HPV (cervical, anal and penile cancer), HSV-8 (Kaposi's sarcoma), Merkel cell polyomavirus (Merkel cell carcinoma) and, EBV (Burkitt's lymphoma, Hodgkin's lymphoma, post-transplantation lymphoproliferative disease and Nasopharyngeal carcinoma).

The nucleic acid sample used for stretching may be genomic DNA, in particular total genomic DNA or more preferably chromosomal genomic DNA (nuclear genomic DNA) of infected cells or tissues, and/or fragments thereof. The term "nucleic acid" is in particular used herein to designate a nucleic acid representative of one or several chromosome(s) and/or of one or several fragment(s) of chromosomes. Said fragments can be of any size, the longest molecules reaching several megabases. Said fragment are generally comprised between 10 and 2000 kb, more preferably between 20 and 500 kb and are in average of about 300 kb.

The nucleic acid sample used in the method of the invention is obtained from a biological fluid or from a tissue of biological origin, said sample or tissue being isolated for example from a human, a non human mammal or a bird.

As defined herein, a probe is a polynucleotide, a nucleic acid/polypeptide hybrid or a polypeptide, which has the capacity to hybridize to nucleic acid representative of virus DNA as defined herein, in particular to RNA and DNA. This term encompasses RNA (in particular mRNA) and DNA (in particular viral cDNA or viral genomic DNA) molecules, peptide nuclear acid (PNA), and protein domains. Said polynucleotide or nucleic acid hybrid generally comprises or consists of at least 100, 300, 500 nucleotides, preferably at least 700, 800 or 900 nucleotides, and more preferably at least 1, 2, 3, 4 or 5 kb. For example, probes of 1, 2, 3, 4, 5, 6, 7, 8, 9, 10, 11, 12, 13, 14, 15 kb or more than 15 kb, in particular 30, 50, 100 or 150 kb can be used. Such a probe or a set of probes may correspond or cover 5, 10, 15, 20, 25, 30, 35, 40, 45, 50, 55, 60, 65, 70,75, 80, 85, 90, 95, 98, 99 or 100% of a viral genome, especially that of an infectious virus. A nucleic acid probe may be a single or double-stranded polynucleotide or a modified polynucleotide. A set of continuous set of probes will cover a particular section of the genome or the entire genome or overlap each other with respect to this section. A suitable probe may be based on a viral polynucleotide sequence, such as those disclosed herein, and may be identified or synthesized by any appropriate method. Probes may be labelled or tagged radioactivity, fluorescently or by other means known in the art.

A polypeptide probe generally specifically binds to a sequence of at least 6 nucleotides, and more preferably at least 10, 15, or 20 nucleotides. As used herein, the sequence of a probe, when the probe is a polypeptide, should be understood as the sequence to which said polypeptide specifically binds.

By "a portion of" a particular region, it is meant herein consecutive nucleotides of the sequence of said particular region. A portion according to the invention can comprise or consist of at least 15 or 20 consecutive nucleotides, preferably at least 100, 200, 300, 500 or 700 consecutive nucleotides, and more preferably at least 1, 2, 3, 4 or 5 consecutive kb of said particular region. For example, a portion can comprise or consist of 1, 2, 3, 4, 5, 6, 7, 8, 9, 10, 11, 12, 13, 14, 15 consecutive kb of said particular region.

In a particular embodiment, the probe used or at least one of the probes used is a nucleotide variant of the probe showing a complementary sequence of 100% to a portion of one strand of the target nucleic acid. The sequence of said variant can have at least 70, 80, 85, 90 or 95% complementarity to the sequence of a portion of one strand of the target nucleic acid. Said variant can in particular differ from the probe which is 100% identical or complementary by 1 to 20, preferably by 1 to 10, nucleotide deletion(s), insertion(s) and/or more preferably substitution(s), in particular by, 1, 2, 3, 4, 5, 6, 7, 8, 9 or 10 nucleotide deletion(s), insertion(s) and/or more preferably substitution(s) in the original nucleotide sequence. In a particular embodiment, the variant keeps the capacity to hybridize, in particular to specifically hybridize, to the sequence of the nucleic acid target, similarly to the probe that is 100% identical or 100% complementary to a sequence of the nucleic acid target (in particular in the hybridization conditions defined herein).

The term "complementary sequences" in the context of the invention means "complementary" and "reverse" or "inverse" sequences, i.e. the sequence of a DNA strand that would bind by Watson-Crick interaction to a DNA strand with the said sequence.

The probes or one or several probes used to carry out the invention may be labelled with one or several hapten(s) (for example biotin and digoxygenin) and revealed with specific antibodies directed against these haptens. Use of different haptens for a given probe or set of probe will allow to detect rearrangements within a given viral DNA. Said probes can be labelled as defined herein and as described in patent application WO 2008/028931.

A set of probes as used herein comprises of at least two probes. For example, said set of probes can consist of 2 to 20 probes (2, 3, 4, 5, 6, 7, 8, 9, 10, 11, 12, 13, 14, 15, 16, 17, 18, 19 or 20 probes). The number of probes in a set does usually not exceed 10, 20, 30, 40, 50, 60, 70, 80, 90 or 100 probes depending on the sensitivity that is required and the length of the probe; a set of probes preferably consists of 2, 3, 4, 5, 6, 7, 8, 9 or 10 probes at the most.

The probes or the set of probes of the invention not only allow the detection of the entire whole genomic HPV DNA but also to identify rearrangements that can occur within the genomic HPV DNA. As described herein, for example, the probe sets used in the Example 1 for HSV detection allow the detection of the different HSV genome isomers that are generated by homologous recombination between the inversed repeated sequences that surround the unique regions U_{L} and Us. Moreover, each probes set (H1 to H6) for the detection of HSV are composed of several probes (3 to 19 different fragments) that are labeled with different two different haptens (digoxygenin for the fragments consisting of H1, H3 and H5 probes; biotin for the fragments consisting of H2, H4 and H6 probes). Changing the haptens of one or of several fragments by other haptens (whatever the nature of this hapten) within a given probe set permits the generation of a different color-fluorescence array that allows the identification of rearrangements within this specific region of the viral DNA.

Molecular Combing can also be a useful tool for an early diagnosis of patients susceptible of developing a cancer caused by a viral infection, for example, those at risk of conversion of a proto-oncogene into an oncogene. Indeed, Molecular Combing can discriminate between integrated and episomal viral genome. In the case where the viral DNA is integrated, by using a specific set of probes Molecular Combing will allow to determine whether the integrated viral DNA contains a complete viral oncogene or whether it is integrated in a close proximity of a cellular proto-oncogene.

The invention may also be used in conjunction with gene therapy. By using specific sets of probes for each transgene and/or viral vector, Molecular Combing provides a powerful tool for an evaluation of efficacy and safety of the viral vector-based gene therapy. Indeed, Molecular Combing can discriminate between integrated and episomal transgene. In the case where the transgene is integrated (lentiviruses and retrovirus based vectors), by using a specific set of probes Molecular Combing will allow to determine whether the integrated viral DNA is integrated in a gene (insertional mutagenesis) or in a close proximity of a cellular proto-oncogene. In the case the transgene is episomal (Adenoviral vector, AAV or HSV vector); Molecular Combing will be useful for the quantification of the transgene in the growing cell population and may help to define the right time for readministration of the transgene.

Specific embodiments of the invention include the following methods and products. A method for detecting rearrangements of a HPV DNA in a biological sample comprising: extracting a polynucleotide from said sample, Molecular Combing said HPV DNA to form a stretched polynucleotide, contacting said stretched polynucleotide with a set of labelled probes specific for HPV DNA that recognize a HPV sequence, detecting hybridization of the probes to the combed HPV DNA; thereby detecting rearrangements of the HPV DNA. This method is performed using a biological sample that is a tissue or cell sample obtained from a subject, for example, a blood, plasma, serum, CSF, synovial fluid sample or some other kind of biological fluid from the subject. A polynucleotide used in this method may be extracted from the tissue or cell sample or from components of a biological fluid obtained from the subject. Generally, the sample will be obtained from a living subject, but samples may also be obtained from deceased subjects. Samples can be obtained from humans or other mammals such as cattle, bovines, sheep, goats, horses, pigs, dogs, cats and non-human primates, or from other animals such as avian species such as a chicken, turkey, duck, goose, ostrich, emu, or other bird. The method can be practiced with polynucleotides which are DNAs which may also contain or comprise infectious or non-infectious genomic viral DNA or non-infectious non-genomic viral polynucleotides such as DNA or RNA. The polynucleotide detected or analyzed by this method may be integrated into the DNA of the subject or can be in episomal form. The polynucleotide to be detected can be contacted with one or more probes that bind to a DNA virus, including both single-stranded and double-stranded DNA viruses. As described herein, the polynucleotide may be contacted with one or more probes that bind to a herpes virus, such as herpes simplex virus (HSV). As described herein, other probes may be used which bind to other viruses like papilloma virus, hepatitis B virus, or retroviruses like HIV. In some embodiments the method will use a set of probes that bind to at least 80%, 90%, 95%, or 99% of the genomic or infectious DNA of a virus. The set of probes used in the method of the invention will comprise at least two subsets of probes that are tagged with different labels, for example, to permit the identification of different portions or segments of a viral genome to which the different probes bind or to permit the identification of rearrangements in a viral genome.

The disclosure also describes:

A method for detecting, identifying or visualizing an infectious or genomic viral polynucleotide sequence in a mammalian cell, tissue or biological fluid comprising using Molecular Combing to detect the presence or the quantity of infectious viral polynucleotide or genomic viral polynucleotide in a cell, tissue or biological fluid.

A method for quantifying an infectious or genomic viral polynucleotide sequence comprising using Molecular Combing to detect quantity of infectious viral polynucleotide or genomic viral polynucleotide in a cell, tissue or biological fluid compared to an uninfected control sample or an otherwise similar sample obtained at a different point in time or from a different source or clinical sample.

A method for detecting or following viral presence or replication or viral genomic rearrangements in a mammalian cell comprising Molecular Combing for the presence of latent or replicating viral DNA or rearranged viral DNA in a cell, tissue or biological fluid.

A method for evaluating the efficacy of anti-viral treatment comprising detecting using Molecular Combing the presence, arrangement or quantity of infectious or genomic viral DNA in a sample obtained from a subject, treating said subject with an anti-viral agent, and re-evaluating using Molecular Combing the presence, arrangement or quantity of infectious viral or genomic viral DNA in said subject.

In a particular embodiment, the invention relates to a set of probes covering 5-100% of the HPV genome.

The invention also relates to the use in the method of the invention of a kit comprising reagents, comprising 0.1% SDS for the lysis of viral particles in low-melting agarose plugs and one or more probes that bind to an infectious viral polynucleotide for detecting or identifying genomic viral HPV DNA in a combed DNA molecule. The kit may further compre software for detecting or classifying the infectious sequences.

The disclosure describes the following biological materials which have been deposited under the terms of the Budapest Treaty at the CNCM, Institut Pasteur 25 Rue du Docteur Roux, F-75724 Paris Cedex 15 on April 20, 2010: HSV-B4 (CNCM I-4298), HSV-B19 (CNCM I-4299), HSV-Sc54 (CNCM I-4300), and HSV-P4 (CNCM I-4301). Each biological material contains a polynucleotide fragment corresponding to a probe for the detection of HSV. The disclosure describes each of these polynucleotide fragments and to any of their combinations.

### EXAMPLES

### Example 1-Herpes Simplex Virus Detection

### Preparation of embedded DNA plugs from viral particles

HSV-1 DNA was extracted from viral particles by standard phenol:chloroform extraction (Ben-Zeev, Weinberg et al. 1974) or by a modified procedure described in Lebofsky *et al.* (Lebofsky, Heilig et al. 2006). Briefly, HSV-1 particles were resuspended in 1X PBS at a concentration of 5·10⁶ viral particles/mL, and mixed thoroughly at a 1:1 ratio with a 1.2% w/v solution of low-melting point agarose (Nusieve GTG, ref. 50081, Cambrex) prepared in PBS, at 50 °C. 90µL of the viral particles /agarose mix was poured in a plug-forming well (BioRad, ref. 170-3713) and left to cool at least 30 min at 4 °C. Embedded viral particles were lysed in 0.1% SDS - 0.5M EDTA (pH 8.0) solution at 50°C for 30 minutes. After three washing steps in 0.5M EDTA (pH 8.0) buffer of 10 minutes at room temperature, plugs were digested by overnight incubation at 50°C with 2 mg/mL Proteinase K (Eurobio code GEXPRK01, France) in 250 µL digestion buffer (0.5M EDTA, pH 8.0). The use of 0.1% SDS instead of Sarkosyl was very productive and allows a very high quality of extracted viral DAN to be collected.

### Preparation of embedded DNA plugs from infected cells

The extraction of HSV-1 DNA from infected cells culture (BSR, COS-7, Neuro 2A and Vero) was performed as previously described (Schurra and Bensimon 2009). Briefly, infected cells were pelleted by centrifugation at 5000g for 5 minutes, resuspended at a concentration of 2 ·10⁶cells/mL in 1X PBS buffer and mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 30 min at 4 °C.

Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed twice in a Tris 10mM, EDTA 1 mM solution for 30 in at room temperature.

### Preparation of embedded DNA plugs from infected cornea

HSV-1 strain Sc16 infected mouse cornea was collected at a final stage of infection, and kept in Corneamax® (Eurobio code EYEMAX00, France) medium. After rinsing three times during 15 minutes at room temperature with 1X PBS solution, the entire cornea was cut into small pieces. Tissue lysis was carried out for up to 16h at 37°C in 0.3 mg/mL Collagenase type A (Roche, code 10 103 578 001), and 0.8 mg/mL GIBCO™ Dispase (Invitrogen, France, code 17105-041), both prepared in GIBCO™ 1X Hanks' Balanced Salt Solution HBSS buffer (Invitrogen, France, code 14060040). Lysates were pelleted by centrifugation at 5000g for 10 minutes, resuspended at a concentration of 1•10⁶ to 2•10⁶cells/mL in 1X PBS buffer and mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 30 min at 4 °C.

Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed twice in a Tris 10mM, EDTA 1 mM solution for 30 in at room temperature.

### Final extraction of DNA and Molecular Combing

Plugs of embedded DNA from viral particles or corneas were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and Molecular Combing coverslips (20 mm x 20 mm, Genomic Vision S.A., Paris, France). The combed surfaces were dried for 4 hours at 60 °C.

### Syntheses and labelling of HSTV-1 Probes

The coordinates of all the probes relative to the Genbank sequence NC_001806.1 are listed in Table A. Probe size ranges from 1110 to 9325 bp in this example.

The HSV-1 specific probes were produced by either SacI or BspEI (New England Biolabs Inc., Beverly, MA, USA code R0156L and R0156L, respectively) enzymatic digestion of the HSV-1 scl6 strain obtained from the CNRS (Prof. Marc Labetoulle, laboratoire de virology moléculaire et structurale, UMR CNRS 2472-INRA 1157, Gif-sur-Yvette, France) or by long-range PCR using LR Taq DNA polymerase (Roche, kit code: 11681842001) using the primers listed in table B and the DNA from HSV-1 sc16 as template DNA. Sac*I* and BspE*I* HSV-1 fragments were ligated in SacI and XmaI-digested pNEB193 plasmid (New England Biolabs Inc., Beverly, MA, USA, code N3051S), respectively. PCR products were ligated in the pCR®2.1 vector using the TOPO® TA cloning Kit (Invitrogen, France, code K455040). The two extremities of each probe were sequenced for verification purpose. The apparent H1 (21 kb), H2 (56 kb), H3 (45 kb), H4 (13 kb), H5 (7.5 kb) and H6 (6.5 kb) probes are mixes of several adjacent or overlapping probes listed in table A. For the visualisation of the non canonical structure implicated the H4 and H6 probes, the apparent H2 probe was split in two probes H2A (31 kb) and H2B (25 kb).

The labelling of the probes was performed using conventional random priming protocols. For biotin-11-dCTP labelling, the BioPrime® DNA kit (Invitrogen, code: 18094-011, CA, USA) was used according to the manufacturer's instruction, except the labelling reaction was allowed to proceed overnight. For 11-digoxygenin-dUTP and Alexa488-7-OBEA-dCTP, the dNTP mix from the kit was replaced by the mix specified in table C. 200 ng of each plasmid was labelled in separate reactions. For isomer classification, H1, H3, H5 probes were labelled with 11-digoxygenin-dUTP while H2, H4 and H6 probes were labelled with biotin-11-dCTP. For the visualisation of the non canonical structure implicated the H4 and H6 probes, H1, H2B and H3 probes were labelled with biotin-11-dCTP, H2A and H5 probes with 11-digoxygenin-dUTP and H4, H6 with Alexa 488-7-OBEA-dCTP. The reaction products were visualized on an agarose gel to verify the synthesis of DNA.

### Hybridization of HSTV-1 probes on combed viral DNA and detection

Subsequent steps were also performed essentially as previously described in Schurra and Bensimon, 2009 (Schurra and Bensimon 2009). Briefly, a mix of labelled probes (250 ng of each probe, see below for details regarding probe synthesis and labelling) were ethanol-precipitated together with 10µg herring sperm DNA and 2,5µg Human Cot-1 DNA (Invitrogen, ref. 15279-011, CA, USA), resuspended in 20 µL of hybridization buffer (50 % formamide, 2X SSC, 0.5 % SDS, 0.5 % Sarkosyl, 10mM NaCl, 30 % Block-aid (Invitrogen, ref. B-10710, CA,USA). The probe solution and probes were heat-denatured together on the Hybridizer (Dako, ref. S2451) at 90 °C for 5 min and hybridization was left to proceed on the Hybridizer overnight at 37 °C. Slides were washed 3 times in 50 % formamide, 2x SSC and 3 times in 2x SSC solutions, for 5 min at room temperature. Detection antibody layers and their respective dilution in Block-Aid are described in table D and E. For each layer, 20 µL of the antibody solution was added on the slide and covered with a combed coverslip and the slide was incubated in humid atmosphere at 37 °C for 20 min. The slides were washed 3 times in a 2x SSC, 1 % Tween20 solution for 3 min at room temperature between each layer and after the last layer. For isomer classification, detection was carried out using a Texas Red coupled mouse anti digoxygenin (Jackson Immunoresearch, France) antibody in a 1:25 dilution for H1, H3, and H5 probes, and an Alexa488-coupled streptavidin antibody (Invitrogen, France) in a 1:25 dilution for H2, H4 and H6 probes as primary antibodies. As second layer, an Alexa594-coupled goat anti mouse (Invitrogen, France) diluted at 1:25 and a biotinylated goat antistreptavidin (Vector Laboratories, UK) diluted at 1:50 were used. To amplify the Alexa488-fluorescence signal of H2, H4 and H6 probes, an additional detection layer was realized by using the same Alexa488 coupled-streptavidin used for the first layer at a 1:25 dilution. For the visualisation of the non-canonical structure implicated the H4 and H6 probes, detection was carried out using an AMCA-coupled mouse anti-digoxygenin (Jackson Immunoresearch, France) antibody in a 1:25 dilution for the H2A and H5 probes, an Alexa 594-coupled streptavidin antibody (Invitrogen, France) in a 1:25 dilution for the H1, H2B and H3 and a rabbit anti Alexa 488 in a 1:25 dilution as primary antibodies. As second layer, an Alexa 594-coupled goat anti mouse (Invitrogen, France) diluted at 1:25, a biotinylated goat anti-streptavidin (Vector Laboratories, UK) diluted at 1:50 and an Alexa 488-coupled goat anti-mouse diluted at 1:25 were used. To amplify the Alexa 594-fluorescence signal of the H1, H2B and H3 probes and the AMCA/ Alexa 350 signal of the H2A and H5 probes, an additional detection layer was realized by using the same Alexa 594 coupled-streptavidin used for the first layer at a 1:25 dilution and an Alexa350 coupled goat anti rat diluted at 1:25, respectively. After the last washing steps, all glass cover slips were dehydrated in ethanol and air dried.

### Analysis of HSV-1 detected signals

For direct visualisation of combed HSV-1 fibers, cover slips were mounted with 20 µL of a Prolong (Invitrogen, France ref P36930)-YOYO-1 iodide (Molecular Probes, code Y3601) mixture (1/1000 v/v) and scanned with inverted automated epifluorescence microscope, equipped with a 40X objective (ImageXpress Micro, Molecular Devices, USA). Length of the YOYO-1-stained DNA fibers were measured and converted to kb using an extension factor of 2 kb/µm (Schurra and Bensimon 2009), with an internal software GVlab 04.2.1 (Genomic Vision S.A., Paris, France).

For isomers classification, hybridized-combed DNA from viral particles or cornea preparation were scanned without any mounting medium using an inverted automated epifluorescence microscope, equipped with a 40X objective (ImageXpress Micro, Molecular Devices, USA) and the signals can be detected visually or automatically by an in house software (Gvlab 0.4.2). Both FISH signals composed of a continuous signal of Texas Red/ Alexa 594-fluorescence for H1, H3, H5, and Alexa 488-flurorescence for H2, H4 and H6, and signals composed of a continuous signal corresponding to one of the pattern described below were considered:
(a) A minimum of 28kb long of Texas Red/Alexa 594-fluorescence signal, directly followed by an Alexa488-flurorescence signal corresponding to H4 and another Texas Red/Alexa594-fluorescence signal of a length minimal of 3kb.
(b) A minimum of 28kb long of Texas Red/Alexa 594-fluorescence signal, directly followed by an Alexa 488-flurorescence signal corresponding to H6 and another Texas Red/Alexa 594-fluorescence signal of a length minimal of 3kb.
(c) A minimum of 3kb an Alexa 488 fluorescence signal directly followed by a Texas Red/ Alexa 594 fluorescence signal corresponding to H1, next to an Alexa 488- fluorescence signal corresponding to H4 and another Texas Red/Alexa 594-fluorescence signal of a length minimal of 3kb.
(d) A minimum of 3kb an Alexa 488-flurorescence signal directly followed by a Texas Red/Alexa 594-fluorescence signal corresponding to H1, next to an Alexa 488- fluorescence signal corresponding to H6 and another Texas Red/Alexa 594- fluorescence signal of a length minimal of 3kb.

FISH signals selected were then classified depending of the pattern of the continuous FISH signals analysed:
The probe array composed of H1/H2/H3/H4/H5/H6 probes or the pattern (a) is classified as a Prototype (P) form of HSV-1 (Hayward, Jacob et al. 1975)
The pattern H1/H2/H3/H6/H5/H4 or the pattern (b) is classified as a Inverted Short (IS) genomic region form of HSV-1 (Hayward, Jacob et al. 1975)
The pattern H3/H2/H1/H4/H5/H6 or the pattern (c) is classified as a Inversed Long (IL) genomic region of HSV-1 (Hayward, Jacob et al. 1975)
The pattern H3/H2/H1/H6/HS/H4 or the pattern (d) is classified as a Inversed Long and Short (ILS) genomic region form of HSV-1 (Hayward, Jacob et al. 1975)

The hypothesis that the observed distribution differ significantly to the expected distribution (an equivalent number of events for the four isomers, (Bataille and Epstein 1997) was tested by a chi-square test, and accepted when the p-value observed was below 0.05.

For the visualisation of the non canonical structure implicated the H4 and H6 probes, hybridized-combed DNA from infected cells preparation were scanned without any mounting medium using an inverted automated epifluorescence microscope, equipped with a 40X objective (ImageXpress Micro, Molecular Devices, USA) and the signals can be detected visually on an in house software (Gvlab 0.4.2). All signals composed of a continuous signal of Alexa 488-fluorescence for H4 and H6 were selected. Signals corresponding to one of the pattern below were classified as canonical structure:
(e) A continuous signal composed of a minimum of 3kb of an Alexa 594-fluorescence signal, followed of 13 kb of an Alexa 488-flurorescence signal corresponding to the H4 probe, 7.5 kb of an AMCA/ Alexa 350-fluorescence signal corresponding to the H5 probe and 7 kb of an Alexa 488-flurorescence corresponding to the H6 probe.
(f) A continuous signal composed of a minimum of 3kb of an Alexa 594-fluorescence signal, followed of 7 kb of an Alexa 488-flurorescence signal corresponding to the H6 probe, 7.5 kb of an AMCA/ Alexa 350-fluorescence signal corresponding to the H5 probe and 13 kb of an Alexa 488-flurorescence corresponding to the H4 probe.

All other signals were classified as non canonical structure. The proportions of the canonical and non canonical structure were compared.

### Extraction of HSV-1 DNA from viral particles

During sample preparation many DNA molecules are sheared at random location due to uncontrolled manipulation forces resulting in high variability in the size of DNA prepared. It has been showed that high molecular weight DNA can be stretched on by Molecular Combing using a glass coverslip when it is deproteinised in a molten agarose plug (Lebofsky and Bensimon 2003). Thus, the analyzed DNA molecules are of variable length, with an average of about 300 kb, the longest molecules reaching several megabases.

Since HSV-1 DNA has never been used for Molecular Combing, the inventors first evaluated the quality of the DNA fibers in terms of length extracted by two different methods: the standard phenol:chloroform extraction and the method described by (Lebofsky, Heilig et al. 2006) that have been slightly modified as described in the "Example" section.

As shown in Fig. 2, no fiber of size above 80 µm was detected. This is in concordance with the maximum expected size for the HSV-1 genome (152 kb) considering a constant elongation factor of 2 kb. With both methods, we do not detect only 152 kb long DNA fibers because there is still random DNA shearing due to the mechanical manipulation that cannot be avoided and because DNA molecules shorter than the full-length standard HSV-1 viral genome can become encapsidated within nuclear capsids (Vlazny, Kwong et al. 1982).

However, the median size of DNA fibers is 36 kb with 1.2 % of fiber longer than 140 kb when extraction has been performed with the standard phenol:chloroform method. In contrast, the median size of HSV-1 DNA fiber is 84 kb with 2.5% of fiber longer than 140 kb when the extraction of DNA from agarose plug-embedded viral particles using our alternative protocol has been realized. Although the proportion of long molecules is low, there are a lot of long combed DNA molecules available for analysis since there are several ten thousand fibers combed on a glass coverslip.

These results indicate that the alternative method developed by the inventors improved the quality of combed DNA extracted from viral particles compared to standard method allowing analysis by Molecular Combing.

### Structure of the HSV-1 genome in viral particles and its distribution

The inventors applied Molecular Combing to uniformly stretch the HSV-1 DNA extracted from viral particles and infected cells and hybridized the resulting combed HSV-1 DNA with labeled adjacent and overlapping HSV-1-specific DNA probes (Fig. 1; H1, H3, H5: red Texas Red/Alexa 594-fluorescence; H2, H4, H6: green Alexa 488-fluorescence) to determine the structure of the HSV-1 genome (Fig. 3A). Immunofluorescence microscopy (Fig. 3B.) exhibited 405 multicolor linear patterns for each production of HSV-1 KOS strain viral particles produced from COS7, Vero and Neuro2 A cell lines that fulfilled the criteria for evaluation (see "Examples" section). Classification of the signal showed that the distribution of the HSV-1 KOS strain isomers are equivalently distributed in viral particles from COS7 cells whereas the P and IS isomers are the more frequent isomer in the viral particles produced from the Neuro2A and Vero cell lines.

On hybridized combed HSV-1 infected cells, the distribution of the four isomers was compared between Sc16 and KOS HSV-1 strains produced in different cell lines (BSR, COS7, Neuro2A and Vero). 405 multicolor linear patterns corresponding to each production and that fulfilled the criteria for evaluations (see "Experimental procedures" section) were classified. The distribution between the four isomers was statistically equivalent (Chi2 test) in all production of HSV-1 Sc16 (in BSR, Vero, Neuro 2a and COS-7 cells). In the same way, the distribution was found equivalent for HSV-1 strain KOS produced in COS-7 cells. Strikingly, for the first time, the inventors have found that the IS and P isomers are the predominant forms of the HSV-1 DNA strain KOS preparation from Vero and Neuro2A cells while IL isomers is the less present isomers.

The inventors have found that the Molecular Combing techniques as described herein are powerful methods for analysis of the structure of the HSV-1 genome DNA at the level of the unique molecule and to quantify its distribution in a biological sample.

### Extraction of genomic DNA from mouse and rabbit cornea

Molecular Combing has been successfully performed with DNA solution from isolated cells including cultured cells (i.e., established cell strains, immortalized primary cells) or biological fluids (i.e., peripheral blood lymphocytes, amniotic cells) (Gad, Klinger et al. 2002; Caburet, Conti et al. 2005). However, the human cornea is a solid tissue with a complex structure composed of 5 layers: the corneal epithelium, the collagen-rich Bowman's membrane, the corneal stroma which consisting of regularly-arranged collagen fibers along with sparsely distributed interconnected keratocytes), the acellular Descemet's membrane and, the corneal endothelium. In order to extract genomic DNA from cornea, the inventors developed a specific method to isolate corneal cells before proceeding with the standard procedure. Different methods including mechanical disruption and enzymatic digestion of cornea were tested. The latter was given the best results and was optimized using different types of proteases (i.e., trypsin, collagenase A, dispase....) at different concentration. Figure 4A and B shows an example of results obtained with both mouse and rabbit cornea that were digested with 0.3 mg/ml collagenase A and 0.8 mg/ml Dispase for 16h. As for standard extraction for Molecular Combing genomic DNA is broken at random locations. Thus, the analyzed genomic DNA molecules are of variable length, with an average of about 200 kb, the longest molecules above 1 Mb (megabases) for both type of cornea. The size of DNA fiber extracted from cornea is slightly inferior to the typical size that can be obtained from isolated cells (an average of about 300 kb with the longest molecules reaching several megabases).

### Detection of HSV-1 infection in mouse cornea

The inventors therefore adapted and applied Molecular Combing on DNA extracted from HSV-1 infected mouse cornea and hybridized with the HSV-1 specific probes as described above. As shown in Fig. 5, this enabled detection all the types of isomers of HSV-1 genome in mouse infected cornea.

In addition to the detection of mature HSV-1 genome, the Molecular Combing procedures of the invention allow the detection of concatemers (Fig. 6) indicating that the virus is actively replicating in the cornea of the analyzed sample.

### Detection of non-canonical forms in infected cells and mouse cornea

The inventors detected non-canonical structure of the HSV-1 genome (Fig. 7A) that probably arises from recombination during the replication of the virus in infected mouse cornea extract and in infected cells extracts.

The labelled adjacent and overlapping HSV-1 specific probes were hybridized on combed DNA extracts from HSV-1 strain Sc16 infected Vero cells (Fig. 7B; H1, H2A, H3: red Alexa 594-fluorescence that appears in grey; H2B, H5: blue AMCA/ Alexa 350-fluorescence that appears in white; H4, H6: green Alexa 488-fluorescence that appears in black) to evaluate the proportion of the non-canonical structures in the H4/H6 region. A total of 367 multicolour linear patterns were classified, 20% (73) are found to have non-canonical H4/H6 structure.

Molecular Combing enables the visualisation of non canonical structure and by their infinity of combination of barcode possible is a powerful method to analyse them.

**TABLE A**

| Name | Start | End | Size |
|---|---|---|---|
| HSV-B1 | 1 | 1323 | 1323 |
| HSV-B2 | 1324 | 7259 | 5936 |
| HSV-P4 | 9237 | 11276 | 2004 |
| HSV-P5 | 11090 | 13245 | 2156 |
| HSV-Sc4 | 13088 | 14971 | 1884 |
| HSV-P6 | 14554 | 17565 | 3065 |
| HSV-B4 | 14827 | 22438 | 7595 |
| HSV-Sc7 | 17853 | 20762 | 2910 |
| HSV-B7 | 22953 | 25152 | 2200 |
| HSV-Sc11 | 23400 | 25447 | 2048 |
| HSV-B8 | 25153 | 29997 | 4845 |
| HSV-Sc14 | 27944 | 31215 | 3272 |
| HSV-B10 | 30903 | 34697 | 3795 |
| HSV-Sc16 | 33044 | 39471 | 6428 |
| HSV-B13 | 35891 | 40272 | 4382 |
| HSV-Sc18 | 40315 | 42288 | 1974 |
| HSV-B15 | 41017 | 43898 | 2882 |
| HSV-Sc21 | 42621 | 47358 | 4738 |
| HSV-P8 | 44192 | 46987 | 2795 |
| HSV-B18 | 44682 | 47174 | 2493 |
| HSV-B19 | 47175 | 50931 | 3757 |
| HSV-Sc23 | 49040 | 51392 | 2353 |
| HSV-B21 | 50959 | 56138 | 5180 |
| HSV-Sc24 | 51393 | 53348 | 1956 |
| HSV-Sc25 | 53349 | 56049 | 2701 |
| HSV-B22 | 56139 | 63370 | 7232 |
| HSV-Sc30 | 56775 | 64599 | 7825 |
| HSV-Sc31 | 64600 | 69017 | 4418 |
| HSV-B24/25 | 65757 | 66423 | 4740 |
| HSV-Sc32 | 69018 | 72802 | 3785 |
| HSV-B26 | 70497 | 73717 | 3221 |
| HSV-P8 | 73229 | 77332 | 4103 |
| HSV-B27 | 73718 | 77164 | 3447 |
| HSV-B28 | 77165 | 79105 | 1941 |
| HSV-B30 | 79937 | 81056 | 1120 |
| HSV-Sc44 | 80991 | 85801 | 4811 |
| HSV-B31 | 81507 | 83780 | 2724 |
| HSV-B33 | 84298 | 85576 | 1279 |
| HSV-Sc45 | 85802 | 90164 | 4363 |
| HSV-B35 | 86304 | 87747 | 1444 |
| HSV-B38 | 89249 | 90453 | 1205 |
| HSV-B39 | 90453 | 92176 | 1723 |
| HSV-B40 | 92177 | 94195 | 2019 |
| HSV-Sc47 | 91545 | 93723 | 2179 |
| HSV-Sc49 | 94122 | 100285 | 6164 |
| HSV-B41 | 94196 | 96148 | 1953 |
| HSV-B42 | 96149 | 99454 | 3306 |
| HSV-B44 | 99492 | 102440 | 2949 |
| HSV-B45 | 102441 | 106065 | 3625 |
| HSV-B46 | 106066 | 107175 | 1110 |
| HSV-B48 | 108009 | 116579 | 8571 |
| HSV-P8c | 109960 | 113148 | 3188 |
| HSV-Sc54 | 115744 | 125068 | 9325 |
| HSV-B52 | 125044 | 129901 | 4858 |
| HSV-Sc56 | 125079 | 128601 | 3523 |
| HSV-Sc58 | 129089 | 133046 | 3958 |
| HSV-B55 | 130841 | 135542 | 4702 |
| HSV-Sc59 | 133047 | 137945 | 4899 |
| HSV-B56 | 135543 | 137690 | 2148 |
| HSV-Sc60 | 137946 | 140155 | 2210 |
| HSV-P8d | 138148 | 139821 | 1673 |
| HSV-B58 | 138757 | 141926 | 3170 |
| HSV-B60 | 142840 | 145515 | 2276 |
| HSV-Sc64 | 144918 | 149148 | 4231 |

**TABLE B:**

| Probes | Forward Primer | Reverse Primer |
|---|---|---|
| HSV-P4 | TGG TTG TGT TAC TGG GCA AA (SEQ ID NO: 1) | TCG ATC GAC GAC ACC ATA AA (SEQ ID NO: 2) |
| HSV-P5 | CAG ATA CGA CTC CCG CAG AT (SEQ ID NO: 3) | CGA CGA CCT CGA CGT TAT TT (SEQ ID NO: 4) |
| HSV-P6 | CGT GAG GTC CAA AAT CAC CT (SEQ ID NO: 5) | GAC AGG CAA GCT CAA AGT CC (SEQ ID NO: 6) |
| HSV-P8 | AGA TGT CCA CGA GCA CCA G (SEQ ID NO: 7) | CCT GAC TTT GTG GGG CTA AA (SEQ ID NO: 8) |

Primers sequences used for the synthesis of probes by long-range PCR. An extract of DNA from HSV-1 strain Sc16 is used as template.

**TABLE C:**

| | | |
|---|---|---|
| Labelling | Non-labelled dNTPs (Invitrogen, ref. 10297-018, CA, USA) | Labelled dNTP (Roche, ref. 11 558 706 910, France) |
| Dig-dUTP | dATP, dCTP, dGTP 40 µM each dTTP 20µM | Dig-11-dUTP 20 µM |
| Alexa488-7-OBEA-dCTP | dATP, dTTP, dGTP 40 µM each dCTP 20µM | Alexa488-7-OBEA-dCTP 20µM |

Mixes used in replacement of the dNTP mix of the random priming kit for labelling with dig-dUTP and Alexa488-7-OBEA-dCTP. The concentrations indicated are the final concentration in the labelling reaction. The non-labelled dNTPs and the labelled dNTP were added together in replacement of the provided dNTP mix intended for labelling with biotin-11-dCTP-.

**TABLE D:**

| Description | Abbreviation | Supplier |
|---|---|---|
| Streptavidin, coupled to Alexa Fluor 488 | Strep/A488 | Invitrogen (France, S11223) |
| Goat anti-streptavidin, coupled to biotin | G anti-strep/biotin | Vector Laboratories (France; BA-0500) |
| Mouse anti-dig, coupled to Texas Red | M anti-DIG/TR | Jackson Immuno Research (France; 200-072-156) |
| Goat anti-mouse, coupled to A594 | G anti-M/A594 | Invitrogen (France; A11005) |
| Streptavidin, coupled to Alexa Fluor 594 | Strep/A594 | Invitrogen (France, S11227) |
| Mouse anti-Dig, AMCA coupled | M anti-DIG/AMCA | Jackson Immuno Research (France; 200-152-156) |
| Rat anti-mouse, AMCA coupled | R anti-M/AMCA | Jackson Immuno Research (France; 415-155-166) |
| Goat anti-rat, Alexa 350 coupled | G anti-R/A350 | Invitrogen (France, A21093) |
| Rabbit anti Alexa 488 | R anti-A488 | Invitrogen (France, A11094) |
| Goat anti mouse, coupled Alexa 488 | G anti-M/A488 | Invitrogen (France, A11001) |

List of antibodies and other hapten-binding molecules used for the detection of probes.

**TABLE E:**

| | 1^{st} layer | 2^{nd} layer | 3^{rd} layer |
|---|---|---|---|
| 1-color scheme | | | |
| Biotin / green | strep/A488 (1/25) | Goat anti-strep/biotin (1/50) | strep/A488 (1/25) |
| 2-color scheme | | | |
| Biotin / green | strep/A488 (1/25) | Goat anti-strep/biotin (1/50) | strep/A488 (1/25) |
| Dig / red | Mouse anti-DIG/TR (1/25) | Goat anti-M/A594 (1/25) | - |
| 3-color scheme | | | |
| Biotin / red | Strep/A594 (1/25) | G anti-strep/biotin (1/50) | Strep/A594 (1/25) |
| Dig / blue | M anti-DIG/AMCA (1/25) | R anti-M/AMCA (1/25) | G anti-R/A350 (1/25) |
| A488/ green | R anti-A488 (1/25) | G anti-M/A488 (1/25) | - |

Composition of the 2 or 3 layers for the detection of probes by fluorescence. The dilution for each detection agent is indicated in brackets. The abbreviations refer to table D.

### Example 2-Human Immunodeficiency Virus Detection

### Preparation of embedded DNA plugs from ACH-2 cells culture

ACH-2 cell lines (Clouse, Powell et al. 1989) were cultivated according to the authors' instructions. DNA was extracted as described in (Schurra and Bensimon 2009). Briefly, cells were resuspended in 1 X PBS at a concentration of 10⁷ cells / mL mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 30 min at 4 °C. Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed twice in a Tris 10mM, EDTA 1 mM solution for 30 in at room temperature.

### Final extraction of DNA and Molecular Combing

Plugs of embedded DNA from ACH-2 cells were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and Molecular Combing coverslips (20 mm x 20 mm, Genomic Vision S.A., Paris, France). The combed surfaces were dried for 4 hours at 60 °C.

### Syntheses and labelling of HIV-1 Probes

The coordinates of the three probes relative to the Genbank sequence M19921.1 are listed in table F. Probe size ranges from 2927 to 3749 bp in this example.

The HIV specific probes were produced by long-range PCR using LR Taq DNA polymerase (Roche, kit code: 11681842001) using the primers listed in table G and the DNA from HIV pNL4-3 as template DNA. PCR products were ligated in the pCR®2.1 vector using the TOPO® TA cloning Kit (Invitrogen, France, code K455040). The two extremities of each probe were sequenced for verification purpose.

Two fosmids G248P87988G9 and G248P86255A8 flanking the insertion site of HIV-1 or one fosmid G248P84833H9 encompassing the HIV-1 provirus insertion site in ACH-2 cells (Ishida, Hamano et al. 2006), according to Human Mar. 2006 Assembly (NCBI Build 36.1), and the HIV-1 probes were labeled using conventional random priming protocols. For biotin-11-dCTP labelling, the BioPrime® DNA kit (Invitrogen, code: 18094-011, CA, USA) was used according to the manufacturer's instruction, except the labelling reaction was allowed to proceed overnight. For digoxygenin-11-dUTP, the dNTP mix from the kit was replaced by the mix specified in table C. 200 ng of each plasmid/ fosmid was labelled in separate reactions. For entire HIV-1 detection, HIV-1 was labelled with digoxygenin-11-dUTP while fosmids were labelled with biotin-11-dCTP. The reaction products were visualized on an agarose gel to verify the synthesis of DNA.

### Hybridization of HIV-1 probes on combed viral DNA and detection

Subsequent steps were also performed essentially as previously described in Schurra and Bensimon, 2009 (Schurra and Bensimon 2009). Briefly, a mix of labelled probes (250 ng of each probe) were ethanol-precipitated together with 10µg herring sperm DNA and 2,5µg Human Cot-1 DNA (Invitrogen, ref. 15279-011, CA, USA), resuspended in 20 µL of hybridization buffer (50 % formamide, 2X SSC, 0.5 % SDS, 0.5 % Sarkosyl, 10mM NaCl, 30 % Block-aid (Invitrogen, ref. B-10710, CA,USA). The probe solution and probes were heat-denatured together on the Hybridizer (Dako, ref. S2451) at 90 °C for 5 min and hybridization was left to proceed on the Hybridizer overnight at 37 °C. Slides were washed 3 times in 50 % formamide, 2x SSC and 3 times in 2x SSC solutions, for 5 min at room temperature. Detection antibody layers and their respective dilution in Block-Aid are described in table D and E. For each layer, 20 µL of the antibody solution was added on the slide and covered with a combed coverslip and the slide was incubated in humid atmosphere at 37 °C for 20 min. The slides were washed 3 times in a 2x SSC, 1 % Tween20 solution for 3 min at room temperature between each layer and after the last layer.

Detection of entire HIV-1 was carried out using a Texas Red coupled mouse anti-digoxygenin (Jackson Immunoresearch, France) antibody in a 1:25 dilution for HIV-1 probes, and an Alexa488-coupled streptavidin antibody (Invitrogen, France) in a 1:25 dilution for fosmids as primary antibodies. As second layer, an Alexa594-coupled goat anti mouse (Invitrogen, France) diluted at 1:25 and a biotinylated goat antistreptavidin (Vector Laboratories, UK) diluted at 1:50 were used. To amplify the Alexa488-fluorescence signal of fosmids, an additional detection layer was realized by using the same Alexa488 coupled-streptavidin used for the first layer at a 1:25 dilution. After the last washing steps, all glass cover slips were dehydrated in ethanol and air dried.

### Analysis of HIV-1 detected signals

Hybridized-combed DNA from ACH-2 cells preparation were scanned without any mounting medium using an inverted automated epifluorescence microscope, equipped with a 40X objective (ImageXpress Micro, Molecular Devices, USA) and the signals can be detected visually or automatically by an in house software (Gvlab 0.4.2):
- Using the two fosmids G248P87988G9 and G248P86255A8 flanking the insertion site of HIV-1, FISH signals corresponding to one of the pattern as follow were considered and measured, using an extension factor of 2 kb/µm (Schurra and Bensimon 2009):
   - FISH signals composed of a continuous signal of Texas Red/ Alexa 594-fluorescence for HIV-1. The entire signal that correspond of isolated HIV proviral DNA is measured
   - FISH signal array composed of signal chain of Texas Red/ Alexa 594-fluorescence for HIV-1, flanked by two gaps, and two continuous signals of Alexa 488-flurorescence corresponding to the fosmid sequences. The entire Texas Red/ Alexa 594 fluorescence signal and gaps length flanking this signal were measured and corresponds to HIV-1 proviral DNA integrated in the chromosome 7 at 7p15 (Ishida, Hamano et al. 2006).
   - FISH signal array with two Alexa 488-flurorescence signals separated by a gap. Measurement of the gap length that corresponds to the 7p15 locus without integration of HIV-1 proviral DNA was performed.
- Using the fosmid G248P84833H9 encompassing the HIV-1 provirus insertion site, FISH signals corresponding to one of the pattern as follow were considered and measured, using an extension factor of 2 kb/µm (Schurra and Bensimon 2009):
   - FISH signals composed of a continuous signal of Texas Red/ Alexa 594-fluorescence for HIV-1. The entire signal that correspond of isolated HIV proviral DNA is measured
   - FISH signal array composed of a continuous signal chain of Texas Red/ Alexa 594-fluorescence for HIV-1, flanked by two signals of Alexa 488-flurorescence corresponding to the fosmid sequences. The entire Texas Red/ Alexa 594 fluorescence signal was measured and corresponds to HIV-1 proviral DNA integrated in the chromosome 7 at 7p15 (Ishida, Hamano et al. 2006).
   - FISH signal with one long Alexa 488-flurorescence signals corresponds to the 7p15 locus without integration of HIV-1 proviral DNA.

### Detection of HIV-1 in ACH-2 cells culture

The inventors have applied Molecular Combing to detect complete HIV-1 integrated provirus in ACH-2 cell lines (Clouse, Powell et al. 1989), which contain a unique integrated form of HIV-1 in its genome in NT5C3 (Cytosolic 5'-nucleotidase III) gene on 7p14.3 (Ishida, Hamano et al. 2006). Labeled fosmids flanking the insertion site (G248P87988G9 and G248P86255A8) were hybridized on combed ACH-2 DNA simultaneously than labelled HIV-1 probes. A mean size of 10.2 kb +/-0.8kb was obtained from measurement of 124 HIV-1 FISH signals flanking by one or both fosmids, corresponding to the expected size of HIV-1 (9,7 kb) (Figure 8A, HIV probes: red Texas Red/Alexa 594-fluorescence, Fosmids: green Alexa 488-fluorescence). Normal alleles of NT5C3 gene are detected and measurement of the gap length between fosmids G248P87988G9 and G248P86255A8 FISH signal leads to a mean size of 32.8 ± 1.8 kb, lightly superior to the expected size of 31 kb, according to Human Mar. 2006 Assembly (NCBI Build 36.1). This result shows that Molecular Combing by its resolution may have bring some further information about this locus (Figure 8B). Furthermore, 133 isolated HIV-1 FISH signals were measured in this combed ACH-2 DNA preparation, with a mean size of 10.05 kb +/-0.79 kb (Figure 8C). This contrasts with the expected unique HIV-1 site of insertion described previously (Ishida, Hamano et al. 2006) and suggests that it exists in ACH-2 genome another or others insertion site(s) of HIV-1, or that a non integrated form of HIV-1 is persistent in ACH-2 nucleus. Similar observations are performed when the labeled fosmide encompassing the insertion site (G248P84833H9) was hybridized on combed ACH-2 DNA simultaneously than labelled HIV-1 probes (Figure 8D). A mean size of 10.4 kb +/-0.5kb was obtained from measurement of 57 HIV-1 FISH signals within the fosmide signal, corresponding to the expected size of HIV-1 provirus (9,7 kb) (HIV probes: red Texas Red/Alexa 594-fluorescence, fosmid: green Alexa 488-fluorescence). Furthermore, 35 isolated HIV-1 FISH signals were also detected and measured in this combed ACH-2 DNA preparation, with a mean size of 10.03 kb +/- 0.82 kb (not shown).

These results indicate that Molecular Combing is a powerful method to analyze the structure of the HIV genome DNA and to quantify its integration in genomic DNA at the level of the unique molecule and in any biological sample.

**TABLE F:**

| Name | Start | End | Size (bp) |
|---|---|---|---|
| HIV-S1 | 1 | 3026 | 3026 |
| HIV-S2 | 3018 | 5944 | 2927 |
| HIV-S3 | 5961 | 9709 | 3749 |

Coordinates of the three probes used in this example, relative to the Genbank sequence M19921.1

**TABLE G:**

| Probes | Forward Primer | Reverse Primer |
|---|---|---|
| HIV-S1 | TGGAAGGGCTAATTTGGTC (SEQ ID NO : 9) | TATTGCTGGTGATCCTTTCC (SEQ ID NO : 10) |
| HIV-S2 | CCAGCAATATTCCAGTGTAGC (SEQ ID NO: 11) | TGAAACAAACTTGGCAATGA (SEQ ID NO: 12) |
| HIV-S3 | CATCTCCTATGGCAGGAAGA (SEQ ID NO : 13) | TGCTAGAGATTTTCCACACTGA (SEQ ID NO : 14) |

Primers sequences used for the synthesis of probes by long-range PCR. An extract of DNA from HIV-1 pNL-4 is used as template.

### Example 3-Detecting an Oncogene by Molecular Combing

In a manner to analogous to the detection of HSV genomic DNA in Example 1, probes are designed to detect the presence of a viral oncogene. Probes are designed to complement 80-100% of the active viral oncogene of interest and Molecular Combing is performed. The results indicate the presence of an active oncogene in a subject, leading to diagnosis and therapeutic intervention.

### Example 4-Detecting Rearrangements of Infectious Viral DNA Using Molecular Combing

In a manner to analogous to the detection of HSV genomic DNA in Example 1, probes are designed to detect the presence of different arrangements of infectious viral DNA. Different sets of probes tagged with different haptens recognized by different colored fluorescent probes are designed to complement 80-100% of the active viral oncogene of interest and Molecular Combing is performed. Variations in the arrangement of viral genes in a subject's cells, tissue or biological fluid are used to diagnose or prognose the risks of progression of the viral disease or disease associated with rearrangement of the viral genome, such as the risk of or induction of a tumorigenic properties by conversion of proto-oncogenes into oncogenes.

### Example 5-Monitoring Genetic Therapy by Molecular Combing

In a manner to analogous to the detection of proviral forms of HIV-1 in Example 2, probes are specially designed to complement 80-100% of an integrated therapeutic adenovirus vector or the transgenetic sequence(s) it carries. Molecular Combing is performed using the specially designed probes to detect the presence of transgenic material integrated into a host chromosome and whether it is arranged in form that can actively express the transgene(s). The quantity of transgene(s) in the subject is followed longitudinally and a determination is made when and whether to re-administer the therapeutic adenovirus vector.

The following numbered embodiments of the invention are disclosed herein:
1. A method for detecting rearrangements of a human papilloma virus (HPV) DNA in a biological sample comprising:
   extracting a polynucleotide from said sample,
   Molecular Combing said HPV DNA to form a stretched polynucleotide,
   contacting said stretched polynucleotide with a set of labelled probes specific for HPV DNA that recognize a HPV sequence,
   detecting hybridization of the probes to the combed HPV DNA; thereby detecting rearrangements of the HPV DNA;
   wherein said biological sample is a tissue sample, cell(s), serum, blood, cerebrospinal fluid (CSF), or synovial fluid sample obtained from a human, a non-human mammal or a bird; and
   wherein said set of probes comprises at least two subsets of probes that are tagged with different labels selected so as to permit the identification of different portions or segments or rearranged fragments of a viral genome and allows to identify rearrangements that can occur within the genomic viral DNA.
2. The method of embodiment 1, wherein said HPV DNA is an infectious or a non-infectious viral DNA extracted from tissue, cell(s), serum, blood, CSF, or synovial fluid sample of a human, a non-human mammal or a bird.
3. The method of any one of embodiments 1 to 2, wherein said HPV DNA is integrated into the DNA or in episomal form.
4. The method of any one of embodiments 1 to 3, wherein said set of probes binds to at least 1 kb of the genomic DNA of a virus.
5. The method of any one of embodiments 1 to 4, wherein the HPV DNA is extracted by lysing viral particles in low-melting agarose plugs with 0.1% sodium dodecyl sulfate (SDS).
6. The method of any one of embodiments 1 to 5, wherein the set of probes covers 5 to 100% of the HPV genome.
7. The method of any one of embodiments 1 to 6, wherein the set of probes consists of a mixture of probes consisting of 2 to 20 different probes specific of the virus to be detected and capable of binding to a pathogenic or infectious viral polynucleotide sequence, said probes covering at least 20 % of said viral genome.
8. Use in the method of any one of embodiments 1 to 7 of a kit comprising reagents, comprising 0.1 % SDS for the lysis of viral particles in low-melting agarose plugs and
one or more probes that bind to an infectious viral polynucleotide for detecting or identifying genomic viral DNA in a combed DNA molecule.

### References

### Patents

U.S. Patent No. 6,130,044: Surfaces for biological reactions, process for preparing them and process for their use Bensimon D., Bensimon A., Heslot F. Oct 10, 2000
U.S. Patent No. 6,303,296, US2006257910, US6054327: Process for aligning macromolecules by passage of a meniscus and applications, Bensimon D., Bensimon A., Heslot F., Oct 16, 2001.
U.S. Patent No. 6,225,055: Apparatus for the parallel alignment of macromolecules, and use thereof, Bensimon A., Bensimon D, May 22, 2002.
U.S. 2004033510: Method for the diagnosis of genetic diseases by molecular combing and diagnostic kit, Bensimon A., Bensimon D., Michalet X., Feb 19, 2004
EP0263025: Fractions containing antigens specific for herpes virus type 1 (HSV-1) and type 2 (HSV-2), method for isolating these fractions and diagnostic method specific for HSV-1 and/or HSV-2 using these fractions, Markoulatos P., Jun 30, 1993.
EP0139416: Molecularly cloned diagnostic product and method of use, Berman, P.W., Lasky L.A, Nov 05, 2003.
WO9818959: Procédé de diagnostic de maladies génétiques par Peignage Moléculaire et coffret de diagnostic, Bensimon A., Bensimon D., Michalet X., 1998-05-07.
WO0073503: Utilisation du peignage dans l'identification des origines de replication d'ADN, Bensimon A., Herrick J., Hyrien O., 2000-12-07.
WO0202131 : Compositions et méthodes de diagnostic et de traitement de l'infection du virus de l'herpès. Hosken N.A, Day C. H., Dillon D. C., McGowan P., Sleath P. R., 2002-01-10.
WO2004036185 Diagnostic et traitement des maladies du virus de l'herpes simplex (HSV), Kriesel J. D.; Leppert M. F.; Spruance S. L., Otterud B. E M, Peiffer A. P., 2004-04-29.
WO2008028931: Genomic Morse Code, Lebofsky R., Bensimon A., Walrafen P., 2008-03-13. Scientific Publications
Barton, E. S., D. W. White, et al. (2007). "Herpesvirus latency confers symbiotic protection from bacterial infection." Nature 447(7142): 326-329.
Bataille, D. and A. L. Epstein (1997). "Equimolar generation of the four possible arrangements of adjacent L components in herpes simplex virus type 1 replicative intermediates." J Virol 71(10): 7736-7743.
Ben-Zeev, A., E. Weinberg, et al. (1974). "Isolation of herpes simplex virus DNA from virus particles and infected cells by electrophoresis in polyacrylamide gels." J Gen Virol 25(1): 63-73.
Bertoletti, A. and A. Gehring (2007). "Immune response and tolerance during chronic hepatitis B virus infection." Hepatol Res 37 Suppl 3: S331-338.
Boehmer, P. E. and I. R. Lehman (1997). "Herpes simplex virus DNA replication." Annu Rev Biochem 66: 347-384.
Breitbart, M. and F. Rohwer (2005). "Here a virus, there a virus, everywhere the same virus?" Trends Microbiol 13(6): 278-284.
Caburet, S., C. Conti, et al. (2005). "Human ribosomal RNA gene arrays display a broad range of palindromic structures." Genome Res 15(8): 1079-1085.
Cockerham, G. C., K. Bijwaard, et al. (2000). "Primary graft failure : a clinicopathologic and molecular analysis." Ophthalmology 107(11): 2083-2090;discussion 2090-2081.
Collier, L. H. and J. S. Oxford (2006). Human virology : a text for students of medicine, dentistry, and microbiology. Oxford ; New York, Oxford University Press.
Crouse, C. A., S. C. Pflugfelder, et al. (1990). "Detection of herpes viral genomes in normal and diseased corneal epithelium." Curr Eye Res 9(6): 569-581.
Davison, A. J. and N. M. Wilkie (1981). "Nucleotide sequences of the joint between the L and S segments of herpes simplex virus types 1 and 2." J Gen Virol 55(Pt 2): 315-331.
Dimmock, N. J., A. J. Easton, et al. (2007). Introduction to modern virology. Malden, MA, Blackwell Pub.
El-Aal, A. M., M. El Sayed, et al. (2006). "Evaluation of herpes simplex detection in corneal scrapings by three molecular methods." Curr Microbiol 52(5): 379-382.
Fields, B. N., D. M. Knipe, et al. (2007). Fields' virology. Philadelphia, Wolters kluwer/Lippincott Williams & Wilkins.
Fukuda, M., T. Deai, et al. (2008). "Presence of a large amount of herpes simplex virus genome in tear fluid of herpetic stromal keratitis and persistent epithelial defect patients." Semin Ophthalmol 23 (4): 217-220.
Gad, S., A. Aurias, et al. (2001). "Color bar coding the BRCA1 gene on combed DNA: a useful strategy for detecting large gene rearrangements." Genes Chromosomes Cancer 31(1): 75-84.
Gad, S., I. Bieche, et al. (2003). "Characterisation of a 161 kb deletion extending from the NBR1 to the BRCA1 genes in a French breast-ovarian cancer family." Hum Mutat 21(6): 654.
Gad, S., V. Caux-Moncoutier, et al. (2002). "Significant contribution of large BRCA1 gene rearrangements in 120 French breast and ovarian cancer families." Oncogene 21(44): 6841-6847.
Gad, S., M. Klinger, et al. (2002). "Bar code screening on combed DNA for large rearrangements of the BRCA1 and BRCA2 genes in French breast cancer families." J Med Genet 39(11): 817-821.
Gardella, T., P. Medveczky, et al. (1984). "Detection of circular and linear herpesvirus DNA molecules in mammalian cells by gel electrophoresis." J Virol 50(1): 248-254.
Goudsmit, J. (1998). Viral sex : the nature of AIDS. New York ; Oxford, Oxford University Press.
group, T. H. E. D. S. (1998). "Acyclovir for the prevention of recurrent herpes simplex virus eye disease. Herpetic Eye Disease Study Group." N Engl J Med 339(5): 300-306.
Hampson, A. W. and J. S. Mackenzie (2006). "The influenza viruses." Med J Aust 185(10 Suppl): S39-43.
Hayward, G. S., R. J. Jacob, et al. (1975). "Anatomy of herpes simplex virus DNA: evidence for four populations of molecules that differ in the relative orientations of their long and short components." Proc Nat1 Acad Sci U S A 72(11): 4243-4247.
Herrick, J., C. Conti, et al. (2005). "Genomic organization of amplified MYC genes suggests distinct mechanisms of amplification in tumorigenesis." Cancer Res 65(4): 1174-1179.
Herrick, J., S. Jun, et al. (2002). "Kinetic model of DNA replication in eukaryotic organisms." J Mol Biol 320(4): 741-750.
Herrick, J., X. Michalet, et al. (2000). "Quantifying single gene copy number by measuring fluorescent probe lengths on combed genomic DNA." Proc Natl Acad Sci U S A 97(1): 222-227.
Herrick, J., P. Stanislawski, et al. (2000). "Replication fork density increases during DNA synthesis in X. laevis egg extracts." J Mol Biol 300(5): 1133-1142.
Kabra, A., P. Lalitha, et al. (2006). "Herpes simplex keratitis and visual impairment: a case series." Indian J Ophthalmol 54(1): 23-27.
Kessler, H. H., G. Muhlbauer, et al. (2000). "Detection of Herpes simplex virus DNA by real-time PCR." J Clin Microbiol 38(7): 2638-2642.
Kotronias, D. and N. Kapranos (1998). "Detection of herpes simplex virus DNA in human spermatozoa by in situ hybridization technique." In Vivo 12(4): 391-394.
Labetoulle, M., P. Auquier, et al. (2005). "Incidence of herpes simplex virus keratitis in France." Ophthalmology 112(5): 888-895.
Lebofsky, R. and A. Bensimon (2003). "Single DNA molecule analysis: applications of molecular combing." Brief Funct Genomic Proteomic 1(4): 385-396.
Lebofsky, R. and A. Bensimon (2005). "DNA replication origin plasticity and perturbed fork progression in human inverted repeats." Mol Cell Biol 25(15): 6789-6797.
Lebofsky, R., R. Heilig, et al. (2006). "DNA replication origin interference increases the spacing between initiation events in human cells." Mol Biol Cell 17(12): 5337-5345.
Leger, J., B. Larroque, et al. (2001). "Influence of severity of congenital hypothyroidism and adequacy of treatment on school achievement in young adolescents: a population-based cohort study." Acta Paediatr 90(11): 1249-1256.
Liesegang, T. J. (1989). "Epidemiology of ocular herpes simplex. Natural history in Rochester, Minn, 1950 through 1982." Arch Ophthalmol 107(8): 1160-1165.
Liesegang, T. J. (2001). "Herpes simplex virus epidemiology and ocular importance." Cornea 20(1): 1-13.
Liesegang, T. J., L. J. Melton, 3rd, et al. (1989). "Epidemiology of ocular herpes simplex. Incidence in Rochester, Minn, 1950 through 1982." Arch Ophthalmol 107(8): 1155-1159.
Lomholt, J. A., K. Baggesen, et al. (1995). "Recurrence and rejection rates following corneal transplantation for herpes simplex keratitis." Acta Ophthalmol Scand 73(1): 29-32.
Lukashev, A. N. (2005). "Role of recombination in evolution of enteroviruses." Rev Med Virol 15(3): 157-167.
Margolis, T. P., F. L. Elfman, et al. (2007). "Spontaneous reactivation of herpes simplex virus type 1 in latently infected murine sensory ganglia." J Virol 81(20): 11069-11074.
Matsumoto, T., O. Yamada, et al. (1992). "Rapid DNA diagnosis of herpes simplex virus serotypes." J Virol Methods 40(1): 119-125.
McGeoch, D. J., M. A. Dalrymple, et al. (1988). "The complete DNA sequence of the long unique region in the genome of herpes simplex virus type 1." J Gen Virol 69 (Pt 7): 1531-1574.
McGeoch, D. J., A. Dolan, et al. (1986). "Complete DNA sequence of the short repeat region in the genome of herpes simplex virus type 1." Nucleic Acids Res 14(4): 1727-1745.
Metzner, K. J. (2006). "Detection and significance of minority quasispecies of drug-resistant HIV-1." J HIV Ther 11(4): 74-81.
Michalet, X., R. Ekong, et al. (1997). "Dynamic molecular combing: stretching the whole human genome for high-resolution studies." Science 277(5331): 1518-1523.
Mocarski, E. S. and B. Roizman (1982). "Structure and role of the herpes simplex virus DNA termini in inversion, circularization and generation of virion DNA." Cell 31(1): 89-97.
Namvar, L., S. Olofsson, et al. (2005). "Detection and typing of Herpes Simplex virus (HSV) in mucocutaneous samples by TaqMan PCR targeting a gB segment homologous for HSV types 1 and 2." J Clin Microbiol 43(5): 2058-2064.
Ou, C. Y., S. Kwok, et al. (1988). "DNA amplification for direct detection of HIV-1 in DNA of peripheral blood mononuclear cells." Science 239(4837): 295-297.
Pan, X. P., L. J. Li, et al. (2007). "Differences of YMDD mutational patterns, precore/core promoter mutations, serum HBV DNA levels in lamivudine-resistant hepatitis B genotypes B and C." J Viral Hepat 14(11): 767-774.
Pasero, P., A. Bensimon, et al. (2002). "Single-molecule analysis reveals clustering and epigenetic regulation of replication origins at the yeast rDNA locus." Genes Dev 16(19): 2479-2484.
Patel, P. K., B. Arcangioli, et al. (2006). "DNA replication origins fire stochastically in fission yeast." Mol Biol Cell 17(1): 308-316.
Pramod, N. P., P. Rajendran, et al. (1999). "Herpes simplex keratitis in South India: clinico-virological correlation." Jpn J Ophthalmol 43(4): 303-307.
Pressing, J. and D. C. Reanney (1984). "Divided genomes and intrinsic noise." J Mol Evol 20(2): 135-146.
Ramaswamy, M. and A. M. Geretti (2007). "Interactions and management issues in HSV and HIV coinfection." Expert Rev Anti Infect Ther 5(2): 231-243.
Rao, V. A., C. Conti, et al. (2007). "Endogenous gamma-H2AX-ATM-Chk2 checkpoint activation in Bloom's syndrome helicase deficient cells is related to DNA replication arrested forks." Mol Cancer Res 5(7): 713-724.
Reisinger, J., S. Rumpler, et al. (2006). "Visualization of episomal and integrated Epstein-Barr virus DNA by fiber fluorescence in situ hybridization." Int J Cancer 118(7): 1603-1608.
Rodrigues, C., M. Deshmukh, et al. (2001). "Significance of HBV DNA by PCR over serological markers of HBV in acute and chronic patients." Indian J Med Microbiol 19(3): 141-144.
Roizman, B. (1979). "The structure and isomerization of herpes simplex virus genomes." Cell 16(3): 481-494.
Rowley, A. H., R. J. Whitley, et al. (1990). "Rapid detection of herpes-simplex-virus DNA in cerebrospinal fluid of patients with herpes simplex encephalitis." Lancet 335(8687): 440-441.
Schurra, C. and A. Bensimon (2009). "Combing genomic DNA for structural and functional studies." Methods Mol Biol 464: 71-90.
Severini, A., A. R. Morgan, et al. (1994). "Study of the structure of replicative intermediates of HSV-1 DNA by pulsed-field gel electrophoresis." Virology 200(2): 428-435.
Shibata, D. K., N. Arnheim, et al. (1988). "Detection of human papilloma virus in paraffin-embedded tissue using the polymerase chain reaction." J Exp Med 167(1): 225-230.
Sugita, S., N. Shimizu, et al. (2008). "Use of multiplex PCR and real-time PCR to detect human herpes virus genome in ocular fluids of patients with uveitis." Br J Ophthalmol 92(7): 928-932.
Susloparov, M. A., I. M. Susloparov, et al. (2006). "[Herpes simplex virus type 1 and 2 (HSV-1,2) DNA detection by PCR during genital herpes]." Mol Gen Mikrobiol Virusol(1): 38-41.
Taylor, T. J., M. A. Brockman, et al. (2002). "Herpes simplex virus." Front Biosci 7: d752-764.
Umene, K. (1999). "Mechanism and application of genetic recombination in herpesviruses." Rev Med Virol 9(3): 171-182.
Vlazny, D. A., A. Kwong, et al. (1982). "Site-specific cleavage/packaging of herpes simplex virus DNA and the selective maturation of nucleocapsids containing full-length viral DNA." Proc Natl Acad Sci USA 79(5): 1423-1427.
Wadsworth, S., R. J. Jacob, et al. (1975). "Anatomy of herpes simplex virus DNA. II. Size, composition, and arrangement of inverted terminal repetitions." J Virol 15(6): 1487-1497.
Wang, K., T. Y. Lau, et al. (2005). "Laser-capture microdissection: refining estimates of the quantity and distribution of latent herpes simplex virus 1 and varicella-zoster virus DNA in human trigeminal Ganglia at the single-cell level." J Virol 79(22): 14079-14087.
Whitley, R. J. and B. Roizman (2001). "Herpes simplex virus infections." Lancet 357(9267): 1513-1518.
Wildy, P., W. C. Russell, et al. (1960). "The morphology of herpes virus." Virology 12: 204-222.
Wilhelmus, K. R., D. J. Coster, et al. (1981). "Prognosis indicators of herpetic keratitis. Analysis of a five-year observation period after corneal ulceration." Arch Ophthalmol 99(9): 1578-1582.
Worobey, M. and E. C. Holmes (1999). "Evolutionary aspects of recombination in RNA viruses." J Gen Virol 80 (Pt 10): 2535-2543.
Yu, X., B. Shi, et al. (2009). "A random PCR screening system for the identification of type 1 human herpes simplex virus." J Virol Methods 161(1): 91-97.
HIV-related references
(2000). "Time from HIV-1 seroconversion to AIDS and death before widespread use of highly-active antiretroviral therapy: a collaborative re-analysis. Collaborative Group on AIDS Incubation and HIV Survival including the CASCADE EU Concerted Action. Concerted Action on SeroConversion to AIDS and Death in Europe." Lancet 355(9210): 1131-1137.
Buchbinder, S. P., M. H. Katz, et al. (1994). "Long-term HIV-1 infection without immunologic progression." AIDS 8(8): 1123-1128.
Carrillo-Infante, C., G. Abbadessa, et al. (2007). "Viral infections as a cause of cancer (review)." Int J Oncol 30(6): 1521-1528.
Chun, T. W., D. C. Nickle, et al. (2005). "HIV-infected individuals receiving effective antiviral therapy for extended periods of time continually replenish their viral reservoir." J Clin Invest 115(11): 3250-3255.
Clouse, K. A., D. Powell, et al. (1989). "Monokine regulation of human immunodeficiency virus-1 expression in a chronically infected human T cell clone." J Immunol 142(2): 431-438.
del Rio, C. (2006). "Current concepts in antiretroviral therapy failure." Top HIV Med 14(3): 102-106.
Douek, D. C., J. M. Brenchley, et al. (2002). "HIV preferentially infects HIV-specific CD4+ T cells." Nature 417(6884): 95-98.
Douek, D. C., M. Roederer, et al. (2009). "Emerging concepts in the immunopathogenesis of AIDS." Annu Rev Med 60: 471-484.
Douglas, J. T. (2007). "Adenoviral vectors for gene therapy." Mol Biotechnol 36(1): 71-80.
Espy, M. J., J. R. Uhl, et al. (2006). "Real-time PCR in clinical microbiology: applications for routine laboratory testing." Clin Microbiol Rev 19(1): 165-256.
Fischer, M., B. Joos, et al. (2004). "Cellular viral rebound after cessation of potent antiretroviral therapy predicted by levels of multiply spliced HIV-1 RNA encoding nef." J Infect Dis 190(11): 1979-1988.
Frenkel, L. M., Y. Wang, et al. (2003). "Multiple viral genetic analyses detect low-level human immunodeficiency virus type 1 replication during effective highly active antiretroviral therapy." J Virol 77(10): 5721-5730.
Grizot, S., J. Smith, et al. (2009). "Efficient targeting of a SCID gene by an engineered single-chain homing endonuclease." Nucleic Acids Res 37(16): 5405-5419.
Havlir, D. V., M. C. Strain, et al. (2003). "Productive infection maintains a dynamic steady state of residual viremia in human immunodeficiency virus type 1-infected persons treated with suppressive antiretroviral therapy for five years." J Virol 77(20): 11212-11219.
Hermankova, M., J. D. Siliciano, et al. (2003). "Analysis of human immunodeficiency virus type 1 gene expression in latently infected resting CD4+ T lymphocytes in vivo." J Virol 77(13): 7383-7392.
Herrick, J., C. Conti, et al. (2005). "Genomic organization of amplified MYC genes suggests distinct mechanisms of amplification in tumorigenesis." Cancer Res 65(4): 1174-1179.
Huser, D. and R. Heilbronn (2003). "Adeno-associated virus integrates site-specifically into human chromosome 19 in either orientation and with equal kinetics and frequency." J Gen Virol 84(Pt 1): 133-137.
Ishida, T., A. Hamano, et al. (2006). "5' long terminal repeat (LTR)-selective methylation of latently infected HIV-1 provirus that is demethylated by reactivation signals." Retrovirology 3: 69.
Lawn, S. D. (2004). "AIDS in Africa: the impact of coinfections on the pathogenesis of HIV-1 infection." J Infect 48(1): 1-12.
Morgan, D., C. Mahe, et al. (2002). "HIV-1 infection in rural Africa: is there a difference in median time to AIDS and survival compared with that in industrialized countries?" AIDS 16(4): 597-603.
Parsonnet, J. (1999). Microbes and malignancy : infection as a cause of human cancers. New York; Oxford, Oxford University Press.
Ramratnam, B., R. Ribeiro, et al. (2004). "Intensification of antiretroviral therapy accelerates the decay of the HIV-1 latent reservoir and decreases, but does not eliminate, ongoing virus replication." J Acquir Immune Defic Syndr 35(1): 33-37.
Schneider, M. F., S. J. Gange, et al. (2005). "Patterns of the hazard of death after AIDS through the evolution of antiretroviral therapy: 1984-2004." AIDS 19(17): 2009-2018.
Schurra, C. and A. Bensimon (2009). "Combing genomic DNA for structural and functional studies." Methods Mol Biol 464: 71-90.
Siliciano, J. D., J. Kajdas, et al. (2003). "Long-term follow-up studies confirm the stability of the latent reservoir for HIV-1 in resting CD4+ T cells." Nat Med 9(6): 727-728.
Thomas, C. E., A. Ehrhardt, et al. (2003). "Progress and problems with the use of viral vectors for gene therapy." Nat Rev Genet 4(5): 346-358.
Tobin, N. H., G. H. Learn, et al. (2005). "Evidence that low-level viremias during effective highly active antiretroviral therapy result from two processes: expression of archival virus and replication of virus." J Virol 79(15): 9625-9634.
Weiss, R. A. (1993). "How does HIV cause AIDS?" Science 260(5112): 1273-1279.
Zhou, H. S., N. Zhao, et al. (2007). "Site-specific transfer of an intact beta-globin gene cluster through a new targeting vector." Biochem Biophys Res Commun 356(1): 32-37.

## Claims

1. A method for detecting rearrangements of a human papilloma virus (HPV) DNA in a biological sample comprising:
extracting a polynucleotide from said sample;
Molecular Combing said HPV DNA to form a stretched polynucleotide;
contacting said stretched polynucleotide with a set of labelled probes specific for HPV DNA that recognize a HPV sequence;
detecting hybridization of the probes to the combed HPV DNA; thereby detecting rearrangements of the HPV DNA;
wherein said biological sample is a tissue sample, cell(s), serum, blood, cerebrospinal fluid (CSF), or synovial fluid sample obtained from a human, a non-human mammal or a bird; and
wherein said set of probes comprises at least two subsets of probes that are tagged with different labels selected so as to permit the identification of different portions or segments or rearranged fragments of a viral genome and allows to identify rearrangements that can occur within the genomic viral DNA.

2. The method of claim 1, wherein said HPV DNA is an infectious or a non-infectious viral DNA extracted from tissue, cell(s), serum, blood, CSF, or synovial fluid sample of a human, a non-human mammal or a bird.

3. The method of any one of claims 1 to 2, wherein said HPV DNA is integrated into the DNA or in episomal form.

4. The method of any one of claims 1 to 3, wherein said set of probes binds to at least 1 kb of the genomic DNA of a virus.

5. The method of any one of claims 1 to 4, wherein the HPV DNA is extracted by lysing viral particles in low-melting agarose plugs with 0.1% sodium dodecyl sulfate (SDS).

6. The method of any one of claims 1 to 5, wherein the set of probes covers 5 to 100% of the HPV genome.

7. The method of any one of claims 1 to 6, wherein the set of probes consists of a mixture of probes consisting of 2 to 20 different probes specific of the virus to be detected and capable of binding to a pathogenic or infectious viral polynucleotide sequence, said probes covering at least 20 % of said viral genome.

8. Use in the method of any one of claims 1 to 7 of a kit comprising reagents, comprising 0.1 % SDS for the lysis of viral particles in low-melting agarose plugs and one or more probes that bind to an infectious viral polynucleotide for detecting or identifying genomic viral HPV DNA, in a combed DNA molecule.

## Patentansprüche

1. Verfahren zum Nachweisen von Umstrukturierungen einer humanen Papillomavirus (HPV)-DNA in einer biologischen Probe, umfassend:
Extrahieren eines Polynukleotids aus der Probe;
"Molecular Combing" (molekulares Kämmen) der HPV-DNA, um ein gedehntes Polynukleotid herzustellen;
Inkontaktbringen des gedehnten Polynukleotids mit einem Satz an markierten Sonden, die für HPV-DNA spezifisch sind, die eine HPV-Sequenz erkennen;
Nachweisen von Hybridisierung der Sonden an die gekämmte HPV-DNA; dadurch Nachweisen von Umstrukturierungen der HPV-DNA;
wobei die biologische Probe eine Gewebeprobe, Zelle(n), Serum, Blut, Zerebrospinalflüssigkeit (CSF) oder Synovialflüsigkeitsprobe ist, die von einem Mensch, einem nicht humanen Säuger oder einem Vogel erhalten ist; und
wobei der Satz an Sonden mindestens zwei Untersätze an Sonden umfasst, die mit verschiedenen Markern markiert sind, um die Identifizierung verschiedener Teile oder Segmente oder umstrukturierter Fragmente eines viralen Genoms zu erlauben, und erlaubt, Umstrukturierungen zu identifizieren, die innerhalb der genomischen viralen DNA auftreten können.

2. Verfahren nach Anspruch 1, wobei die HPV-DNA eine infektiöse oder nicht infektiöse virale DNA ist, die aus Gewebe, Zelle(n), Serum, Blut, CSF oder einer Synovialflüsigkeitsprobe eines Menschen, eines nicht humanen Säugers oder eines Vogels extrahiert ist.

3. Verfahren nach einem beliebigen der Ansprüche 1 bis 2, wobei die HPV-DNA in die DNA integriert oder in episomaler Form vorliegt.

4. Verfahren nach einem beliebigen der Ansprüche 1 bis 3, wobei der Satz an Sonden an mindestens 1 kb der genomischen DNA eines Virus bindet.

5. Verfahren nach einem beliebigen der Ansprüche 1 bis 4, wobei die HPV-DNA durch Lysieren von viralen Partikeln in niedrigschmelzenden Agarosepfropfen mit 0,1% Natriumdodecylsulfat (SDS) extrahiert wird.

6. Verfahren nach einem beliebigen der Ansprüche 1 bis 5, wobei der Satz an Sonden 5 bis 100% des HPV-Genoms abdeckt.

7. Verfahren nach einem beliebigen der Ansprüche 1 bis 6, wobei der Satz an Sonden aus einer Mischung an Sonden besteht, die aus 2 bis 20 verschiedenen Sonden bestehen, die spezifisch für den nachzuweisenden Virus sind und zum Binden an eine pathogene oder infektiöse virale Polynukleotidsequenz fähig sind, wobei die Sonden mindestens 20% des viralen Genoms abdecken.

8. Verwendung im Verfahren gemäß einem beliebigen der Ansprüche 1 bis 7 eines Kits, das Reagenzien umfasst, umfassend 0,1% SDS für die Lyse von viralen Partikeln in niedrigschmelzenden Agarosepfropfen und eine oder mehrere Sonden, die an ein infektiöses virales Polynukleotid binden, zum Nachweisen oder Identifizieren von genomischer viraler HPV-DNA in einem gekämmten DNA-Molekül.

## Revendications

1. Méthode pour détecter des réarrangements d'un ADN du virus du papillome humain (HPV) dans un échantillon biologique comprenant :
l'extraction d'un polynucléotide dudit échantillon ;
le peigner moléculaire dudit ADN de HPV pour former un polynucléotide étiré ;
la mise en contact dudit polynucléotide étiré avec un ensemble de sondes marquées spécifiques pour l'ADN de HPV qui reconnaissent une séquence de HPV ;
la détection de l'hybridation de sondes à l'ADN de HPV peigné ; par-là la détection de réarrangements de l'ADN de HPV ;
dans laquelle ledit échantillon biologique est un échantillon de tissu, de cellule(s), de sérum, de sang, de liquide céphalo-rachidien (LCR), ou de liquide synovial obtenu d'un humain, d'un mammifère non-humain ou d'un oiseau ; et
dans laquelle ledit ensemble de sondes comprend au moins deux sous-ensembles de sondes qui sont marquées avec différents marqueurs sélectionnés de façon à permettre l'identification de différent(e)s portions ou segments ou fragments réarrangés d'un génome viral et permet d'identifier des réarrangements qui peuvent survenir dans l'ADN du génome viral.

2. La méthode de la revendication 1, dans laquelle ledit ADN de HPV est un ADN viral infectieux ou non-infectieux extrait de tissu, de cellule(s), de sérum, de sang, de LCR, ou de liquide synovial d'un humain, d'un mammifère non-humain ou d'un oiseau.

3. La méthode de l'une quelconque des revendications 1 à 2, dans laquelle ledit ADN de HPV est intégré dans l'ADN ou est sous forme épisomale.

4. La méthode de l'une quelconque des revendications 1 à 3, dans laquelle ledit ensemble de sondes se lie à au moins 1 kb de l'ADN génomique d'un virus.

5. La méthode de l'une quelconque des revendications 1 à 4, dans laquelle ledit ADN de HPV est extrait par lyse de particules virales en bouchons d'agarose à bas point de fusion avec du dodécylsulfate de sodium (SDS) à 0,1%.

6. La méthode de l'une quelconque des revendications 1 à 5, dans laquelle l'ensemble de sondes couvre 5 à 100 % du génome de HPV.

7. La méthode de l'une quelconque des revendications 1 à 6, dans laquelle l'ensemble de sondes consiste en un mélange de sondes consistant en 2 à 20 sondes différentes spécifiques du virus à détecter et capables de se lier à une séquence polynucléotidique virale infectieuse ou pathogénique, lesdites sondes couvrant au moins 20% dudit génome viral.

8. Utilisation dans la méthode de l'une quelconque des revendications 1 à 7 d'une trousse comprenant des réactifs, comprenant du SDS à 0,1% pour la lyse de particules virales en bouchons d'agarose à bas point de fusion et une ou plusieurs sondes qui se lient à un polynucléotide viral infectieux pour la détection ou l'identification d'un ADN viral de HPV génomique, dans une molécule d'ADN étirée.
